(19) Europäisches Patentamt
European Patent Office
Office européen des brevets

(11) **EP 3 060 659 B1**

(12) **EUROPEAN PATENT SPECIFICATION**

(45) Date of publication and mention
of the grant of the patent:
**29.05.2019 Bulletin 2019/22**

(21) Application number: **14782002.1**

(22) Date of filing: **19.09.2014**

(51) Int Cl.:
*C12N 9/28* (2006.01)     *C11D 3/386* (2006.01)

(86) International application number:
**PCT/US2014/056571**

(87) International publication number:
**WO 2015/050723 (09.04.2015 Gazette 2015/14)**

(54) **ALPHA-AMYLASES FROM EXIGUOBACTERIUM, AND METHODS OF USE, THEREOF**

ALPHA-AMYLASE AUS EXIGUOBACTERIUM, UND DEREN VERFAHREN UND ANWENDUNG

ALPHA-AMYLASES DE EXIGUOBACTERIUM, METHODES ET UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.10.2013 PCT/CN2013/084808**

(43) Date of publication of application:
**31.08.2016 Bulletin 2016/35**

(73) Proprietor: **Danisco US Inc.**
**Palo Alto, California 94304 (US)**

(72) Inventors:
• **LIU, Guoqing**
  **Palo Alto, California 94304 (US)**
• **HUA, Ling**
  **Palo Alto, California 94304 (US)**
• **QIAN, Zhen**
  **Palo Alto, California 94304 (US)**
• **SONG, Danfeng**
  **Palo Alto, California 94304 (US)**
• **TANG, Zhongmei**
  **Palo Alto, California 94304 (US)**
• **XIE, Zhiyong**
  **Palo Alto, California 94304 (US)**
• **ZHANG, Bo**
  **Palo Alto, California 94304 (US)**
• **ZOU, Zhengzheng**
  **Palo Alto, California 94304 (US)**
• **XU, Wei**
  **Palo Alto, California 94304 (US)**

(74) Representative: **Mewburn Ellis LLP**
**City Tower**
**40 Basinghall Street**
**London EC2V 5DE (GB)**

(56) References cited:
**WO-A1-2010/104675     WO-A2-2010/115021**
**US-B2- 8 206 966**

• **DATABASE UniProt [Online] 28 November 2012 (2012-11-28), "SubName: Full=Alpha-amylase {ECO:0000313|EMBL:AFS70088.1};",** XP002733768, retrieved from EBI accession no. UNIPROT:K0A9I3 Database accession no. K0A9I3
• **YUTAKA SUZUKI ET AL: "Amino Acid Residues Stabilizing a Bacillus α-Amylase against Irreversible Thermoinactivation", THE JOURNAL OF BIOLOGICAL CHEMISTRY,, vol. 264, no. 32, 15 November 1989 (1989-11-15), pages 18933-18938,** XP001317194,

**Description**

**FIELD OF THE INVENTION**

Disclosed are compositions and methods relating to α-amylase enzymes from *Exiguobacterium*. The α-amylases are useful, for example, for starch liquefaction and saccharification, cleaning starchy stains, textile desizing, baking, and brewing.

**BACKGROUND**

Starch consists of a mixture of amylose (15-30% w/w) and amylopectin (70-85% w/w). Amylose consists of linear chains of α-1,4-linked glucose units having a molecular weight (MW) from about 60,000 to about 800,000. Amylopectin is a branched polymer containing α-1,6 branch points every 24-30 glucose units; its MW may be as high as 100 million.

α-amylases hydrolyze starch, glycogen, and related polysaccharides by cleaving internal α-1,4-glucosidic bonds at random. α-amylases, particularly from *Bacilli,* have been used for a variety of different purposes, including starch liquefaction and saccharification, textile desizing, starch modification in the paper and pulp industry, brewing, baking, production of syrups for the food industry, production of feedstocks for fermentation processes, and in animal feed to increase digestability. α-amylases have also be used to remove starchy soils and stains during dishwashing and laundry washing.

WO20100104675 discloses an α-amylase from Bacillus megaterium strain DSM90 showing 75 % identity to seq.id.4 of the present application. It discloses the use of α-amylase in the preparation of cleaning compositions, such as detergent compositions for cleaning laundry, dishes, or textiles and in the use for liquefaction of solid starch or in the use for brewing.

**SUMMARY**

The present compositions and methods relate to α-amylase polypeptides, and methods of use, thereof. Aspects and embodiments of the present compositions and methods include a recombinant *Exiguobacterium* α-amylase having α-amylase activity and comprising an amino acid sequence having at least 96% amino acid sequence identity to the amino acid sequence of SEQ ID NO: 4 or having at least 93% amino acid sequence identity to the amino acid sequence of SEQ ID NO: 5, wherein the alpha-amylase comprises a deletion of one of more residues corresponding to R179, G180, T181, or G182, and/or one or more substitutions selected from G242Q, T242Q, D188P, N188P, and G477K, referring to SEQ ID NO: 4 or SEQ ID NO: 5 for numbering.

The α-amylase may further comprise conservative substitutions of one or several amino acid residues, and/or a deletion, substitution, insertion, or addition of one or a few amino acid residues other than 179, 180, 181, 182, 242, 188, and 477, referring to SEQ ID NO: 4 or SEQ ID NO: 5 for numbering.

The invention also provides a composition comprising the α-amylase. The composition may further comprising a surfactant. The composition may be a detergent composition. The composition may be a laundry detergent, a laundry detergent additive, or a manual or automatic dishwashing detergent.

The composition may comprise one or more additional enzymes selected from the group consisting of protease, hemicellulase, cellulase, peroxidase, lipolytic enzyme, metallolipolytic enzyme, xylanase, lipase, phospholipase, esterase, perhydrolase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase, laccase, metalloproteinase, amadoriase and an amylase other than a recombinant *Exiguobacterium* α-amylase.

The composition may be for saccharifying a composition comprising starch, for SSF post liquefaction, or for direct SSF without prior liquefaction. The composition may be for producing a fermented beverage or a baked food product. The composition may be for textile desizing, optionally wherein the composition further comprises a surfactant.

Also provided is a recombinant polynucleotide encoding the alpha-amylase polypeptide. The polynucleotide may have at least 80% nucleic acid sequence identity to the polynucleotide of SEQ ID NO: 16.

Also provided is an expression vector comprising the polynucleotide, and a host cell comprising the expression vector.

The invention provides the use of the α-amylase in the production of a composition comprising glucose, in the production of a liquefied starch, in the production of a foodstuff or beverage, in cleaning starchy stains, or in textile desizing.

Additionally, the invention provides a method for removing a starchy stain or soil from a surface, comprising: contacting the surface with a composition comprising an effective amount of the recombinant *Exiguobacterium* α-amylase; and allowing the α-amylase to hydrolyze starch components present in the starchy stain to produce smaller starch-derived molecules that dissolve in aqueous solution; thereby removing the starchy stain from the surface.

In this method, the aqueous composition may further comprise a surfactant. The surface may be a textile surface or a surface on dish ware. The composition may further comprise at least one additional enzyme selected from the group consisting of protease, hemicellulase, cellulase, peroxidase, lipolytic enzyme, metallolipolytic enzyme, xylanase, lipase, phospholipase, esterase, perhydrolase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase, laccase, metalloproteinase, amadoriase, and an amylase other than an *Exiguobacterium* α-amylase.

Also provided is a method for desizing a textile comprising: contacting a sized textile with an effective amount of the *Exiguobacterium* α-amylase; and allowing the α-amylase to hydrolyze starch components in the size to produce smaller starch-derived molecules that dissolve in aqueous solution; thereby removing the size from the textile.

Also provided is a method for saccharifying a composition comprising starch to produce a composition comprising glucose, the method comprising: contacting the composition comprising starch with effective amount of the *Exiguobacterium* α-amylase; and saccharifying the composition comprising starch to produce the composition comprising glucose; wherein the α-amylase catalyzes the saccharification of the starch solution to glucose. The composition comprising starch may comprise liquefied starch, gelatinized starch, or granular starch.

Also provided is a method for preparing a foodstuff or beverage comprising: contacting a foodstuff or beverage comprising starch with the *Exiguobacterium* α-amylase; and allowing the α-amylase to hydrolyze the starch to produce smaller starch-derived molecules. The method may further comprise contacting the foodstuff or beverage with glucoamylase, hexokinase, xylanase, glucose isomerase, xylose isomerase, phosphatase, phytase, pullulanase, β-amylase, α-amylase that is not the variant α-amylase, protease, cellulase, hemicellulase, lipase, cutinase, isoamylase, redox enzyme, esterase, transferase, pectinase, aglucosidase, beta-glucosidase, or a combination thereof.

According to any of these methods, the α-amylase may be expressed and secreted by a host cell. The composition comprising starch may be contacted with the host cell. The host cell may further express and secrete a glucoamylase or other enzyme. The host cell may be capable of fermenting the composition.

## BRIEF DESCRIPTION OF THE DRAWINGS

Figure 1A-C is a Clustal W alignment (default parameters) of the amino acid sequences of *Exiguobacterium* α-amylases compared to several *Bacillus* sp. α-amylases.

Position numbering is for the exemplified *Exiguobacterium* α-amylases having the amino acid sequences of SEQ ID NOs: 2-6. Corresponding position numbers for SEQ ID NO: 1 can be determined from the alignment.

Figure 2 is a map of the expression plasmid made to express EacAmy1. The expression plasmid is representitive of others described herein.

Figure 3 is a graph showing the cleaning performance of EsiAmy1 and EsiAmy1-V1 amylases at 25°C, pH 8 (HEPES buffer) on CS-28 rice starch microswatches.

Figure 4 is a graph showing the cleaning performance of EacAmy1 and EacAmy1-V1 amylases at 30°C, pH 8 (HEPES buffer) on CS-28 rice starch microswatches.

Figure 5 is a graph showing the cleaning performance of EsoAmy1 and EsoAmy1-V1 amylases at 30°C, pH 8 (HEPES buffer) on CS-28 rice starch microswatches.

Figure 6 is a graph showing the cleaning performance of EunAmy1 and EunAmy1-V1 amylases at 30°C, pH 8 (HEPES buffer) on CS-28 rice starch microswatches.

Figure 7 is a graph showing the cleaning performance of EoxAmy1 amylase at 30°C, pH 8 (HEPES buffer) on CS-28 rice starch microswatches.

## DETAILED DESCRIPTION

Described are compositions and methods relating to α-amylase enzymes from *Exiguobacterium*. Exemplary applications for the α-amylase enzymes are for starch liquefaction and saccharification, for cleaning starchy stains in laundry, dishwashing, and other applications, for textile processing (e.g., desizing), in animal feed for improving digestibility, and for baking and brewing. These and other aspects of the compositions and methods are described in detail, below.

Prior to describing the various aspects and embodiments of the present compositions and methods, the following definitions and abbreviations are described.

## 1. Definitions and Abbreviations

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages and equivalents thereof known to those skilled in the art, and so forth.

The present document is organized into a number of sections for ease of reading; however, the reader will appreciate that statements made in one section may apply to other sections. In this manner, the headings used for different sections of the disclosure should not be construed as limiting.

Unless defined otherwise, all technical and scientific terms used herein have the same meaning as commonly understood by one of ordinary skill in the art. The following terms are provided below.

### 1.1. Abbreviations and Acronyms

The following abbreviations/acronyms have the following meanings unless otherwise specified:

| | |
|---|---|
| ABTS | 2,2-azino-bis-3-ethylbenzothiazoline-6-sulfonic acid |
| AE or AEO | alcohol ethoxylate |
| AES or AEOS | alcohol ethoxysulfate |
| AkAA | *Aspergillus kawachii* $\alpha$-amylase |
| AnGA | *Aspergillus niger* glucoamylase |
| AOS | $\alpha$-olefinsulfonate |
| AS | alkyl sulfate |
| cDNA | complementary DNA |
| CMC | carboxymethylcellulose |
| DE | dextrose equivalent |
| DNA | deoxyribonucleic acid |
| DPn | degree of saccharide polymerization having n subunits |
| ds or DS | dry solids |
| DTMPA | diethylenetriaminepentaacetic acid |
| EC | Enzyme Commission |
| EDTA | ethylenediaminetetraacetic acid |
| EO | ethylene oxide (polymer fragment) |
| EOF | End of Fermentation |
| GA | glucoamylase |
| GAU/g ds | glucoamylase activity unit/gram dry solids |
| HFCS | high fructose corn syrup |
| HgGA | *Humicola grisea* glucoamylase |
| IPTG | isopropyl $\beta$-D-thiogalactoside |
| IRS | insoluble residual starch |
| kDa | kiloDalton |
| LAS | linear alkylbenzenesulfonate |
| LAT, BLA | *B. licheniformis* amylase |
| MW | molecular weight |
| MWU | modified Wohlgemuth unit; $1.6 \times 10^{-5}$ mg/MWU = unit of activity |
| NCBI | National Center for Biotechnology Information |
| NOBS | nonanoyloxybenzenesulfonate |
| NTA | nitriloacetic acid |
| OxAm | Purastar HPAM 5000L (Danisco US Inc.) |
| PAHBAH | p-hydroxybenzoic acid hydrazide |
| PEG | polyethyleneglycol |
| pI | isoelectric point |
| PI | performance index |
| ppm | parts per million, *e.g.*, $\mu$g protein per gram dry solid |
| PVA | poly(vinyl alcohol) |
| PVP | poly(vinylpyrrolidone) |
| RCF | relative centrifugal/centripetal force (*i.e.,* x gravity) |
| RNA | ribonucleic acid |

| SAS | alkanesulfonate |
| --- | --- |
| SDS-PAGE | sodium dodecyl sulfate polyacrylamide gel electrophoresis |
| SSF | simultaneous saccharification and fermentation |
| SSU/g solid | soluble starch unit/gram dry solids |
| sp. | species |
| TAED | tetraacetylethylenediamine |
| Tm | melting temperature |
| TrGA | *Trichoderma reesei* glucoamylase |
| w/v | weight/volume |
| w/w | weight/weight |
| v/v | volume/volume |
| wt% | weight percent |
| °C | degrees Centigrade |
| $H_2O$ | water |
| $dH_2O$ or DI | deionized water |
| $dIH_2O$ | deionized water, Milli-Q filtration |
| g or gm | grams |
| $\mu$g | micrograms |
| mg | milligrams |
| kg | kilograms |
| $\mu$L and $\mu$l | microliters |
| mL and ml | milliliters |
| mm | millimeters |
| $\mu$m | micrometer |
| M | molar |
| mM | millimolar |
| $\mu$M | micromolar |
| U | units |
| sec | seconds |
| min(s) | minute/minutes |
| hr(s) | hour/hours |
| DO | dissolved oxygen |
| Ncm | Newton centimeter |
| ETOH | ethanol |
| eq. | equivalents |
| N | normal |
| MWCO | molecular weight cut-off |
| SSRL | Stanford Synchrotron Radiation Lightsource |
| PDB | Protein Database |
| CAZy | Carbohydrate-Active Enzymes database |
| Tris-HCl | tris(hydroxymethyl)aminomethane hydrochloride |
| HEPES | 4-(2-hydroxyethyl)-1-piperazineethanesulfonic acid |
| mS/cm | milli-Siemens/cm |
| CV | column volumes |

### 1.2. Definitions

The terms "amylase" or "amylolytic enzyme" refer to an enzyme that is, among other things, capable of catalyzing the degradation of starch, $\alpha$-amylases are hydrolases that cleave the $\alpha$-D-($1\rightarrow4$) O-glycosidic linkages in starch. Generally, $\alpha$-amylases (EC 3.2.1.1; $\alpha$-D-($1\rightarrow4$)-glucan glucanohydrolase) are defined as endo-acting enzymes cleaving $\alpha$-D-($1\rightarrow4$) O-glycosidic linkages within the starch molecule in a random fashion yielding polysaccharides containing three or more (1-4)-$\alpha$-linked D-glucose units. In contrast, the exo-acting amylolytic enzymes, such as $\beta$-amylases (EC 3.2.1.2; $\alpha$-D-($1\rightarrow4$)-glucan maltohydrolase) and some product-specific amylases like maltogenic $\alpha$-amylase (EC 3.2.1.133) cleave the polysaccharide molecule from the non-reducing end of the substrate. $\beta$-amylases, a-glucosidases (EC 3.2.1.20; $\alpha$-D-glucoside glucohydrolase), glucoamylase (EC 3.2.1.3; $\alpha$-D-($1\rightarrow4$)-glucan glucohydrolase), and product-specific amylases like the maltotetraosidases (EC 3.2.1.60) and the maltohexaosidases (EC 3.2.1.98) can produce malto-oligosaccharides of a specific length or enriched syrups of specific maltooligosaccharides.

"Enzyme units" herein refer to the amount of product formed per time under the specified conditions of the assay. For example, a "glucoamylase activity unit" (GAU) is defined as the amount of enzyme that produces 1 g of glucose per hour from soluble starch substrate (4% DS) at 60°C, pH 4.2. A "soluble starch unit" (SSU) is the amount of enzyme that produces 1 mg of glucose per minute from soluble starch substrate (4% DS) at pH 4.5, 50°C. DS refers to "dry solids."

The term "starch" refers to any material comprised of the complex polysaccharide carbohydrates of plants, comprised of amylose and amylopectin with the formula $(C_6H_{10}O_5)_x$, wherein X can be any number. The term includes plant-based materials such as grains, cereal, grasses, tubers and roots, and more specifically materials obtained from wheat, barley, corn, rye, rice, sorghum, brans, cassava, millet, milo, potato, sweet potato, and tapioca. The term "starch" includes granular starch. The term "granular starch" refers to raw, *i.e.,* uncooked starch, *e.g.,* starch that has not been subject to gelatinization.

The terms, "wild-type," "parental," or "reference," with respect to a polypeptide, refer to a naturally-occurring polypeptide that does not include a man-made substitution, insertion, or deletion at one or more amino acid positions. Similarly, the terms "wild-type," "parental," or "reference," with respect to a polynucleotide, refer to a naturally-occurring polynucleotide that does not include a man-made nucleoside change. However, note that a polynucleotide encoding a wild-type, parental, or reference polypeptide is not limited to a naturally-occurring polynucleotide, and encompasses any polynucleotide encoding the wild-type, parental, or reference polypeptide.

Reference to the wild-type polypeptide is understood to include the mature form of the polypeptide. A "mature" polypeptide or variant, thereof, is one in which a signal sequence is absent, for example, cleaved from an immature form of the polypeptide during or following expression of the polypeptide.

The term "variant," with respect to a polypeptide, refers to a polypeptide that differs from a specified wild-type, parental, or reference polypeptide in that it includes one or more naturally-occurring or man-made substitutions, insertions, or deletions of an amino acid. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context.

In the case of the present a-amylases, "activity" refers to $\alpha$-amylase activity, which can be measured as described, herein.

The term "recombinant," when used in reference to a subject cell, nucleic acid, protein or vector, indicates that the subject has been modified from its native state. Thus, for example, recombinant cells express genes that are not found within the native (non-recombinant) form of the cell, or express native genes at different levels or under different conditions than found in nature. Recombinant nucleic acids differ from a native sequence by one or more nucleotides and/or are operably linked to heterologous sequences, *e.g.*, a heterologous promoter in an expression vector. Recombinant proteins may differ from a native sequence by one or more amino acids and/or are fused with heterologous sequences. A vector comprising a nucleic acid encoding an amylase is a recombinant vector.

The terms "recovered," "isolated," and "separated," refer to a compound, protein (polypeptides), cell, nucleic acid, amino acid, or other specified material or component that is removed from at least one other material or component with which it is naturally associated as found in nature. An "isolated" polypeptides, thereof, includes, but is not limited to, a culture broth containing secreted polypeptide expressed in a heterologous host cell.

The term "purified" refers to material (*e.g.*, an isolated polypeptide or polynucleotide) that is in a relatively pure state, *e.g.,* at least about 90% pure, at least about 95% pure, at least about 98% pure, or even at least about 99% pure.

The term "enriched" refers to material (*e.g.*, an isolated polypeptide or polynucleotide) that is in about 50% pure, at least about 60% pure, at least about 70% pure, or even at least about 70% pure.

The terms "thermostable" and "thermostability," with reference to an enzyme, refer to the ability of the enzyme to retain activity after exposure to an elevated temperature. The thermostability of an enzyme, such as an amylase enzyme, is measured by its half-life ($t_{1/2}$) given in minutes, hours, or days, during which half the enzyme activity is lost under defined conditions. The half-life may be calculated by measuring residual $\alpha$-amylase activity following exposure to (*i.e.,* challenge by) an elevated temperature.

A "pH range," with reference to an enzyme, refers to the range of pH values under which the enzyme exhibits catalytic activity.

The terms "pH stable" and "pH stability," with reference to an enzyme, relate to the ability of the enzyme to retain activity over a wide range of pH values for a predetermined period of time (*e.g.,* 15 min., 30 min., 1 hour).

The term "amino acid sequence" is synonymous with the terms "polypeptide," "protein," and "peptide," and are used interchangeably. Where such amino acid sequences exhibit activity, they may be referred to as an "enzyme." The conventional one-letter or three-letter codes for amino acid residues are used, with amino acid sequences being presented in the standard amino-to-carboxy terminal orientation (*i.e.,* N$\rightarrow$C).

The term "nucleic acid" encompasses DNA, RNA, heteroduplexes, and synthetic molecules capable of encoding a polypeptide. Nucleic acids may be single stranded or double stranded, and may be chemical modifications. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

"Hybridization" refers to the process by which one strand of nucleic acid forms a duplex with, *i.e.,* base pairs with, a complementary strand, as occurs during blot hybridization techniques and PCR techniques. Stringent hybridization conditions are exemplified by hybridization under the following conditions: 65°C and 0.1X SSC (where 1X SSC = 0.15 M NaCl, 0.015 M $Na_3$ citrate, pH 7.0). Hybridized, duplex nucleic acids are characterized by a melting temperature ($T_m$), where one half of the hybridized nucleic acids are unpaired with the complementary strand. Mismatched nucleotides within the duplex lower the $T_m$.

A "synthetic" molecule is produced by *in vitro* chemical or enzymatic synthesis rather than by an organism.

The terms "transformed," "stably transformed," and "transgenic," used with reference to a cell means that the cell contains a non-native (*e.g.*, heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

A "host strain" or "host cell" is an organism into which an expression vector, phage, virus, or other DNA construct, including a polynucleotide encoding a polypeptide of interest (*e.g.,* an amylase) has been introduced. Exemplary host strains are microorganism cells (*e.g.,* bacteria, filamentous fungi, and yeast) capable of expressing the polypeptide of interest and/or fermenting saccharides. The term "host cell" includes protoplasts created from cells.

The term "heterologous" with reference to a polynucleotide or protein refers to a polynucleotide or protein that does not naturally occur in a host cell.

The term "endogenous" with reference to a polynucleotide or protein refers to a polynucleotide or protein that occurs naturally in the host cell.

The term "expression" refers to the process by which a polypeptide is produced based on a nucleic acid sequence. The process includes both transcription and translation.

A "selective marker" or "selectable marker" refers to a gene capable of being expressed in a host to facilitate selection of host cells carrying the gene. Examples of selectable markers include but are not limited to antimicrobials (*e.g.*, hygromycin, bleomycin, or chloramphenicol) and/or genes that confer a metabolic advantage, such as a nutritional advantage on the host cell.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" refers to a DNA construct comprising a DNA sequence encoding a polypeptide of interest, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

The term "operably linked" means that specified components are in a relationship (including but not limited to juxtaposition) permitting them to function in an intended manner. For example, a regulatory sequence is operably linked to a coding sequence such that expression of the coding sequence is under control of the regulatory sequences.

A "signal sequence" is a sequence of amino acids attached to the N-terminal portion of a protein, which facilitates the secretion of the protein outside the cell. The mature form of an extracellular protein lacks the signal sequence, which is cleaved off during the secretion process.

"Biologically active" refers to a sequence having a specified biological activity, such an enzymatic activity.

The term "specific activity" refers to the number of moles of substrate that can be converted to product by an enzyme or enzyme preparation per unit time under specific conditions. Specific activity is generally expressed as units (U)/mg of protein.

As used herein, "water hardness" is a measure of the minerals (*e.g.*, calcium and magnesium) present in water.

A "swatch" is a piece of material such as a fabric that has a stain applied thereto. The material can be, for example, fabrics made of cotton, polyester or mixtures of natural and synthetic fibers. The swatch can further be paper, such as filter paper or nitrocellulose, or a piece of a hard material such as ceramic, metal, or glass. For amylases, the stain is starch based, but can include blood, milk, ink, grass, tea, wine, spinach, gravy, chocolate, egg, cheese, clay, pigment, oil, or mixtures of these compounds.

A "smaller swatch" is a section of the swatch that has been cut with a single hole punch device, or has been cut with a custom manufactured 96-hole punch device, where the pattern of the multi-hole punch is matched to standard 96-well microtiter plates, or the section has been otherwise removed from the swatch. The swatch can be of textile, paper, metal, or other suitable material. The smaller swatch can have the stain affixed either before or after it is placed into the well of a 24-, 48- or 96-well microtiter plate. The smaller swatch can also be made by applying a stain to a small piece of material. For example, the smaller swatch can be a stained piece of fabric 5/8" or 0.25" in diameter. The custom manufactured punch is designed in such a manner that it delivers 96 swatches simultaneously to all wells of a 96-well plate. The device allows delivery of more than one swatch per well by simply loading the same 96-well plate multiple times. Multi-hole punch devices can be conceived of to deliver simultaneously swatches to any format plate, including but not limited to 24-well, 48-well, and 96-well plates. In another conceivable method, the soiled test platform can be a bead made of metal, plastic, glass, ceramic, or another suitable material that is coated with the soil substrate. The one or more coated beads are then placed into wells of 96-, 48-, or 24-well plates or larger formats, containing suitable buffer and enzyme.

"A cultured cell material comprising an amylase" or similar language, refers to a cell lysate or supernatant (including media) that includes an amylase as a component. The cell material may be from a heterologous host that is grown in culture for the purpose of producing the amylase.

"Percent sequence identity" means that a particular sequence has at least a certain percentage of amino acid residues identical to those in a specified reference sequence, when aligned using the CLUSTAL W algorithm with default parameters. *See* Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF. |

Deletions are counted as non-identical residues, compared to a reference sequence. Deletions occurring at either termini are included. For example, a variant 500-amino acid residue polypeptide with a deletion of five amino acid residues from the C-terminus would have a percent sequence identity of 99% (495/500 identical residues $\times$ 100) relative to the parent polypeptide. Such a variant would be encompassed by the language, "a variant having at least 99% sequence identity to the parent."

"Fused" polypeptide sequences are connected, *i.e.,* operably linked, via a peptide bond between two subject polypeptide sequences.

The term "filamentous fungi" refers to all filamentous forms of the subdivision Eumycotina, particularly Pezizomycotina species.

The term "degree of polymerization" (DP) refers to the number (n) of anhydro-glucopyranose units in a given saccharide. Examples of DP1 are the monosaccharides glucose and fructose. Examples of DP2 are the disaccharides maltose and sucrose. The term "DE," or "dextrose equivalent," is defined as the percentage of reducing sugar, *i.e.,* D-glucose, as a fraction of total carbohydrate in a syrup.

The term "dry solids content" (ds) refers to the total solids of a slurry in a dry weight percent basis. The term "slurry" refers to an aqueous mixture containing insoluble solids.

The phrase "simultaneous saccharification and fermentation (SSF)" refers to a process in the production of biochemicals in which a microbial organism, such as an ethanologenic microorganism, and at least one enzyme, such as an amylase, are present during the same process step. SSF includes the contemporaneous hydrolysis of starch substrates (granular, liquefied, or solubilized) to saccharides, including glucose, and the fermentation of the saccharides into alcohol or other biochemical or biomaterial in the same reactor vessel.

An "ethanologenic microorganism" refers to a microorganism with the ability to convert a sugar or oligosaccharide to ethanol.

The term "fermented beverage" refers to any beverage produced by a method comprising a fermentation process, such as a microbial fermentation, *e.g.,* a bacterial and/or fungal fermentation. "Beer" is an example of such a fermented beverage, and the term "beer" is meant to comprise any fermented wort produced by fermentation/brewing of a starch-containing plant material. Often, beer is produced exclusively from malt or adjunct, or any combination of malt and adjunct.

The term "malt" refers to any malted cereal grain, such as malted barley or wheat.

The term "adjunct" refers to any starch and/or sugar containing plant material that is not malt, such as barley or wheat malt. Examples of adjuncts include common corn grits, refined corn grits, brewer's milled yeast, rice, sorghum, refined corn starch, barley, barley starch, dehusked barley, wheat, wheat starch, torrified cereal, cereal flakes, rye, oats, potato, tapioca, cassava and syrups, such as corn syrup, sugar cane syrup, inverted sugar syrup, barley and/or wheat syrups, and the like.

The term "mash" refers to an aqueous slurry of any starch and/or sugar containing plant material, such as grist, *e.g.*, comprising crushed barley malt, crushed barley, and/or other adjunct or a combination thereof, mixed with water later to be separated into wort and spent grains.

The term "wort" refers to the unfermented liquor run-off following extracting the grist during mashing.

"Iodine-positive starch" or "IPS" refers to (1) amylose that is not hydrolyzed after liquefaction and saccharification, or (2) a retrograded starch polymer. When saccharified starch or saccharide liquor is tested with iodine, the high DPn amylose or the retrograded starch polymer binds iodine and produces a characteristic blue color. The saccharide liquor is thus termed "iodine-positive saccharide," "blue saccharide," or "blue sac."

The terms "retrograded starch" or "starch retrogradation" refer to changes that occur spontaneously in a starch paste or gel on ageing.

The term "about" refers to ± 15% to the referenced value.

## 2. α-Amylases from *Exiguobacterium*

An aspect of the present compositions and methods relates to α-amylase enzymes from bacteria of the genus *Exiguobacterium. Exiguobacterium* are low G+C content, Gram-positive, facultative anaerobes isolated from diverse environment ranging from ancient Siberian permafrost to hot springs at Yellowstone National Park (Vishnivetskaya, T.A. et al. (2009) Extremophiles-13:541-55*). While some Exiguobacterium* have been studied, the use of a-amylases from these organisms in industrial applications does not appear to have been contemplated.

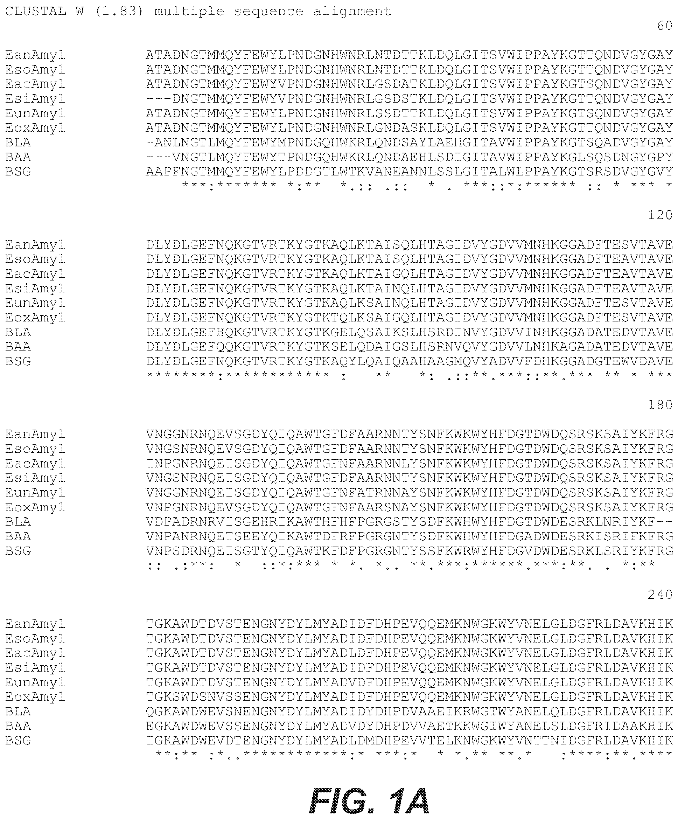

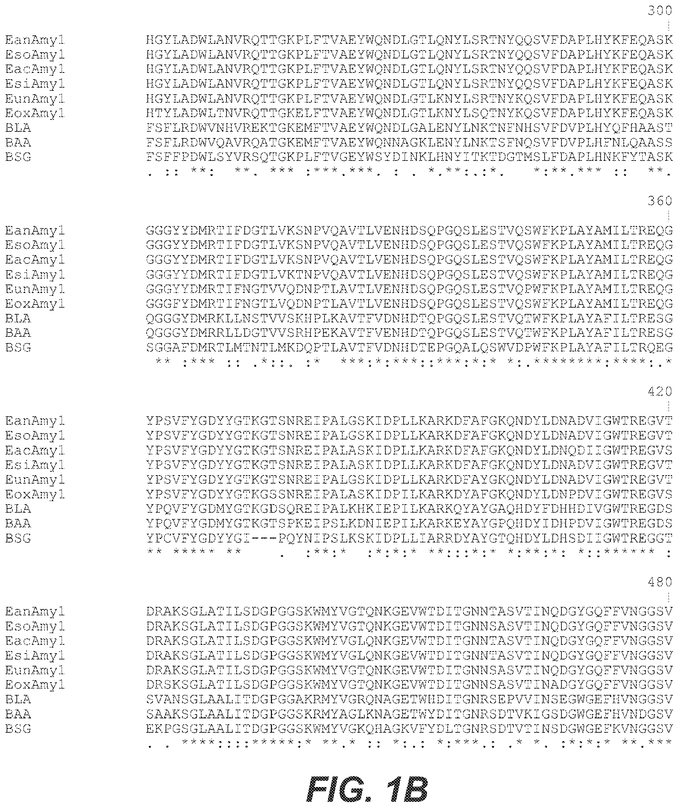

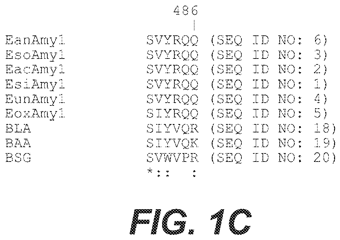

Figure 1 shown a Clustal W (default parameters) amino acid sequence alignment of the *Exiguobacterium* α-amylases, as exemplified by SEQ ID NOs: 1-6, compared to the α-amylases of *B. licheniformis* (SEQ ID NO: 18), *B. amyloliquifaciens* (SEQ ID NO: 19), and *B. stearothermophilus* (SEQ ID NO: 20).

The α-amylases of the invention have have at least 96% amino acid sequence identity to the amino acid sequence of SEQ ID NO: 4 or at least 93% amino acid sequence identity to the amino acid sequence of SEQ ID NO: 5. or example, at least 95%, at least 96%, at least 97%, at least 98%, or even at least 99%, amino acid sequence identity.

In some embodiments, the present α-amylases comprise conservative substitution of one or several amino acid residues relative to the amino acid sequence of SEQ ID NO: 4 or SEQ ID NO: 5. Exemplary conservative amino acid substitutions are listed in the Table 1. Some conservative mutations can be produced by genetic manpulation, while others are produced by introducing synthetic amino acids into a polypeptide other means.

**Table 1**. Conservative amino acid substitutions

| For Amino Acid | Code | Replace with any of |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, b-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4-carboxylic acid, D-or L-1-oxazolidine-4-carboxylic acid |

(continued)

| For Amino Acid | Code | Replace with any of |
|---|---|---|
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

In some embodiments, the present α-amylases comprises a deletion, substitution, insertion, or addition of one or a few amino acid residues relative to the amino acid sequence of SEQ ID NO: 4, or SEQ ID NO: 5 Exemplary deletions, substitutions, and insertions correspond to those that have been made in Bacillus CAzy Family 13 α-amylases. In some embodiments, the present α-amylases are derived from the amino acid sequence of SEQ ID NO: 4 or SEQ ID NO: 5 by conservative substitution of one or serveral amino acid residues. In some embodiments, the present α-amylases are derived from the amino acid sequence of SEQ ID NO: 4, or SEQ ID NO: 5, by deletion, substitution, insertion, or addition of one or few amino acid residues. In all cases, the expression "one or a few amino acid residues" refers to 10 or less, i.e., 1, 2, 3, 4, 5, 6, 7, 8, 9 or 10, amino acid residues.

The present α-amylases are characterized by having a deletion of one of more residues corresponding to R179, G180, T181, or G182, and/or one or more substitutions selected from G242Q, T242Q, D188P, N188P, and G477K, referring to SEQ ID NO: 4 or SEQ ID NO: 5 for numbering. In particular embodiments, the deletion is a pair-wise deletion of residues R179 and G180 or T181 and G182, referring to SEQ ID NO: 4, or SEQ ID NO: 5 for numbering.

In some embodiments, the present α-amylases have the residues R179 and G180, referring to SEQ ID NO: 4, or SEQ ID NO: 5

for numbering, or R176 and G177, referring to SEQ ID NO: 1 for numbering, and expressly exclude α-amylases that have the residues K179 or R179 and S180, referring to SEQ ID NO: 2, SEQ ID NO: 3, SEQ ID NO: 4, SEQ ID NO: 5, or SEQ ID NO: 6 for numbering, or K176 or R176 and S177, referring to SEQ ID NO: 1 for numbering.

In some embodiments, the present α-amylases expressly exclude α-amylases that have the amino acid sequence XNLRGKGIG (SEQ ID NO: 21) at residues corresponding to positions 89-97, where X is S or T; that have the amino acid sequence ADSLGL (SEQ ID NO: 22) at residues corresponding to positions 223-228; and/or that have the amino acid sequence GYTH (SEQ ID NO: 23) at residues corresponding to positions 281-284; referring to SEQ ID NO: 2, SEQ ID NO: 3, SEQ ID NO: 4, SEQ ID NO: 5, or SEQ ID NO: 6 for numbering.

In some embodiments, the present α-amylases are characterized by having one or more of the substitutions G242Q or T242Q, D188P, N188P, and G477K, referring to SEQ ID NO: 2, SEQ ID NO: 3, SEQ ID NO: 4, SEQ ID NO: 5, or SEQ ID NO: 6 for numbering, or G239Q, D185P, and D474K, referring to SEQ ID NO: 1 for numbering, which substitutions may be in combination with the aforementioned deletions. Examples of *Exiguobacterium* α-amylase having deletions and substitutions include SEQ ID NO: 11, SEQ ID NO: 12, SEQ ID NO: 13, SEQ ID NO: 14, and SEQ ID NO: 17. In some embodiments, the present α-amylases are characterized by having one or a few additional N-terminal and/or C-terminal residues that do not adversely affect stability or activity. Examples of *Exiguobacterium* α-amylase having additional N-terminal residues that do not interfere with stability or activity include SEQ ID NO: 7, SEQ ID NO: 8, SEQ ID NO: 9, and SEQ ID NO: 10.

α-amylases described herein may be encoded by a nucleic acid that hybridizes under stringent conditions to a nucleic acid sequence that is complementary to a nucleic acid that encodes SEQ ID NO: 1, SEQ ID NO: 2, SEQ ID NO: 3, SEQ ID NO: 4, SEQ ID NO: 5, or SEQ ID NO: 6.

The present amylases may be "precursor," "immature," or "full-length," in which case they include a signal sequence, or "mature," in which case they lack a signal sequence. Mature forms of the polypeptides are generally the most useful. Unless otherwise noted, the amino acid residue numbering used herein refers to the mature forms of the respective amylase polypeptides. The present amylase polypeptides may also be truncated to remove the N or C-termini, so long as the resulting polypeptides retain amylase activity.

The present amylase may be a "chimeric" or "hybrid" polypeptide, in that it includes at least a portion of a first amylase polypeptide, and at least a portion of a second amylase polypeptide (such chimeric amylases have recently been "rediscovered" as domain-swap amylases). The present amylases may further include heterologous signal sequence, an epitope to allow tracking or purification, or the like. Exemplary heterologous signal sequences are from *B. licheniformis* amylase (LAT), *B. subtilis* (AmyE or AprE), and *Streptomyces* CelA.

In another aspect, nucleic acids encoding an α-amylase polypeptide is provided. In some embodiments, the nucleic acid has at least 80%, at least 85%, at least 90%, at least 95%, or even at least 98% nucleotide sequence identity to SEQ ID NO: 16.

In some embodiments, the present compositions and methods include nucleic acids that encode any recombinant *Exiguobacterium* α-amylases having deletions, insertions, or substitutions, such as those mentioned, above. It will be appreciated that due to the degeneracy of the genetic code, a plurality of nucleic acids may encode the same polypeptide.

Nucleic acids may encode a "full-length" ("fl" or "FL") amylase, which includes a signal sequence, only the mature form of an amylase, which lacks the signal sequence, or a truncated form of an amylase, which lacks the N or C-terminus of the mature form. Preferrably, the nucleic acid are of sufficient length to encode an active amylase enzyme.

A nucleic acid that encodes an α-amylase can be operably linked to various promoters and regulators in a vector suitable for expressing the α-amylase in host cells. Exemplary promoters are from *B. licheniformis* amylase (LAT), *B. subtilis* (AmyE or AprE), and *Streptomyces* CelA. Such a nucleic acid can also be linked to other coding sequences, *e.g.,* to encode a chimeric polypeptide.

### 3. Production of α-Amylases

The present α-amylases can be produced in host cells, for example, by secretion or intracellular expression. A cultured cell material (*e.g.*, a whole-cell broth) comprising an α-amylase can be obtained following secretion of the α-amylase into the cell medium. Optionally, the α-amylase can be isolated from the host cells, or even isolated from the cell broth, depending on the desired purity of the final α-amylase. A gene encoding an α-amylase can be cloned and expressed according to methods well known in the art. Suitable host cells include bacterial, fungal (including yeast and filamentous fungi), and plant cells (including algae). Particularly useful host cells include *Aspergillus niger, Aspergillus oryzae* or *Trichoderma reesei.* Other host cells include bacterial cells, *e.g., Bacillus subtilis or B. licheniformis,* as well as *Streptomyces.*

The host cell further may express a nucleic acid encoding a homologous or heterologous glucoamylase, *i.e.,* a glucoamylase that is not the same species as the host cell, or one or more other enzymes. The glucoamylase may be a variant glucoamylase, such as one of the glucoamylase variants disclosed in U.S. Patent No. 8,058,033 (Danisco US Inc.), for example. Additionally, the host may express one or more accessory enzymes, proteins, peptides. These may benefit liquefaction, saccharification, fermentation, SSF, etc processes. Furthermore, the host cell may produce biochemicals in addition to enzymes used to digest the various feedstock(s). Such host cells may be useful for fermentation or simultaneous saccharification and fermentation processes to reduce or eliminate the need to add enzymes.

### 3.1. Vectors

A DNA construct comprising a nucleic acid encoding α-amylases can be constructed to be expressed in a host cell. Because of the well-known degeneracy in the genetic code, different polynucleotides that encode an identical amino acid sequence can be designed and made with routine skill. It is also well-known in the art to optimize codon use for a particular host cell. Nucleic acids encoding α-amylases can be incorporated into a vector. Vectors can be transferred to a host cell using well-known transformation techniques, such as those disclosed below.

The vector may be any vector that can be transformed into and replicated within a host cell. For example, a vector comprising a nucleic acid encoding an α-amylase can be transformed and replicated in a bacterial host cell as a means of propagating and amplifying the vector. The vector also may be transformed into an expression host, so that the encoding nucleic acids can be expressed as a functional amylase. Host cells that serve as expression hosts can include filamentous fungi, for example. The Fungal Genetics Stock Center (FGSC) Catalogue of Strains lists suitable vectors for expression in fungal host cells. *See* FGSC, Catalogue of Strains, University of Missouri, *at* www.fgsc.net (last modified January 17, 2007). A representative vector is pJG153, a promoterless Cre expression vector that can be replicated in a bacterial host. *See* Harrison et al. (2011) Applied Environ. Microbiol. 77:3916-22. pJG153can be modified with routine skill to comprise and express a nucleic acid encoding an amylase variant.

A nucleic acid encoding an α-amylase can be operably linked to a suitable promoter, which allows transcription in the host cell. The promoter may be any DNA sequence that shows transcriptional activity in the host cell of choice and may be derived from genes encoding proteins either homologous or heterologous to the host cell. Exemplary promoters for directing the transcription of the DNA sequence encoding an α-amylase, especially in a bacterial host, are the promoter of the lac operon of *E. coli,* the *Streptomyces coelicolor* agarase gene dagA or celA promoters, the promoters of the *Bacillus licheniformis* α-amylase gene (amyL), the promoters of the *Bacillus stearothermophilus* maltogenic amylase gene (amyM), the promoters of the *Bacillus amyloliquefaciens* α-amylase (amyQ), the promoters of the *Bacillus subtilis* xylA and xylB genes *etc.* For transcription in a fungal host, examples of useful promoters are those derived from the gene encoding *Aspergillus oryzae* TAKA amylase, *Rhizomucor miehei* aspartic proteinase, *Aspergillus niger* neutral α-amylase, *A. niger* acid stable α-amylase, *A. niger* glucoamylase, *Rhizomucor miehei* lipase, *A. oryzae* alkaline protease, *A. oryzae* triose phosphate isomerase, or *A. nidulans* acetamidase. When a gene encoding an amylase is expressed in a bacterial species such as *E. coli,* a suitable promoter can be selected, for example, from a bacteriophage promoter including a T7 promoter and a phage lambda promoter. Examples of suitable promoters for the expression in a yeast species include but are not limited to the Gal 1 and Gal 10 promoters of *Saccharomyces cerevisiae* and the *Pichia pastoris* AOX1 or AOX2 promoters. *cbh1* is an endogenous, inducible promoter from *T. reesei. See* Liu et al. (2008) "Improved heterologous gene expression in Trichoderma reesei by cellobiohydrolase I gene (cbh1) promoter optimization," Acta Biochim. Biophys. Sin (Shanghai) 40(2): 158-65.

The coding sequence can be operably linked to a signal sequence. The DNA encoding the signal sequence may be the DNA sequence naturally associated with the amylase gene to be expressed or from a different Genus or species. A signal sequence and a promoter sequence comprising a DNA construct or vector can be introduced into a fungal host cell and can be derived from the same source. For example, the signal sequence is the *cbh1* signal sequence that is operably linked to a *cbh1* promoter.

An expression vector may also comprise a suitable transcription terminator and, in eukaryotes, polyadenylation sequences operably linked to the DNA sequence encoding an α-amylase. Termination and polyadenylation sequences may suitably be derived from the same sources as the promoter.

The vector may further comprise a DNA sequence enabling the vector to replicate in the host cell. Examples of such sequences are the origins of replication of plasmids pUC19, pACYC177, pUBllO, pE194, pAMB1, and pIJ702.

The vector may also comprise a selectable marker, *e.g.,* a gene the product of which complements a defect in the isolated host cell, such as the *dal* genes from *B. subtilis* or *B. licheniformis,* or a gene that confers antibiotic resistance such as, *e.g.,* ampicillin, kanamycin, chloramphenicol or tetracycline resistance. Furthermore, the vector may comprise *Aspergillus* selection markers such as *amdS, argB, niaD* and xx*sC*, a marker giving rise to hygromycin resistance, or the selection may be accomplished by co-transformation, such as known in the art. *See e.g.,* International PCT Application WO 91/17243.

Intracellular expression may be advantageous in some respects, *e.g.*, when using certain bacteria or fungi as host cells to produce large amounts of amylase for subsequent enrichment or purification. Extracellular secretion of amylase into the culture medium can also be used to make a cultured cell material comprising the isolated amylase.

The expression vector typically includes the components of a cloning vector, such as, for example, an element that permits autonomous replication of the vector in the selected host organism and one or more phenotypically detectable markers for selection purposes. The expression vector normally comprises control nucleotide sequences such as a promoter, operator, ribosome binding site, translation initiation signal and optionally, a repressor gene or one or more activator genes. Additionally, the expression vector may comprise a sequence coding for an amino acid sequence capable of targeting the amylase to a host cell organelle such as a peroxisome, or to a particular host cell compartment. Such a targeting sequence includes but is not limited to the sequence, SKL. For expression under the direction of control sequences, the nucleic acid sequence of the amylase is operably linked to the control sequences in proper manner with respect to expression.

The procedures used to ligate the DNA construct encoding an amylase, the promoter, terminator and other elements, respectively, and to insert them into suitable vectors containing the information necessary for replication, are well known to persons skilled in the art (*see, e.g.,* Sambrook et al., MOLECULAR CLONING: A LABORATORY MANUAL, 2nd ed., Cold Spring Harbor, 1989, and 3rd ed., 2001).

### 3.2. Transformation and Culture of Host Cells

An isolated cell, either comprising a DNA construct or an expression vector, is advantageously used as a host cell in the recombinant production of an amylase. The cell may be transformed with the DNA construct encoding the enzyme, conveniently by integrating the DNA construct (in one or more copies) in the host chromosome. This integration is generally considered to be an advantage, as the DNA sequence is more likely to be stably maintained in the cell. Integration of the DNA constructs into the host chromosome may be performed according to conventional methods, *e.g.*, by homologous or heterologous recombination. Alternatively, the cell may be transformed with an expression vector as described above in connection with the different types of host cells.

Examples of suitable bacterial host organisms are Gram positive bacterial species such as *Bacillaceae* including *Bacillus subtilis, Bacillus licheniformis, Bacillus lentus, Bacillus brevis, Geobacillus* (formerly *Bacillus*) *stearothermophilus, Bacillus alkalophilus, Bacillus amyloliquefaciens, Bacillus coagulans, Bacillus lautus, Bacillus megaterium,* and *Bacillus thuringiensis; Streptomyces* species such as *Streptomyces murinus;* lactic acid bacterial species including *Lactococcus* sp. such as *Lactococcus lactis; Lactobacillus* sp. including *Lactobacillus reuteri; Leuconostoc* sp.; *Pediococcus* sp.; and *Streptococcus* sp. Alternatively, strains of a Gram negative bacterial species belonging to *Enterobacteriaceae* including *E. coli,* or to *Pseudomonadaceae* can be selected as the host organism.

A suitable yeast host organism can be selected from the biotechnologically relevant yeasts species such as but not limited to yeast species such as *Pichia* sp., *Hansenula* sp., or *Kluyveromyces, Yarrowinia, Schizosaccharomyces* species or a species of *Saccharomyces,* including *Saccharomyces cerevisiae* or a species belonging to *Schizosaccharomyces* such as, for example, *S. pombe* species. A strain of the methylotrophic yeast species, *Pichia pastoris,* can be used as the host organism. Alternatively, the host organism can be a *Hansenula* species. Suitable host organisms among filamentous fungi include species of *Aspergillus, e.g., Aspergillus niger, Aspergillus oryzae, Aspergillus tubigensis, Aspergillus awamori,* or *Aspergillus nidulans.* Alternatively, strains of a *Fusarium* species, *e.g., Fusarium oxysporum* or of a *Rhizomucor* species such as *Rhizomucor miehei* can be used as the host organism. Other suitable strains include *Thermomyces* and *Mucor* species. In addition, *Trichoderma sp.* can be used as a host. A suitable procedure for transformation of *Aspergillus* host cells includes, for example, that described in EP 238023. An amylase expressed by a fungal host cell can be glycosylated, *i.e.*, will comprise a glycosyl moiety. The glycosylation pattern can be the same or different as present in the wild-type amylase. The type and/or degree of glycosylation may impart changes in enzymatic and/or biochemical properties.

It is advantageous to delete genes from expression hosts, where the gene deficiency can be cured by the transformed expression vector. Known methods may be used to obtain a fungal host cell having one or more inactivated genes. Gene inactivation may be accomplished by complete or partial deletion, by insertional inactivation or by any other means that renders a gene nonfunctional for its intended purpose, such that the gene is prevented from expression of a functional protein. Any gene from a *Trichoderma* sp. or other filamentous fungal host that has been cloned can be deleted, for example, *cbh1, cbh2, egl1,* and *egl2* genes. Gene deletion may be accomplished by inserting a form of the desired gene to be inactivated into a plasmid by methods known in the art.

Introduction of a DNA construct or vector into a host cell includes techniques such as transformation; electroporation; nuclear microinjection; transduction; transfection, *e.g.*, lipofection mediated and DEAE-Dextrin mediated transfection; incubation with calcium phosphate DNA precipitate; high velocity bombardment with DNA-coated microprojectiles; and protoplast fusion. General transformation techniques are known in the art. *See, e.g.,* Sambrook *et al.* (2001), *supra.* The expression of heterologous protein in *Trichoderma* is described, for example, in U.S. Patent No. 6,022,725. Reference is also made to Cao et al. (2000) Science 9:991-1001 for transformation of *Aspergillus* strains. Genetically stable transformants can be constructed with vector systems whereby the nucleic acid encoding an amylase is stably integrated into a host cell chromosome. Transformants are then selected and purified by known techniques.

The preparation of *Trichoderma* sp. for transformation, for example, may involve the preparation of protoplasts from fungal mycelia. *See* Campbell et al. (1989) Curr. Genet. 16: 53-56. The mycelia can be obtained from germinated vegetative spores. The mycelia are treated with an enzyme that digests the cell wall, resulting in protoplasts. The protoplasts are protected by the presence of an osmotic stabilizer in the suspending medium. These stabilizers include sorbitol, mannitol, potassium chloride, magnesium sulfate, and the like. Usually the concentration of these stabilizers varies between 0.8 M and 1.2 M, *e.g.*, a 1.2 M solution of sorbitol can be used in the suspension medium.

Uptake of DNA into the host *Trichoderma* sp. strain depends upon the calcium ion concentration. Generally, between about 10-50 mM $CaCl_2$ is used in an uptake solution. Additional suitable compounds include a buffering system, such as TE buffer (10 mM Tris, pH 7.4; 1 mM EDTA) or 10 mM MOPS, pH 6.0 and polyethylene glycol. The polyethylene glycol is believed to fuse the cell membranes, thus permitting the contents of the medium to be delivered into the cytoplasm of the *Trichoderma* sp. strain. This fusion frequently leaves multiple copies of the plasmid DNA integrated into the host chromosome.

Usually transformation of *Trichoderma* sp. uses protoplasts or cells that have been subjected to a permeability treatment, typically at a density of $10^5$ to $10^7$/mL, particularly $2 \times 10^6$/mL. A volume of 100 $\mu$L of these protoplasts or cells in an appropriate solution (e.g., 1.2 M sorbitol and 50 mM $CaCl_2$) may be mixed with the desired DNA. Generally, a high concentration of PEG is added to the uptake solution. From 0.1 to 1 volume of 25% PEG 4000 can be added to the protoplast suspension; however, it is useful to add about 0.25 volumes to the protoplast suspension. Additives, such as dimethyl sulfoxide, heparin, spermidine, potassium chloride and the like, may also be added to the uptake solution to facilitate transformation. Similar procedures are available for other fungal host cells. *See, e.g.,* U.S. Patent No. 6,022,725.

### 3.3. Expression

A method of producing an amylase may comprise cultivating a host cell as described above under conditions conducive to the production of the enzyme and recovering the enzyme from the cells and/or culture medium.

The medium used to cultivate the cells may be any conventional medium suitable for growing the host cell in question and obtaining expression of an amylase. Suitable media and media components are available from commercial suppliers or may be prepared according to published recipes (*e.g.*, as described in catalogues of the American Type Culture Collection).

An enzyme secreted from the host cells can be used in a whole broth preparation. In the present methods, the preparation of a spent whole fermentation broth of a recombinant microorganism can be achieved using any cultivation method known in the art resulting in the expression of an α-amylase. Fermentation may, therefore, be understood as comprising shake flask cultivation, small- or large-scale fermentation (including continuous, batch, fed-batch, or solid state fermentations) in laboratory or industrial fermenters performed in a suitable medium and under conditions allowing the amylase to be expressed or isolated. The term "spent whole fermentation broth" is defined herein as unfractionated contents of fermentation material that includes culture medium, extracellular proteins (*e.g.*, enzymes), and cellular biomass. It is understood that the term "spent whole fermentation broth" also encompasses cellular biomass that has been lysed or permeabilized using methods well known in the art.

An enzyme secreted from the host cells may conveniently be recovered from the culture medium by well-known procedures, including separating the cells from the medium by centrifugation or filtration, and precipitating proteinaceous components of the medium by means of a salt such as ammonium sulfate, followed by the use of chromatographic procedures such as ion exchange chromatography, affinity chromatography, or the like.

The polynucleotide encoding an amylase in a vector can be operably linked to a control sequence that is capable of providing for the expression of the coding sequence by the host cell, *i.e.* the vector is an expression vector. The control sequences may be modified, for example by the addition of further transcriptional regulatory elements to make the level of transcription directed by the control sequences more responsive to transcriptional modulators. The control sequences may in particular comprise promoters.

Host cells may be cultured under suitable conditions that allow expression of an amylase. Expression of the enzymes may be constitutive such that they are continually produced, or inducible, requiring a stimulus to initiate expression. In the case of inducible expression, protein production can be initiated when required by, for example, addition of an inducer substance to the culture medium, for example dexamethasone or IPTG or Sophorose. Polypeptides can also be produced recombinantly in an *in vitro* cell-free system, such as the TNT™ (Promega) rabbit reticulocyte system.

An expression host also can be cultured in the appropriate medium for the host, under aerobic conditions. Shaking or a combination of agitation and aeration can be provided, with production occurring at the appropriate temperature for that host, *e.g.,* from about 25°C to about 75°C (*e.g.,* 30°C to 45°C), depending on the needs of the host and production of the desired α-amylase. Culturing can occur from about 12 to about 100 hours or greater (and any hour value there between, *e.g.*, from 24 to 72 hours). Typically, the culture broth is at a pH of about 4.0 to about 8.0, again depending on the culture conditions needed for the host relative to production of an amylase.

### 3.4. Identification of Amylase Activity

To evaluate the expression of an amylase in a host cell, assays can measure the expressed protein, corresponding mRNA, or α-amylase activity. For example, suitable assays include Northern blotting, reverse transcriptase polymerase chain reaction, and *in situ* hybridization, using an appropriately labeled hybridizing probe. Suitable assays also include measuring amylase activity in a sample, for example, by assays directly measuring reducing sugars such as glucose in the culture media. For example, glucose concentration may be determined using glucose reagent kit No. 15-UV (Sigma Chemical Co.) or an instrument, such as Technicon Autoanalyzer. α-Amylase activity also may be measured by any known method, such as the PAHBAH or ABTS assays, described below.

### 3.5. Methods for Enriching and Purifying α-Amylases

Fermentation, separation, and concentration techniques are well known in the art and conventional methods can be used in order to prepare a concentrated an α-α-amylase polypeptide-containing solution.

After fermentation, a fermentation broth is obtained, the microbial cells and various suspended solids, including residual raw fermentation materials, are removed by conventional separation techniques in order to obtain an amylase solution. Filtration, centrifugation, microfiltration, rotary vacuum drum filtration, ultrafiltration, centrifugation followed by ultra-filtration, extraction, or chromatography, or the like, are generally used.

It is desirable to concentrate an α-amylase polypeptide-containing solution in order to optimize recovery. Use of unconcentrated solutions requires increased incubation time in order to collect the enriched or purified enzyme precipitate.

The enzyme containing solution is concentrated using conventional concentration techniques until the desired enzyme level is obtained. Concentration of the enzyme containing solution may be achieved by any of the techniques discussed herein. Exemplary methods of enrichment and purification include but are not limited to rotary vacuum filtration and/or ultrafiltration.

The enzyme solution is concentrated into a concentrated enzyme solution until the enzyme activity of the concentrated α-amylase polypeptide-containing solution is at a desired level.

Concentration may be performed using, *e.g.,* a precipitation agent, such as a metal halide precipitation agent. Metal halide precipitation agents include but are not limited to alkali metal chlorides, alkali metal bromides and blends of two or more of these metal halides. Exemplary metal halides include sodium chloride, potassium chloride, sodium bromide, potassium bromide and blends of two or more of these metal halides. The metal halide precipitation agent, sodium chloride, can also be used as a preservative.

The metal halide precipitation agent is used in an amount effective to precipitate an amylase. The selection of at least an effective amount and an optimum amount of metal halide effective to cause precipitation of the enzyme, as well as the conditions of the precipitation for maximum recovery including incubation time, pH, temperature and concentration of enzyme, will be readily apparent to one of ordinary skill in the art, after routine testing.

Generally, at least about 5% w/v (weight/volume) to about 25% w/v of metal halide is added to the concentrated enzyme solution, and usually at least 8% w/v. Generally, no more than about 25% w/v of metal halide is added to the concentrated enzyme solution and usually no more than about 20% w/v. The optimal concentration of the metal halide precipitation agent will depend, among others, on the nature of the specific $\alpha$-amylase polypeptide and on its concentration in the concentrated enzyme solution.

Another alternative way to precipitate the enzyme is to use organic compounds. Exemplary organic compound precipitating agents include: 4-hydroxybenzoic acid, alkali metal salts of 4-hydroxybenzoic acid, alkyl esters of 4-hydroxybenzoic acid, and blends of two or more of these organic compounds. The addition of the organic compound precipitation agents can take place prior to, simultaneously with or subsequent to the addition of the metal halide precipitation agent, and the addition of both precipitation agents, organic compound and metal halide, may be carried out sequentially or simultaneously.

Generally, the organic precipitation agents are selected from the group consisting of alkali metal salts of 4-hydroxybenzoic acid, such as sodium or potassium salts, and linear or branched alkyl esters of 4-hydroxybenzoic acid, wherein the alkyl group contains from 1 to 12 carbon atoms, and blends of two or more of these organic compounds. The organic compound precipitation agents can be, for example, linear or branched alkyl esters of 4-hydroxybenzoic acid, wherein the alkyl group contains from 1 to 10 carbon atoms, and blends of two or more of these organic compounds. Exemplary organic compounds are linear alkyl esters of 4-hydroxybenzoic acid, wherein the alkyl group contains from 1 to 6 carbon atoms, and blends of two or more of these organic compounds. Methyl esters of 4-hydroxybenzoic acid, propyl esters of 4-hydroxybenzoic acid, butyl ester of 4-hydroxybenzoic acid, ethyl ester of 4-hydroxybenzoic acid and blends of two or more of these organic compounds can also be used. Additional organic compounds also include but are not limited to 4-hydroxybenzoic acid methyl ester (named methyl PARABEN), 4-hydroxybenzoic acid propyl ester (named propyl PARABEN), which also are both amylase preservative agents. For further descriptions, *see, e.g.,* U.S. Patent No. 5,281,526.

Addition of the organic compound precipitation agent provides the advantage of high flexibility of the precipitation conditions with respect to pH, temperature, $\alpha$-amylase concentration, precipitation agent concentration, and time of incubation.

The organic compound precipitation agent is used in an amount effective to improve precipitation of the enzyme by means of the metal halide precipitation agent. The selection of at least an effective amount and an optimum amount of organic compound precipitation agent, as well as the conditions of the precipitation for maximum recovery including incubation time, pH, temperature and concentration of enzyme, will be readily apparent to one of ordinary skill in the art, in light of the present disclosure, after routine testing.

Generally, at least about 0.01% w/v of organic compound precipitation agent is added to the concentrated enzyme solution and usually at least about 0.02% w/v. Generally, no more than about 0.3% w/v of organic compound precipitation agent is added to the concentrated enzyme solution and usually no more than about 0.2% w/v.

The concentrated polypeptide solution, containing the metal halide precipitation agent, and the organic compound precipitation agent, can be adjusted to a pH, which will, of necessity, depend on the enzyme to be enriched or purified. Generally, the pH is adjusted at a level near the isoelectric point of the amylase. The pH can be adjusted at a pH in a range from about 2.5 pH units below the isoelectric point (pI) up to about 2.5 pH units above the isoelectric point.

The incubation time necessary to obtain an enriched or purified enzyme precipitate depends on the nature of the specific enzyme, the concentration of enzyme, and the specific precipitation agent(s) and its (their) concentration. Generally, the time effective to precipitate the enzyme is between about 1 to about 30 hours; usually it does not exceed about 25 hours. In the presence of the organic compound precipitation agent, the time of incubation can still be reduced to less about 10 hours and in most cases even about 6 hours.

Generally, the temperature during incubation is between about 4°C and about 50°C. Usually, the method is carried out at a temperature between about 10°C and about 45°C (*e.g.,* between about 20°C and about 40°C). The optimal temperature for inducing precipitation varies according to the solution conditions and the enzyme or precipitation agent(s) used.

The overall recovery of enriched or purified enzyme precipitate, and the efficiency with which the process is conducted, is improved by agitating the solution comprising the enzyme, the added metal halide and the added organic compound. The agitation step is done both during addition of the metal halide and the organic compound, and during the subsequent incubation period. Suitable agitation methods include mechanical stirring or shaking, vigorous aeration, or any similar technique.

After the incubation period, the enriched or purified enzyme is then separated from the dissociated pigment and other impurities and collected by conventional separation techniques, such as filtration, centrifugation, microfiltration, rotary vacuum filtration, ultrafiltration, press filtration, cross membrane microfiltration, cross flow membrane microfiltration, or the like. Further enrichment or purification of the enzyme precipitate can be obtained by washing the precipitate with water. For example, the enriched or purified enzyme precipitate is washed with water containing the metal halide precipitation agent, or with water containing the metal halide and the organic compound precipitation agents.

During fermentation, an α-amylase polypeptide accumulates in the culture broth. For the isolation, enrichment, or purification of the desired α-amylase, the culture broth is centrifuged or filtered to eliminate cells, and the resulting cell-free liquid is used for enzyme enrichment or purification. In one embodiment, the cell-free broth is subjected to salting out using ammonium sulfate at about 70% saturation; the 70% saturation-precipitation fraction is then dissolved in a buffer and applied to a column such as a Sephadex G-100 column, and eluted to recover the enzyme-active fraction. For further enrichment or purification, a conventional procedure such as ion exchange chromatography may be used.

Enriched or purified enzymes are useful for laundry and cleaning applications. For example, they can be used in laundry detergents and spot removers. They can be made into a final product that is either liquid (solution, slurry) or solid (granular, powder).

A more specific example of enrichment or purification, is described in Sumitani et al. (2000) "New type of starch-binding domain: the direct repeat motif in the C-terminal region of Bacillus sp. 195 α-amylase contributes to starch binding and raw starch degrading," Biochem. J. 350: 477-484, and is briefly summarized here. The enzyme obtained from 4 liters of a *Streptomyces lividans* TK24 culture supernatant was treated with $(NH_4)_2SO_4$ at 80% saturation. The precipitate was recovered by centrifugation at $10,000 \times g$ (20 min. and 4°C) and re-dissolved in 20 mM Tris/HCl buffer (pH 7.0) containing 5 mM $CaCl_2$. The solubilized precipitate was then dialyzed against the same buffer. The dialyzed sample was then applied to a Sephacryl S-200 column, which had previously been equilibrated with 20 mM Tris/HCl buffer, (pH 7.0), 5 mM $CaCl_2$, and eluted at a linear flow rate of 7 mL/hr with the same buffer. Fractions from the column were collected and assessed for activity as judged by enzyme assay and SDS-PAGE. The protein was further purified as follows. A Toyopearl HW55 column (Tosoh Bioscience, Montgomeryville, PA; Cat. No. 19812) was equilibrated with 20 mM Tris/HCl buffer (pH 7.0) containing 5 mM $CaCl_2$ and 1.5 M $(NH_4)_2SO_4$. The enzyme was eluted with a linear gradient of 1.5 to 0 M $(NH_4)_2SO_4$ in 20 mM Tris/HCL buffer, pH 7.0 containing 5 mM $CaCl_2$. The active fractions were collected, and the enzyme precipitated with $(NH_4)_2SO_4$ at 80% saturation. The precipitate was recovered, re-dissolved, and dialyzed as described above. The dialyzed sample was then applied to a Mono Q HR5/5 column (Amersham Pharmacia; Cat. No. 17-5167-01) previously equilibrated with 20 mM Tris/HCl buffer (pH 7.0) containing 5 mM $CaCl_2$, at a flow rate of 60 mL/hour. The active fractions are collected and added to a 1.5 M $(NH_4)_2SO_4$ solution. The active enzyme fractions were re-chromatographed on a Toyopearl HW55 column, as before, to yield a homogeneous enzyme as determined by SDS-PAGE. *See* Sumitani et al. (2000) Biochem. J. 350: 477-484, for general discussion of the method and variations thereon.

For production scale recovery, α-amylase polypeptides can be enriched or partially purified as generally described above by removing cells via flocculation with polymers. Alternatively, the enzyme can be enriched or purified by microfiltration followed by concentration by ultrafiltration using available membranes and equipment. However, for some applications, the enzyme does not need to be enriched or purified, and whole broth culture can be lysed and used without further treatment. The enzyme can then be processed, for example, into granules.

### 4. Compositions and Uses of α-amylases

The present α-amylases are useful for a variety of industrial applications. For example, α-amylases are useful in a starch conversion process, particularly in a saccharification process of a starch that has undergone liquefaction. The desired end-product may be any product that may be produced by the enzymatic conversion of the starch substrate. For example, the desired product may be a syrup rich in glucose and maltose, which can be used in other processes, such as the preparation of HFCS, or which can be converted into a number of other useful products, such as ascorbic acid intermediates (*e.g.*, gluconate; 2-keto-L-gulonic acid; 5-keto-gluconate; and 2,5-diketogluconate); 1,3-propanediol; aromatic amino acids (*e.g.*, tyrosine, phenylalanine and tryptophan); organic acids (*e.g.*, lactate, pyruvate, succinate, isocitrate, and oxaloacetate); amino acids (*e.g.,* serine and glycine); antibiotics; antimicrobials; enzymes; vitamins; and hormones.

The starch conversion process may be a precursor to, or simultaneous with, a fermentation process designed to produce alcohol for fuel or drinking (*i.e.,* potable alcohol). One skilled in the art is aware of various fermentation conditions that may be used in the production of these end-products. α-amylases are also useful in compositions and methods of food preparation. These various uses of α-amylases are described in more detail below.

### 4.1. Preparation of Starch Substrates

Those of general skill in the art are well aware of available methods that may be used to prepare starch substrates for use in the processes disclosed herein. For example, a useful starch substrate may be obtained from tubers, roots, stems, legumes, cereals or whole grain. More specifically, the granular starch may be obtained from corn, cobs, wheat, barley, rye, triticale, milo, sago, millet, cassava, tapioca, sorghum, rice, peas, bean, banana, or potatoes. Corn contains about 60-68% starch; barley contains about 55-65% starch; millet contains about 75-80% starch; wheat contains about 60-65% starch; and polished rice contains 70-72% starch. Specifically contemplated starch substrates are corn starch and wheat starch. The starch from a grain may be ground or whole and includes corn solids, such as kernels, bran and/or cobs. The starch may also be highly refined raw starch or feedstock from starch refinery processes. Various starches also are commercially available. For example, corn starch is available from Cerestar, Sigma, and Katayama Chemical Industry Co. (Japan); wheat starch is available from Sigma; sweet potato starch is available from Wako Pure Chemical Industry Co. (Japan); and potato starch is available from Nakaari Chemical Pharmaceutical Co. (Japan).

The starch substrate can be a crude starch from milled whole grain, which contains non-starch fractions, *e.g.*, germ residues and fibers. Milling may comprise either wet milling or dry milling or grinding. In wet milling, whole grain is soaked in water or dilute acid to separate the grain into its component parts, *e.g.*, starch, protein, germ, oil, kernel fibers. Wet milling efficiently separates the germ and meal (*i.e.*, starch granules and protein) and is especially suitable for production of syrups. In dry milling or grinding, whole kernels are ground into a fine powder and often processed without fractionating the grain into its component parts. In some cases, oils from the kernels are recovered. Dry ground grain thus will comprise significant amounts of non-starch carbohydrate compounds, in addition to starch. Dry grinding of the starch substrate can be used for production of ethanol and other biochemicals. The starch to be processed may be a highly refined starch quality, for example, at least 90%, at least 95%, at least 97%, or at least 99.5% pure.

### 4.2. Gelatinization and Liquefaction of Starch

As used herein, the term "liquefaction" or "liquefy" means a process by which starch is converted to less viscous and shorter chain dextrins. Generally, this process involves gelatinization of starch simultaneously with or followed by the addition of an α-amylase, although additional liquefaction-inducing enzymes optionally may be added. In some embodiments, the starch substrate prepared as described above is slurried with water. The starch slurry may contain starch as a weight percent of dry solids of about 10-55%, about 20-45%, about 30-45%, about 30-40%, or about 30-35%. α-Amylase (EC 3.2.1.1) may be added to the slurry, with a metering pump, for example. The α-amylase typically used for this application is a thermally stable, bacterial α-amylase, such as a *Geobacillus stearothermophilus* α-amylase. The α-amylase is usually supplied, for example, at about 1500 units per kg dry matter of starch. To optimize α-amylase stability and activity, the pH of the slurry typically is adjusted to about pH 5.5-6.5 and about 1 mM of calcium (about 40 ppm free calcium ions) can also be added. *Geobacillus stearothermophilus* variants or other α-amylases may require different conditions. Bacterial α-amylase remaining in the slurry following liquefaction may be deactivated via a number of methods, including lowering the pH in a subsequent reaction step or by removing calcium from the slurry in cases where the enzyme is dependent upon calcium.

The slurry of starch plus the α-amylase may be pumped continuously through a jet cooker, which is steam heated to 105°C. Gelatinization occurs rapidly under these conditions, and the enzymatic activity, combined with the significant shear forces, begins the hydrolysis of the starch substrate. The residence time in the jet cooker is brief. The partly gelatinized starch may be passed into a series of holding tubes maintained at 105-110°C and held for 5-8 min. to complete the gelatinization process ("primary liquefaction"). Hydrolysis to the required DE is completed in holding tanks at 85-95°C or higher temperatures for about 1 to 2 hours ("secondary liquefaction"). These tanks may contain baffles to discourage back mixing. As used herein, the term "minutes of secondary liquefaction" refers to the time that has elapsed from the start of secondary liquefaction to the time that the Dextrose Equivalent (DE) is measured. The slurry is then allowed to cool to room temperature. This cooling step can be 30 minutes to 180 minutes, *e.g.* 90 minutes to 120 minutes. The liquefied starch typically is in the form of a slurry having a dry solids content (w/w) of about 10-50%; about 10-45%; about 15-40%; about 20-40%; about 25-40%; or about 25-35%.

Liquefaction with α-amylases advantageously can be conducted at low pH, eliminating the requirement to adjust the pH to about pH 5.5-6.5. α-amylases can be used for liquefaction at a pH range of 2 to 7, *e.g.*, pH 3.0 - 7.5, pH 4.0 - 6.0, or pH 4.5 - 5.8. α-amylases can maintain liquefying activity at a temperature range of about 85°C - 95°C, *e.g.*, 85°C, 90°C, or 95°C. For example, liquefaction can be conducted with 800 μg an amylase in a solution of 25% DS corn starch for 10 min at pH 5.8 and 85°C, or pH 4.5 and 95°C, for example. Liquefying activity can be assayed using any of a number of known viscosity assays in the art.

In particular embodiments using the present a-amylases, startch liquifaction is performed at a temperature range of 90-115°C, for the purpose of producing high-purity glucose syrups, HFCS, maltodextrins, etc.

### 4.3. Saccharification

The liquefied starch can be saccharified into a syrup rich in lower DP (*e.g.*, DP1 + DP2) saccharides, using a-amylases, optionally in the presence of another enzyme(s). The exact composition of the products of saccharification depends on the combination of enzymes used, as well as the type of granular starch processed. Advantageously, the syrup obtainable using the provided α-amylases may contain a weight percent of DP2 of the total oligosaccharides in the saccharified starch exceeding 30%, *e.g.*, 45% - 65% or 55% - 65%. The weight percent of (DPI + DP2) in the saccharified starch may exceed about 70%, *e.g.,* 75% - 85% or 80% - 85%. The present amylases also produce a relatively high yield of glucose, *e.g.,* DP1 > 20%, in the syrup product.

Whereas liquefaction is generally run as a continuous process, saccharification is often conducted as a batch process. Saccharification typically is most effective at temperatures of about 60-65°C and a pH of about 4.0-4.5, *e.g.,* pH 4.3, necessitating cooling and adjusting the pH of the liquefied starch. Saccharification may be performed, for example, at a temperature between about 40°C, about 50°C, or about 55°C to about 60°C or about 65°C. Saccharification is normally conducted in stirred tanks, which may take several hours to fill or empty. Enzymes typically are added either at a fixed ratio to dried solids as the tanks are filled or added as a single dose at the commencement of the filling stage. A saccharification reaction to make a syrup typically is run over about 24-72 hours, for example, 24-48 hours. When a maximum or desired DE has been attained, the reaction is stopped by heating to 85°C for 5 min., for example. Further incubation will result in a lower DE, eventually to about 90 DE, as accumulated glucose re-polymerizes to isomaltose and/or other reversion products via an enzymatic reversion reaction and/or with the approach of thermodynamic equilibrium. When using an amylase, saccharification optimally is conducted at a temperature range of about 30°C to about 75°C, *e.g.,* 45°C - 75°C or 47°C - 74°C. The saccharifying may be conducted over a pH range of about pH 3 to about pH 7, *e.g.,* pH 3.0 - pH 7.5, pH 3.5 - pH 5.5, pH 3.5, pH 3.8, or pH 4.5.

An amylase may be added to the slurry in the form of a composition. Amylase can be added to a slurry of a granular starch substrate in an amount of about 0.6 - 10 ppm ds, *e.g.,* 2 ppm ds. An amylase can be added as a whole broth, clarified, enriched, partially purified, or purified enzyme. The specific activity of the amylase may be about 300 U/mg of enzyme, for example, measured with the PAHBAH assay. The amylase also can be added as a whole broth product.

An amylase may be added to the slurry as an isolated enzyme solution. For example, an amylase can be added in the form of a cultured cell material produced by host cells expressing an amylase. An amylase may also be secreted by a host cell into the reaction medium during the fermentation or SSF process, such that the enzyme is provided continuously into the reaction. The host cell producing and secreting amylase may also express an additional enzyme, such as a glucoamylase. For example, U.S. Patent No. 5,422,267 discloses the use of a glucoamylase in yeast for production of alcoholic beverages. For example, a host cell, *e.g., Trichoderma reesei* or *Aspergillus niger,* may be engineered to co-express an amylase and a glucoamylase, *e.g.,* HgGA, TrGA, or a TrGA variant, during saccharification. The host cell can be genetically modified so as not to express its endogenous glucoamylase and/or other enzymes, proteins or other materials. The host cell can be engineered to express a broad spectrum of various saccharolytic enzymes. For example, the recombinant yeast host cell can comprise nucleic acids encoding a glucoamylase, an alpha-glucosidase, an enzyme that utilizes pentose sugar, an $\alpha$-amylase, a pullulanase, an isoamylase, and/or an isopullulanase. *See, e.g.,* WO 2011/153516 A2.

### 4.4. Isomerization

The soluble starch hydrolysate produced by treatment with amylase can be converted into high fructose starch-based syrup (HFSS), such as high fructose corn syrup (HFCS). This conversion can be achieved using a glucose isomerase, particularly a glucose isomerase immobilized on a solid support. The pH is increased to about 6.0 to about 8.0, *e.g.,* pH 7.5 (depending on the isomerase), and $Ca^{2+}$ is removed by ion exchange. Suitable isomerases include SWEETZYME®, IT (Novozymes A/S); G-ZYME® IMGI, and G-ZYME® G993, KETOMAX®, G-ZYME® G993, G-ZYME® G993 liquid, and GENSWEET® IGI. Following isomerization, the mixture typically contains about 40-45% fructose, *e.g.,* 42% fructose.

### 4.5. Fermentation

The soluble starch hydrolysate, particularly a glucose rich syrup, can be fermented by contacting the starch hydrolysate with a fermenting organism typically at a temperature around 32°C, such as from 30°C to 35°C for alcohol-producing yeast. The temperature and pH of the fermentation will depend upon the fermenting organism. EOF products include metabolites, such as citric acid, lactic acid, succinic acid, monosodium glutamate, gluconic acid, sodium gluconate, calcium gluconate, potassium gluconate, itaconic acid and other carboxylic acids, glucono delta-lactone, sodium erythorbate, lysine and other amino acids, omega 3 fatty acid, butanol, isoprene, 1,3-propanediol and other biomaterials.

Ethanologenic microorganisms include yeast, such as *Saccharomyces cerevisiae* and bacteria, *e.g., Zymomonas moblis,* expressing alcohol dehydrogenase and pyruvate decarboxylase. The ethanologenic microorganism can express xylose reductase and xylitol dehydrogenase, which convert xylose to xylulose. Improved strains of ethanologenic microorganisms, which can withstand higher temperatures, for example, are known in the art and can be used. *See* Liu et al. (2011) Sheng Wu Gong Cheng Xue Bao 27:1049-56. Commercial sources of yeast include ETHANOL RED® (LeSaffre); THERMOSACC® (Lallemand); RED STAR® (Red Star); FERMIOL® (DSM Specialties); and SUPERSTART® (Alltech). Microorganisms that produce other metabolites, such as citric acid and lactic acid, by fermentation are also known in the art. *See, e.g.,* Papagianni (2007) Biotechnol. Adv. 25:244-63; John et al. (2009) Biotechnol. Adv. 27:145-52.

The saccharification and fermentation processes may be carried out as an SSF process. Fermentation may comprise subsequent enrichment purification, and recovery of ethanol, for example. During the fermentation, the ethanol content of the broth or "beer" may reach about 8-18% v/v, *e.g.,* 14-15% v/v. The broth may be distilled to produce enriched, *e.g.,* 96% pure, solutions of ethanol. Further, $CO_2$ generated by fermentation may be collected with a $CO_2$ scrubber, compressed, and marketed for other uses, *e.g.*, carbonating beverage or dry ice production. Solid waste from the fermentation process may be used as protein-rich products, *e.g.*, livestock feed.

As mentioned above, an SSF process can be conducted with fungal cells that express and secrete amylase continuously throughout SSF. The fungal cells expressing amylase also can be the fermenting microorganism, *e.g.*, an ethanologenic microorganism. Ethanol production thus can be carried out using a fungal cell that expresses sufficient amylase so that less or no enzyme has to be added exogenously. The fungal host cell can be from an appropriately engineered fungal strain. Fungal host cells that express and secrete other enzymes, in addition to amylase, also can be used. Such cells may express glucoamylase and/or a pullulanase, phytase, *alpha*-glucosidase, isoamylase, beta-amylase cellulase, xylanase, other hemicellulases, protease, *beta*-glucosidase, pectinase, esterase, redox enzymes, transferase, or other enzyme.

A variation on this process is a "fed-batch fermentation" system, where the substrate is added in increments as the fermentation progresses. Fed-batch systems are useful when catabolite repression may inhibit the metabolism of the cells and where it is desirable to have limited amounts of substrate in the medium. The actual substrate concentration in fed-batch systems is estimated by the changes of measurable factors such as pH, dissolved oxygen and the partial pressure of waste gases, such as $CO_2$. Batch and fed-batch fermentations are common and well known in the art.

Continuous fermentation is an open system where a defined fermentation medium is added continuously to a bioreactor, and an equal amount of conditioned medium is removed simultaneously for processing. Continuous fermentation generally maintains the cultures at a constant high density where cells are primarily in log phase growth. Continuous fermentation permits modulation of cell growth and/or product concentration. For example, a limiting nutrient such as the carbon source or nitrogen source is maintained at a fixed rate and all other parameters are allowed to moderate. Because growth is maintained at a steady state, cell loss due to medium being drawn off should be balanced against the cell growth rate in the fermentation. Methods of optimizing continuous fermentation processes and maximizing the rate of product formation are well known in the art of industrial microbiology.

## 4.6. Compositions Comprising α-Amylases

α-amylases may be combined with a glucoamylase (EC 3.2.1.3), *e.g.,* a *Trichoderma* glucoamylase or variant thereof. An exemplary glucoamylase is *Trichoderma reesei* glucoamylase (TrGA) and variants thereof that possess superior specific activity and thermal stability. *See* U.S. Published Applications Nos. 2006/0094080, 2007/0004018, and 2007/0015266 (Danisco US Inc.). Suitable variants of TrGA include those with glucoamylase activity and at least 80%, at least 90%, or at least 95% sequence identity to wild-type TrGA. α-amylases advantageously increase the yield of glucose produced in a saccharification process catalyzed by TrGA.

Alternatively, the glucoamylase may be another glucoamylase derived from plants (including algae), fungi, or bacteria. For example, the glucoamylases may be *Aspergillus niger* G1 or G2 glucoamylase or its variants (*e.g.,* Boel et al. (1984) EMBO J. 3:1097-1102; WO 92/00381; WO 00/04136 (Novo Nordisk A/S)); and *A. awamori* glucoamylase *(e.g.,* WO 84/02921 (Cetus Corp.)). Other contemplated *Aspergillus* glucoamylase include variants with enhanced thermal stability, *e.g.,* G137A and G139A (Chen et al. (1996) Prot. Eng. 9:499-505); D257E and D293E/Q (Chen et al. (1995) Prot. Eng. 8:575-582); N182 (Chen et al. (1994) Biochem. J. 301:275-281); A246C (Fierobe et al. (1996) Biochemistry, 35: 8698-8704); and variants with Pro residues in positions A435 and S436 (Li et al. (1997) Protein Eng. 10:1199-1204). Other contemplated glucoamylases include *Talaromyces* glucoamylases, in particular derived from *T. emersonii (e.g.,* WO 99/28448 (Novo Nordisk A/S), *T. leycettanus (e.g.,* U.S. Patent No. RE 32,153 (CPC International, Inc.)), *T. duponti,* or *T. thermophilus (e.g.,* U.S. Patent No. 4,587,215). Contemplated bacterial glucoamylases include glucoamylases from the genus *Clostridium,* in particular *C. thermoamylolyticum (e.g.,* EP 135138 (CPC International, Inc.) and *C. thermohydrosulfuricum (e.g.,* WO 86/01831 (Michigan Biotechnology Institute)). Suitable glucoamylases include the glucoamylases derived from *Aspergillus oryzae,* such as a glucoamylase shown in SEQ ID NO:2 in WO 00/04136 (Novo Nordisk A/S). Also suitable are commercial glucoamylases, such as AMG 200L; AMG 300 L; SAN™ SUPER and AMG™ E (Novozymes); OPTIDEX® 300 and OPTIDEX L-400 (Danisco US Inc.); AMIGASE™ and AMIGASE™ PLUS (DSM); G-ZYME® G900 (Enzyme Bio-Systems); and G-ZYME® G990 ZR (A *niger* glucoamylase with a low protease content). Still other suitable glucoamylases include *Aspergillus fumigatus* glucoamylase, *Talaromyces* glucoamylase, *Thielavia* glucoamylase, *Trametes* glucoamylase, *Thermomyces* glucoamylase, *Athelia* glucoamylase, or *Humicola* glucoamylase (*e.g.,* HgGA). Glucoamylases typically are added in an amount of about 0.1 - 2 glucoamylase units (GAU)/g ds, *e.g.*, about 0.16 GAU/g ds, 0.23 GAU/g ds, or 0.33 GAU/g ds.

Other suitable enzymes that can be used with amylase include a phytase, protease, pullulanase, β-amylase, isoamylase, a different α-amylase, alpha-glucosidase, cellulase, xylanase, other hemicellulases, beta-glucosidase, transferase, pectinase, lipase, cutinase, esterase, redox enzymes, or a combination thereof. For example, a debranching enzyme, such as an isoamylase (EC 3.2.1.68), may be added in effective amounts well known to the person skilled in the art. A pullulanase (EC 3.2.1.41), *e.g.*, PROMOZYME®, is also suitable. Pullulanase typically is added at 100 U/kg ds. Further suitable enzymes include proteases, such as fungal and bacterial proteases. Fungal proteases include those obtained from *Aspergillus,* such as A. *niger, A. awamori, A. oryzae; Mucor* (*e.g., M. miehei*); *Rhizopus;* and *Trichoderma.*

β-Amylases (EC 3.2.1.2) are exo-acting maltogenic amylases, which catalyze the hydrolysis of 1,4-α-glucosidic linkages into amylopectin and related glucose polymers, thereby releasing maltose. β-Amylases have been isolated from various plants and microorganisms. *See* Fogarty et al. (1979) in PROGRESS IN INDUSTRIAL MICROBIOLOGY, Vol. 15, pp. 112-115. These β-Amylases have optimum temperatures in the range from 40°C to 65°C and optimum pH in the range from about 4.5 to about 7.0. Contemplated β-amylases include, but are not limited to, β-amylases from barley SPEZYME® BBA 1500, SPEZYME® DBA, OPTIMALT™ ME, OPTIMALT™ BBA (Danisco US Inc.); and NOVOZYM™ WBA (Novozymes A/S).

Compositions comprising the present amylases may be aqueous or non-aqueous formulations, granules, powders, gels, slurries, pastes, etc., which may further comprise any one or more of the additional enzymes listed, herein, along with buffers, salts, preservatives, water, co-solvents, surfactants, and the like. Such compositions may work in combination with endogenous enzymes or other ingredients already present in a slurry, water bath, washing machine, food or drink product, etc, for example, endogenous plant (including algal) enzymes, residual enzymes from a prior processing step, and the like.

**5. Compositions and Methods for Baking and Food Preparation**

The present invention also relates to a "food composition," including but not limited to a food product, animal feed and/or food/feed additives, comprising an amylase, and methods for preparing such a food composition comprising mixing α-amylase with one or more food ingredients, or uses thereof.

Furthermore, the present invention relates to the use of an amylase in the preparation of a food composition, wherein the food composition is baked subsequent to the addition of the polypeptide of the invention. As used herein the term "baking composition" means any composition and/or additive prepared in the process of providing a baked food product, including but not limited to bakers flour, a dough, a baking additive and/or a baked product. The food composition or additive may be liquid or solid.

As used herein, the term "flour" means milled or ground cereal grain. The term "flour" also may mean Sago or tuber products that have been ground or mashed. In some embodiments, flour may also contain components in addition to the milled or mashed cereal or plant matter. An example of an additional component, although not intended to be limiting, is a leavening agent. Cereal grains include wheat, oat, rye, and barley. Tuber products include tapioca flour, cassava flour, and custard powder. The term "flour" also includes ground corn flour, maize-meal, rice flour, whole-meal flour, self-rising flour, tapioca flour, cassava flour, ground rice, enriched flower, and custard powder.

For the commercial and home use of flour for baking and food production, it is important to maintain an appropriate level of α-amylase activity in the flour. A level of activity that is too high may result in a product that is sticky and/or doughy and therefore unmarketable. Flour with insufficient α-amylase activity may not contain enough sugar for proper yeast function, resulting in dry, crumbly bread, or baked products. Accordingly, an amylase, by itself or in combination with another α-amylase(s), may be added to the flour to augment the level of endogenous α-amylase activity in flour.

An amylase can further be added alone or in a combination with other amylases to prevent or retard staling, *i.e.,* crumb firming of baked products. The amount of anti-staling amylase will typically be in the range of 0.01-10 mg of enzyme protein per kg of flour, *e.g.,* 0.5 mg/kg ds. Additional anti-staling amylases that can be used in combination with an amylase include an endo-amylase, *e.g.,* a bacterial endo-amylase from *Bacillus.* The additional amylase can be another maltogenic α-amylase (EC 3.2.1.133), *e.g.,* from *Bacillus.* NOVAMYL® is an exemplary maltogenic α-amylase from *B. stearothermophilus* strain NCIB 11837 and is described in Christophersen et al. (1997) Starch 50:39-45. Other examples of anti-staling endo-amylases include bacterial α-amylases derived from *Bacillus,* such as *B. licheniformis* or *B. amyloliquefaciens.* The anti-staling amylase may be an exo-amylase, such as β-amylase, *e.g.,* from plant sources, such as soy bean, or from microbial sources, such as *Bacillus.*

The baking composition comprising an amylase further can comprise a phospholipase or enzyme with phospholipase activity. An enzyme with phospholipase activity has an activity that can be measured in Lipase Units (LU). The phospholipase may have $A_1$ or $A_2$ activity to remove fatty acid from the phospholipids, forming a lysophospholipid. It may or may not have lipase activity, *i.e.,* activity on triglyceride substrates. The phospholipase typically has a temperature optimum in the range of 30-90°C., *e.g.,* 30-70°C. The added phospholipases can be of animal origin, for example, from pancreas, *e.g.,* bovine or porcine pancreas, snake venom or bee venom. Alternatively, the phospholipase may be of microbial origin, *e.g.,* from filamentous fungi, yeast or bacteria, for example.

The phospholipase is added in an amount that improves the softness of the bread during the initial period after baking, particularly the first 24 hours. The amount of phospholipase will typically be in the range of 0.01-10 mg of enzyme protein per kg of flour, *e.g.,* 0.1-5 mg/kg. That is, phospholipase activity generally will be in the range of 20-1000 LU/kg of flour, where a Lipase Unit is defined as the amount of enzyme required to release 1 μmol butyric acid per minute at 30°C, pH 7.0, with gum arabic as emulsifier and tributyrin as substrate.

Compositions of dough generally comprise wheat meal or wheat flour and/or other types of meal, flour or starch such as corn flour, cornstarch, rye meal, rye flour, oat flour, oatmeal, soy flour, sorghum meal, sorghum flour, potato meal, potato flour or potato starch. The dough may be fresh, frozen or par-baked. The dough can be a leavened dough or a dough to be subjected to leavening. The dough may be leavened in various ways, such as by adding chemical leavening agents, *e.g.,* sodium bicarbonate or by adding a leaven, *i.e.,* fermenting dough. Dough also may be leavened by adding a suitable yeast culture, such as a culture of *Saccharomyces cerevisiae* (baker's yeast), *e.g.,* a commercially available strain of *S. cerevisiae.*

The dough may also comprise other conventional dough ingredients, *e.g.,* proteins, such as milk powder, gluten, and soy; eggs *(e.g.,* whole eggs, egg yolks or egg whites); an oxidant, such as ascorbic acid, potassium bromate, potassium iodate, azodicarbonamide (ADA) or ammonium persulfate; an amino acid such as L-cysteine; a sugar; or a salt, such as sodium chloride, calcium acetate, sodium sulfate or calcium sulfate. The dough further may comprise fat, *e.g.,* triglyceride, such as granulated fat or shortening. The dough further may comprise an emulsifier such as mono- or diglycerides, diacetyl tartaric acid esters of mono- or diglycerides, sugar esters of fatty acids, polyglycerol esters of fatty acids, lactic acid esters of monoglycerides, acetic acid esters of monoglycerides, polyoxyethylene stearates, or lysolecithin. In particular, the dough can be made without addition of emulsifiers.

The dough product may be any processed dough product, including fried, deep fried, roasted, baked, steamed and boiled doughs, such as steamed bread and rice cakes. In one embodiment, the food product is a bakery product. Typical bakery (baked) products include bread - such as loaves, rolls, buns, bagels, pizza bases etc. pastry, pretzels, tortillas, cakes, cookies, biscuits, crackers etc.

Optionally, an additional enzyme may be used together with the anti-staling amylase and the phospholipase. The additional enzyme may be a second amylase, such as an amyloglucosidase, a β-amylase, a cyclodextrin glucanotransferase, or the additional enzyme may be a peptidase, in particular an exopeptidase, a transglutaminase, a lipase, a cellulase, a xylanase, a protease, a protein disulfide isomerase, *e.g.,* a protein disulfide isomerase as disclosed in WO 95/00636, for example, a glycosyltransferase, a branching enzyme (1,4-α-glucan branching enzyme), a 4-α-glucanotransferase (dextrin glycosyltransferase) or an oxidoreductase, *e.g.,* a peroxidase, a laccase, a glucose oxidase, an amadoriase, a metalloproteinase, a pyranose oxidase, a lipooxygenase, an L-amino acid oxidase or a carbohydrate oxidase. The additional enzyme(s) may be of any origin, including mammalian and plant, and particularly of microbial (bacterial, yeast or fungal) origin and may be obtained by techniques conventionally used in the art.

The xylanase is typically of microbial origin, *e.g.,* derived from a bacterium or fungus, such as a strain of *Aspergillus.* Xylanases include PENTOPAN® and NOVOZYM 384®, for example, which are commercially available xylanase preparations produced from *Trichoderma reesei.* The amyloglucosidase may be an *A. niger* amyloglucosidase (such as AMG®). Other useful amylase products include GRINDAMYL® A 1000 or A 5000 (Grindsted Products, Denmark) and AMYLASE H™ or AMYLASE P™ (DSM). The glucose oxidase may be a fungal glucose oxidase, in particular an *Aspergillus niger* glucose oxidase (such as GLUZYME®). An exemplary protease is NEUTRASE®.

The process may be used for any kind of baked product prepared from dough, either of a soft or a crisp character, either of a white, light or dark type. Examples are bread, particularly white, whole-meal or rye bread, typically in the form of loaves or rolls, such as, but not limited to, French baguette-type bread, pita bread, tortillas, cakes, pancakes, biscuits, cookies, pie crusts, crisp bread, steamed bread, pizza and the like.

An amylase may be used in a pre-mix, comprising flour together with an anti-staling amylase, a phospholipase, and/or a phospholipid. The pre-mix may contain other dough-improving and/or bread-improving additives, *e.g.,* any of the additives, including enzymes, mentioned above. An amylase can be a component of an enzyme preparation comprising an anti-staling amylase and a phospholipase, for use as a baking additive.

The enzyme preparation is optionally in the form of a granulate or agglomerated powder. The preparation can have a narrow particle size distribution with more than 95% (by weight) of the particles in the range from 25 to 500 μm. Granulates and agglomerated powders may be prepared by conventional methods, *e.g.,* by spraying an amylase onto a carrier in a fluid-bed granulator. The carrier may consist of particulate cores having a suitable particle size. The carrier may be soluble or insoluble, *e.g.,* a salt (such as NaCl or sodium sulfate), a sugar (such as sucrose or lactose), a sugar alcohol (such as sorbitol), starch, rice, corn grits, or soy.

Enveloped particles, *i.e.,* α-amylase particles, can comprise an amylase. To prepare enveloped α-amylase particles, the enzyme is contacted with a food grade lipid in sufficient quantity to suspend all of the α-amylase particles. Food grade lipids, as used herein, may be any naturally organic compound that is insoluble in water but is soluble in non-polar organic solvents such as hydrocarbon or diethyl ether. Suitable food grade lipids include, but are not limited to, triglycerides either in the form of fats or oils that are either saturated or unsaturated. Examples of fatty acids and combinations thereof which make up the saturated triglycerides include, but are not limited to, butyric (derived from milk fat), palmitic (derived from animal and plant fat), and/or stearic (derived from animal and plant fat). Examples of fatty acids and combinations thereof which make up the unsaturated triglycerides include, but are not limited to, palmitoleic (derived from animal and plant fat), oleic (derived from animal and plant fat), linoleic (derived from plant oils), and/or linolenic (derived from linseed oil). Other suitable food grade lipids include, but are not limited to, monoglycerides and diglycerides derived from the triglycerides discussed above, phospholipids and glycolipids.

The food grade lipid, particularly in the liquid form, is contacted with a powdered form of the $\alpha$-amylase particles in such a fashion that the lipid material covers at least a portion of the surface of at least a majority, *e.g.*, 100% of the $\alpha$-amylase particles. Thus, each $\alpha$-amylase particle is individually enveloped in a lipid. For example, all or substantially all of the $\alpha$-amylase particles are provided with a thin, continuous, enveloping film of lipid. This can be accomplished by first pouring a quantity of lipid into a container, and then slurrying the $\alpha$-amylase particles so that the lipid thoroughly wets the surface of each $\alpha$-amylase particle. After a short period of stirring, the enveloped $\alpha$-amylase particles, carrying a substantial amount of the lipids on their surfaces, are recovered. The thickness of the coating so applied to the particles of $\alpha$-amylase can be controlled by selection of the type of lipid used and by repeating the operation in order to build up a thicker film, when desired.

The storing, handling and incorporation of the loaded delivery vehicle can be accomplished by means of a packaged mix. The packaged mix can comprise the enveloped $\alpha$-amylase. However, the packaged mix may further contain additional ingredients as required by the manufacturer or baker. After the enveloped $\alpha$-amylase has been incorporated into the dough, the baker continues through the normal production process for that product.

The advantages of enveloping the $\alpha$-amylase particles are two-fold. First, the food grade lipid protects the enzyme from thermal denaturation during the baking process for those enzymes that are heat labile. Consequently, while the $\alpha$-amylase is stabilized and protected during the proving and baking stages, it is released from the protective coating in the final baked good product, where it hydrolyzes the glucosidic linkages in polyglucans. The loaded delivery vehicle also provides a sustained release of the active enzyme into the baked good. That is, following the baking process, active $\alpha$-amylase is continually released from the protective coating at a rate that counteracts, and therefore reduces the rate of, staling mechanisms.

In general, the amount of lipid applied to the $\alpha$-amylase particles can vary from a few percent of the total weight of the $\alpha$-amylase to many times that weight, depending upon the nature of the lipid, the manner in which it is applied to the $\alpha$-amylase particles, the composition of the dough mixture to be treated, and the severity of the dough-mixing operation involved.

The loaded delivery vehicle, *i.e.,* the lipid-enveloped enzyme, is added to the ingredients used to prepare a baked good in an effective amount to extend the shelf-life of the baked good. The baker computes the amount of enveloped $\alpha$-amylase, prepared as discussed above, that will be required to achieve the desired anti-staling effect. The amount of the enveloped $\alpha$-amylase required is calculated based on the concentration of enzyme enveloped and on the proportion of $\alpha$-amylase to flour specified. A wide range of concentrations has been found to be effective, although, as has been discussed, observable improvements in anti-staling do not correspond linearly with the $\alpha$-amylase concentration, but above certain minimal levels, large increases in $\alpha$-amylase concentration produce little additional improvement. The $\alpha$-amylase concentration actually used in a particular bakery production could be much higher than the minimum necessary to provide the baker with some insurance against inadvertent under-measurement errors by the baker. The lower limit of enzyme concentration is determined by the minimum anti-staling effect the baker wishes to achieve.

A method of preparing a baked good may comprise: a) preparing lipid-coated $\alpha$-amylase particles, where substantially all of the $\alpha$-amylase particles are coated; b) mixing a dough containing flour; c) adding the lipid-coated $\alpha$-amylase to the dough before the mixing is complete and terminating the mixing before the lipid coating is removed from the $\alpha$-amylase; d) proofing the dough; and e) baking the dough to provide the baked good, where the $\alpha$-amylase is inactive during the mixing, proofing and baking stages and is active in the baked good.

The enveloped $\alpha$-amylase can be added to the dough during the mix cycle, *e.g.,* near the end of the mix cycle. The enveloped $\alpha$-amylase is added at a point in the mixing stage that allows sufficient distribution of the enveloped $\alpha$-amylase throughout the dough; however, the mixing stage is terminated before the protective coating becomes stripped from the $\alpha$-amylase particle(s). Depending on the type and volume of dough, and mixer action and speed, anywhere from one to six minutes or more might be required to mix the enveloped $\alpha$-amylase into the dough, but two to four minutes is average. Thus, several variables may determine the precise procedure. First, the quantity of enveloped $\alpha$-amylase should have a total volume sufficient to allow the enveloped $\alpha$-amylase to be spread throughout the dough mix. If the preparation of enveloped $\alpha$-amylase is highly concentrated, additional oil may need to be added to the pre-mix before the enveloped $\alpha$-amylase is added to the dough. Recipes and production processes may require specific modifications; however, good results generally can be achieved when 25% of the oil specified in a bread dough formula is held out of the dough and is used as a carrier for a concentrated enveloped $\alpha$-amylase when added near the end of the mix cycle. In bread or other baked goods, particularly those having a low fat content, *e.g.*, French-style breads, an enveloped $\alpha$-amylase mixture of approximately 1 % of the dry flour weight is sufficient to admix the enveloped $\alpha$-amylase properly

with the dough. The range of suitable percentages is wide and depends on the formula, finished product, and production methodology requirements of the individual baker. Second, the enveloped $\alpha$-amylase suspension should be added to the mix with sufficient time for complete mixture into the dough, but not for such a time that excessive mechanical action strips the protective lipid coating from the enveloped $\alpha$-amylase particles.

In a further aspect of the invention, the food composition is an oil, meat, lard, composition comprising an amylase. In this context the term "oil, meat, lard, composition" means any composition, based on, made from and/or containing oil, meat or lard, respectively. Another aspect the invention relates to a method of preparing an oil or meat or lard composition and/or additive comprising an amylase, comprising mixing the polypeptide of the invention with a oil/meat/lard composition and/or additive ingredients.

In a further aspect of the invention, the food composition is an animal feed composition, animal feed additive and/or pet food comprising an amylase and variants thereof. The present invention further relates to a method for preparing such an animal feed composition, animal feed additive composition and/or pet food comprising mixing an amylase and variants thereof with one or more animal feed ingredients and/or animal feed additive ingredients and/or pet food ingredients. Furthermore, the present invention relates to the use of an amylase in the preparation of an animal feed composition and/or animal feed additive composition and/or pet food.

The term "animal" includes all non-ruminant and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment the animal is a ruminant animal including, but not limited to, cattle, young calves, goats, sheep, giraffes, bison, moose, elk, yaks, water buffalo, deer, camels, alpacas, llamas, antelope, pronghorn and nilgai.

In the present context, it is intended that the term "pet food" is understood to mean a food for a household animal such as, but not limited to dogs, cats, gerbils, hamsters, chinchillas, fancy rats, guinea pigs; avian pets, such as canaries, parakeets, and parrots; reptile pets, such as turtles, lizards and snakes; and aquatic pets, such as tropical fish and frogs.

The terms "animal feed composition," "feedstuff" and "fodder" are used interchangeably and may comprise one or more feed materials selected from the group comprising a) cereals, such as small grains (*e.g.*, wheat, barley, rye, oats and combinations thereof) and/or large grains such as maize or sorghum; b) by products from cereals, such as corn gluten meal, Distillers Dried Grain Solubles (DDGS) (particularly corn based Distillers Dried Grain Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

## 6. Textile Desizing Compositions and Use

Also contemplated are compositions and methods of treating fabrics (*e.g.*, to desize a textile) using an amylase. Fabric-treating methods are well known in the art (*see, e.g.,* U.S. Patent No. 6,077,316). For example, the feel and appearance of a fabric can be improved by a method comprising contacting the fabric with an amylase in a solution. The fabric can be treated with the solution under pressure.

An amylase can be applied during or after the weaving of a textile, or during the desizing stage, or one or more additional fabric processing steps. During the weaving of textiles, the threads are exposed to considerable mechanical strain. Prior to weaving on mechanical looms, warp yarns are often coated with sizing starch or starch derivatives to increase their tensile strength and to prevent breaking. An amylase can be applied during or after the weaving to remove these sizing starch or starch derivatives. After weaving, an amylase can be used to remove the size coating before further processing the fabric to ensure a homogeneous and wash-proof result.

An amylase can be used alone or with other desizing chemical reagents and/or desizing enzymes to desize fabrics, including cotton-containing fabrics, as detergent additives, *e.g.,* in aqueous compositions. An amylase also can be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of amylolytic enzymes to provide softness to the fabric and make the cotton more accessible to the subsequent enzymatic finishing steps. An amylase can be used in methods of finishing denim garments (*e.g.,* a "bio-stoning process"), enzymatic desizing and providing softness to fabrics, and/or finishing process.

**7. Cleaning Compositions**

An aspect of the present compositions and methods is a cleaning composition that includes an amylase as a component. An amylase polypeptide can be used as a component in detergent compositions for hand washing, laundry washing, dishwashing, and other hard-surface cleaning.

**7.1. Overview**

Preferably, an amylase is incorporated into detergents at or near a concentration conventionally used for amylase in detergents. For example, an amylase polypeptide may be added in amount corresponding to 0.00001 - 1 mg (calculated as pure enzyme protein) of amylase per liter of wash/dishwash liquor. Exemplary formulations are provided herein, as exemplified by the following:

An amylase polypeptide may be a component of a detergent composition, as the only enzyme or with other enzymes including other amylolytic enzymes. As such, it may be included in the detergent composition in the form of a non-dusting granulate, a stabilized liquid, or a protected enzyme. Non-dusting granulates may be produced, *e.g.*, as disclosed in U.S. Patent Nos. 4,106,991 and 4,661,452 and may optionally be coated by methods known in the art. Examples of waxy coating materials are poly(ethylene oxide) products (polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB 1483591. Liquid enzyme preparations may, for instance, be stabilized by adding a polyol such as propylene glycol, a sugar or sugar alcohol, lactic acid or boric acid according to established methods. Other enzyme stabilizers are known in the art. Protected enzymes may be prepared according to the method disclosed in for example EP 238 216. Polyols have long been recognized as stabilizers of proteins, as well as improving protein solubility.

The detergent composition may be in any useful form, *e.g.,* as powders, granules, pastes, or liquid. A liquid detergent may be aqueous, typically containing up to about 70% of water and 0% to about 30% of organic solvent. It may also be in the form of a compact gel type containing only about 30% water.

The detergent composition comprises one or more surfactants, each of which may be anionic, nonionic, cationic, or zwitterionic. The detergent will usually contain 0% to about 50% of anionic surfactant, such as linear alkylbenzenesulfonate (LAS); $\alpha$-olefinsulfonate (AOS); alkyl sulfate (fatty alcohol sulfate) (AS); alcohol ethoxysulfate (AEOS or AES); secondary alkanesulfonates (SAS); $\alpha$-sulfo fatty acid methyl esters; alkyl- or alkenylsuccinic acid; or soap. The composition may also contain 0% to about 40% of nonionic surfactant such as alcohol ethoxylate (AEO or AE), carboxylated alcohol ethoxylates, nonylphenol ethoxylate, alkylpolyglycoside, alkyldimethylamineoxide, ethoxylated fatty acid monoethanolamide, fatty acid monoethanolamide, or polyhydroxy alkyl fatty acid amide (as described for example in WO 92/06154).

The detergent composition may additionally comprise one or more other enzymes, such as proteases, another amylolytic enzyme, cutinase, lipase, cellulase, pectate lyase, perhydrolase, xylanase, peroxidase, and/or laccase in any combination.

The detergent may contain about 1% to about 65% of a detergent builder or complexing agent such as zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (*e.g.*, SKS-6 from Hoechst). The detergent may also be unbuilt, *i.e.* essentially free of detergent builder. The enzymes can be used in any composition compatible with the stability of the enzyme. Enzymes generally can be protected against deleterious components by known forms of encapsulation, for example, by granulation or sequestration in hydro gels. Enzymes, and specifically amylases, either with or without starch binding domains, can be used in a variety of compositions including laundry and dishwashing applications, surface cleaners, as well as in compositions for ethanol production from starch or biomass.

The detergent may comprise one or more polymers. Examples include carboxymethylcellulose (CMC), poly(vinylpyrrolidone) (PVP), polyethyleneglycol (PEG), poly(vinyl alcohol) (PVA), polycarboxylates such as polyacrylates, maleic/acrylic acid copolymers and lauryl methacrylate/acrylic acid copolymers.

The detergent may contain a bleaching system, which may comprise a $H_2O_2$ source such as perborate or percarbonate, which may be combined with a peracid-forming bleach activator such as tetraacetylethylenediamine (TAED) or nonanoyloxybenzenesulfonate (NOBS). Alternatively, the bleaching system may comprise peroxyacids (*e.g.*, the amide, imide, or sulfone type peroxyacids). The bleaching system can also be an enzymatic bleaching system, for example, perhydrolase, such as that described in International PCT Application WO 2005/056783.

The enzymes of the detergent composition may be stabilized using conventional stabilizing agents, *e.g.,* a polyol such as propylene glycol or glycerol; a sugar or sugar alcohol; lactic acid; boric acid or a boric acid derivative such as, *e.g.,* an aromatic borate ester; and the composition may be formulated as described in, *e.g.,* WO 92/19709 and WO 92/19708.

The detergent may also contain other conventional detergent ingredients such as *e.g.,* fabric conditioners including clays, foam boosters, suds suppressors, anti-corrosion agents, soil-suspending agents, anti-soil redeposition agents, dyes, bactericides, tarnish inhibiters, optical brighteners, or perfumes.

The pH (measured in aqueous solution at use concentration) is usually neutral or alkaline, *e.g.*, pH about 7.0 to about 11.0.

Particular forms of detergent compositions for inclusion of the present $\alpha$-amylase are described, below.

**7.2. Heavy Duty Liquid (HDL) laundry detergent composition**

Exemplary HDL laundry detergent compositions includes a detersive surfactant (10%-40% wt/wt), including an anionic detersive surfactant (selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof), and optionally non-ionic surfactant (selected from a group of linear or branched or random chain, substituted or unsubstituted alkyl alkoxylated alcohol, for example a $C_8$-$C_{18}$ alkyl ethoxylated alcohol and/or $C_6$-$C_{12}$ alkyl phenol alkoxylates), wherein the weight ratio of anionic detersive surfactant (with a hydrophilic index (HIc) of from 6.0 to 9) to non-ionic detersive surfactant is greater than 1: 1. Suitable detersive surfactants also include cationic detersive surfactants (selected from a group of alkyl pyridinium compounds, alkyl quarternary ammonium compounds, alkyl quarternary phosphonium compounds, alkyl ternary sulphonium compounds, and/or mixtures thereof); zwitterionic and/or amphoteric detersive surfactants (selected from a group of alkanolamine sulpho-betaines); ampholytic surfactants; semi-polar non-ionic surfactants and mixtures thereof.

The composition may optionally include, a surfactancy boosting polymer consisting of amphiphilic alkoxylated grease cleaning polymers (selected from a group of alkoxylated polymers having branched hydrophilic and hydrophobic properties, such as alkoxylated polyalkylenimines in the range of 0.05 wt%-10 wt%) and/or random graft polymers (typically comprising of hydrophilic backbone comprising monomers selected from the group consisting of: unsaturated $C_1$-$C_6$ carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: $C_4$-$C_{25}$ alkyl group, polypropylene, polybutylene, vinyl ester of a saturated $C_1$-$C_6$ mono-carboxylic acid, $C_1$-$C_6$ alkyl ester of acrylic or methacrylic acid, and mixtures thereof.

The composition may include additional polymers such as soil release polymers (include anionically end-capped polyesters, for example SRP1, polymers comprising at least one monomer unit selected from saccharide, dicarboxylic acid, polyol and combinations thereof, in random or block configuration, ethylene terephthalate-based polymers and co-polymers thereof in random or block configuration, for example Repel-o-tex SF, SF-2 and SRP6, Texcare SRA100, SRA300, SRN100, SRN170, SRN240, SRN300 and SRN325, Marloquest SL), antiredeposition polymers (0.1 wt% to 10 wt%, include carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof, vinylpyrrolidone homopolymer, and/or polyethylene glycol, molecular weight in the range of from 500 to 100,000 Da); cellulosic polymer (including those selected from alkyl cellulose, alkyl alkoxyalkyl cellulose, carboxyalkyl cellulose, alkyl carboxyalkyl cellulose examples of which include carboxymethyl cellulose, methyl cellulose, methyl hydroxyethyl cellulose, methyl carboxymethyl cellulose, and mixures thereof) and polymeric carboxylate (such as maleate/acrylate random copolymer or polyacrylate homopolymer).

The composition may further include saturated or unsaturated fatty acid, preferably saturated or unsaturated $C_{12}$-$C_{24}$ fatty acid (0 wt% to 10 wt%); deposition aids (examples for which include polysaccharides, preferably cellulosic polymers, poly diallyl dimethyl ammonium halides (DADMAC), and co-polymers of DAD MAC with vinyl pyrrolidone, acrylamides, imidazoles, imidazolinium halides, and mixtures thereof, in random or block configuration, cationic guar gum, cationic cellulose such as cationic hydoxyethyl cellulose, cationic starch, cationic polyacylamides, and mixtures thereof.

The composition may further include dye transfer inhibiting agents, examples of which include manganese phthalocyanine, peroxidases, polyvinylpyrrolidone polymers, polyamine N-oxide polymers, copolymers of N-vinylpyrrolidone and N-vinylimidazole, polyvinyloxazolidones and polyvinylimidazoles and/or mixtures thereof; chelating agents, examples of which include ethylene-diamine-tetraacetic acid (EDTA), diethylene triamine penta methylene phosphonic acid (DTPMP), hydroxy-ethane diphosphonic acid (HEDP), ethylenediamine N,N'-disuccinic acid (EDDS), methyl glycine diacetic acid (MGDA), diethylene triamine penta acetic acid (DTPA), propylene diamine tetracetic acid (PDT A), 2-hydroxypyridine-N-oxide (HPNO), or methyl glycine diacetic acid (MGDA), glutamic acid N,N-diacetic acid (N,N-dicarboxymethyl glutamic acid tetrasodium salt (GLDA), nitrilotriacetic acid (NTA), 4,5-dihydroxy-m-benzenedisulfonic acid, citric acid and any salts thereof, N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), and derivatives thereof.

The composition preferably included enzymes (generally about 0.01 wt% active enzyme to 0.03 wt% active enzyme) selected from proteases, amylases, lipases, cellulases, choline oxidases, peroxidases/oxidases, pectate lyases, mannanases, cutinases, laccases, phospholipases, lysophospholipases, acyltransferases, perhydrolases, arylesterases, and any mixture thereof. The composition may include an enzyme stabilizer (examples of which include polyols such as propylene glycol or glycerol, sugar or sugar alcohol, lactic acid, reversible protease inhibitor, boric acid, or a boric acid derivative, *e.g.*, an aromatic borate ester, or a phenyl boronic acid derivative such as 4-formylphenyl boronic acid).

The composition optionally include silicone or fatty-acid based suds suppressors; heuing dyes, calcium and magnesium cations, visual signaling ingredients, anti-foam (0.001 wt% to about 4.0 wt%), and/or structurant/thickener (0.01 wt% to 5 wt%, selected from the group consisting of diglycerides and triglycerides, ethylene glycol distearate, microcrystalline cellulose, cellulose based materials, microfiber cellulose, biopolymers, xanthan gum, gellan gum, and mixtures thereof).

The composition can be any liquid form, for example a liquid or gel form, or any combination thereof. The composition may be in any unit dose form, for example a pouch.

## 7.3. Heavy Duty Dry/Solid (HDD) laundry detergent composition

Exemplary HDD laundry detergent compositions includes a detersive surfactant, including anionic detersive surfactants (*e.g.*, linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant (*e.g.,* linear or branched or random chain, substituted or unsubstituted $C_8$-$C_{18}$ alkyl ethoxylates, and/or $C_6$-$C_{12}$ alkyl phenol alkoxylates), cationic detersive surfactants (*e.g.*, alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (*e.g.*, alkanolamine sulpho-betaines), ampholytic surfactants, semi-polar non-ionic surfactants, and mixtures thereof; builders including phosphate free builders (for example zeolite builders examples which include zeolite A, zeolite X, zeolite P and zeolite MAP in the range of 0 wt% to less than 10 wt%), phosphate builders (for example sodium tri-polyphosphate in the range of 0 wt% to less than 10 wt%), citric acid, citrate salts and nitrilotriacetic acid, silicate salt (*e.g.*, sodium or potassium silicate or sodium meta-silicate in the range of 0 wt% to less than 10 wt%, or layered silicate (SKS-6)); carbonate salt (*e.g.*, sodium carbonate and/or sodium bicarbonate in the range of 0 wt% to less than 80 wt%); and bleaching agents including photobleaches (*e.g.*, sulfonated zinc phthalocyanines, sulfonated aluminum phthalocyanines, xanthenes dyes, and mixtures thereof) hydrophobic or hydrophilic bleach activators (*e.g.*, dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacetyl ethylene diamine-TAED, nonanoyloxybenzene sulfonate-NOBS, nitrile quats, and mixtures thereof), sources of hydrogen peroxide (*e.g.*, inorganic perhydrate salts examples of which include mono or tetra hydrate sodium salt of perborate, percarbonate, persulfate, perphosphate, or persilicate), preformed hydrophilic and/or hydrophobic peracids (*e.g.*, percarboxylic acids and salts, percarbonic acids and salts, perimidic acids and salts, peroxymonosulfuric acids and salts, and mixtures thereof), and/or bleach catalysts (*e.g.*, imine bleach boosters (examples of which include iminium cations and polyions), iminium zwitterions, modified amines, modified amine oxides, N-sulphonyl imines, N-phosphonyl imines, N-acyl imines, thiadiazole dioxides, perfluoroimines, cyclic sugar ketones, and mixtures thereof, and metal-containing bleach catalysts (*e.g.*, copper, iron, titanium, ruthenium, tungsten, molybdenum, or manganese cations along with an auxiliary metal cations such as zinc or aluminum and a sequestrate such as ethylenediaminetetraacetic acid, ethylenediaminetetra(methylenephosphonic acid), and watersoluble salts thereof).

The composition preferably includes enzymes, *e.g.*, proteases, amylases, lipases, cellulases, choline oxidases, peroxidases/oxidases, pectate lyases, mannanases, cutinases, laccases, phospholipases, lysophospholipases, acyltransferase, perhydrolase, arylesterase, and any mixture thereof.

The composition may optionally include additional detergent ingredients including perfume microcapsules, starch encapsulated perfume accord, hueing agents, additional polymers, including fabric integrity and cationic polymers, dye-lock ingredients, fabric-softening agents, brighteners (for example C.I. Fluorescent brighteners), flocculating agents, chelating agents, alkoxylated polyamines, fabric deposition aids, and/or cyclodextrin.

## 7.4. Automatic dishwashing (ADW) detergent composition

Exemplary ADW detergent composition includes non-ionic surfactants, including ethoxylated non-ionic surfactants, alcohol alkoxylated surfactants, epoxy-capped poly(oxyalkylated) alcohols, or amine oxide surfactants present in amounts from 0 to 10% by weight; builders in the range of 5-60% including phosphate builders (*e.g.*, mono-phosphates, diphosphates, tri-polyphosphates, other oligomeric-poylphosphates, sodium tripolyphosphate-STPP) and phosphate-free builders (*e.g.*, amino acid-based compounds including methylglycine-diacetic acid (MGDA) and salts and derivatives thereof, glutamic-N,N-diacetic acid (GLDA) and salts and derivatives thereof, iminodisuccinic acid (IDS) and salts and derivatives thereof, carboxy methyl inulin and salts and derivatives thereof, nitrilotriacetic acid (NTA), diethylene triamine penta acetic acid (DTPA), B-alaninediacetic acid (B-ADA) and their salts, homopolymers and copolymers of poly-carboxylic acids and their partially or completely neutralized salts, monomeric polycarboxylic acids and hydroxycarboxylic acids and their salts in the range of 0.5% to 50% by weight; sulfonated/carboxylated polymers in the range of about 0.1 % to about 50% by weight to to provide dimensional stability; drying aids in the range of about 0.1 % to about 10% by weight (*e.g.,* polyesters, especially anionic polyesters, optionally together with further monomers with 3 to 6 functionalities - typically acid, alcohol or ester functionalities which are conducive to polycondensation, polycarbonate-, polyurethane- and/or polyurea-polyorganosiloxane compounds or precursor compounds, thereof, particularly of the reactive cyclic carbonate and urea type); silicates in the range from about 1 % to about 20% by weight (including sodium or potassium silicates for example sodium disilicate, sodium meta-silicate and crystalline phyllosilicates); inorganic bleach (*e.g.,* perhydrate salts such as perborate, percarbonate, perphosphate, persulfate and persilicate salts) and organic bleach (*e.g.,* organic peroxyacids, including diacyl and tetraacylperoxides, especially diperoxydodecanedioc acid, diperoxytetrade-canedioc acid, and diperoxyhexadecanedioic acid); bleach activators (*i.e.,* organic peracid precursors in the range from about 0.1 % to about 10% by weight); bleach catalysts (*e.g.,* manganese triazacyclononane and related complexes, Co, Cu, Mn, and Fe bispyridylamine and related complexes, and pentamine acetate cobalt(III) and related complexes); metal care agents in the range from about 0.1% to 5% by weight (*e.g.,* benzatriazoles, metal salts and complexes, and/or silicates); enzymes in the range from about 0.01 to 5.0 mg of active enzyme per gram of automatic dishwashing detergent composition (*e.g.,* proteases, amylases, lipases, cellulases, choline oxidases, peroxidases/oxidases, pectate lyases, mannanases, cutinases, laccases, phospholipases, lysophospholipases, acyltransferase, perhydrolase, arylesterase, and mixtures thereof); and enzyme stabilizer components (*e.g.,* oligosaccharides, polysaccharides, and inorganic divalent metal salts).

### 7.5. Additional detergent compositions

Additional exemplary detergent formulations to which the present amylase can be added are described, below, in the numbered paragraphs.

1) A detergent composition formulated as a granulate having a bulk density of at least 600 g/L comprising linear alkylbenzenesulfonate (calculated as acid) about 7% to about 12%; alcohol ethoxysulfate (*e.g.,* $C_{12-18}$ alcohol, 1-2 ethylene oxide (EO)) or alkyl sulfate (*e.g.,* $C_{16-18}$) about 1% to about 4%; alcohol ethoxylate (*e.g.,* $C_{14-15}$ alcohol, 7 EO) about 5% to about 9%; sodium carbonate (*e.g.,* $Na_2CO_3$) about 14% to about 20%; soluble silicate (*e.g.,* $Na_2O$, $2SiO_2$) about 2 to about 6%; zeolite (*e.g.,* $NaAlSiO_4$) about 15% to about 22%; sodium sulfate (*e.g.,* $Na_2SO_4$) 0% to about 6%; sodium citrate/citric acid (*e.g.,* $C_6H_5Na_3O_7/C_6H_8O_7$) about 0% to about 15%; sodium perborate (*e.g.,* $NaBO_3H_2O$) about 11% to about 18%; TAED about 2% to about 6%; carboxymethylcellulose (CMC) and 0% to about 2%; polymers (*e.g.,* maleic/acrylic acid, copolymer, PVP, PEG) 0-3%; enzymes (calculated as pure enzyme) 0.0001-0.1% protein; and minor ingredients (*e.g.,* suds suppressors, perfumes, optical brightener, photobleach) 0-5%.

2) A detergent composition formulated as a granulate having a bulk density of at least 600 g/L comprising linear alkylbenzenesulfonate (calculated as acid) about 6% to about 11%; alcohol ethoxysulfate (*e.g.,* $C_{12-18}$ alcohol, 1-2 EO) or alkyl sulfate (*e.g.,* $C_{16-18}$) about 1% to about 3%; alcohol ethoxylate (*e.g.,* $C_{14-15}$ alcohol, 7 EO) about 5% to about 9%; sodium carbonate (*e.g.,* $Na_2CO_3$) about 15% to about 21%; soluble silicate (*e.g.,* $Na_2O$, $2SiO_2$) about 1% to about 4%; zeolite (*e.g.,* $NaAlSiO_4$) about 24% to about 34%; sodium sulfate (*e.g.,.* $Na_2SO_4$) about 4% to about 10%; sodium citrate/citric acid (*e.g.,* $C_6H_5Na_3O_7/C_6H_8O_7$) 0% to about 15%; carboxymethylcellulose (CMC) 0% to about 2%; polymers (*e.g.,* maleic/acrylic acid copolymer, PVP, PEG) 1-6%; enzymes (calculated as pure enzyme protein) 0.0001-0.1%; minor ingredients (*e.g.,* suds suppressors, perfume) 0-5%.

3) A detergent composition formulated as a granulate having a bulk density of at least 600 g/L comprising linear alkylbenzenesulfonate (calculated as acid) about 5% to about 9%; alcohol ethoxylate (*e.g.,* $C_{12-15}$ alcohol, 7 EO) about 7% to about 14%; Soap as fatty acid (*e.g.,* $C_{16-22}$ fatty acid) about 1 to about 3%; sodium carbonate (as $Na_2CO_3$) about 10% to about 17%; soluble silicate (*e.g.,* $Na_2O$, $2SiO_2$) about 3% to about 9%; zeolite (as $NaAlSiO_4$) about 23% to about 33%; sodium sulfate (*e.g.,* $Na_2SO_4$) 0% to about 4%; sodium perborate (*e.g.,* $NaBO_3H_2O$) about 8% to about 16%; TAED about 2% to about 8%; phosphonate (*e.g.,* EDTMPA) 0% to about 1%; carboxymethylcellulose (CMC) 0% to about 2%; polymers (*e.g.,* maleic/acrylic acid copolymer, PVP, PEG) 0-3%; enzymes (calculated as pure enzyme protein) 0.0001-0.1%; minor ingredients (*e.g.,* suds suppressors, perfume, optical brightener) 0-5%.

4) A detergent composition formulated as a granulate having a bulk density of at least 600 g/L comprising linear alkylbenzenesulfonate (calculated as acid) about 8% to about 12%; alcohol ethoxylate (*e.g.,* $C_{12-15}$ alcohol, 7 EO) about 10% to about 25%; sodium carbonate (as $Na_2CO_3$) about 14% to about 22%; soluble silicate (*e.g.,* $Na_2O$, $2SiO_2$) about 1% to about 5%; zeolite (*e.g.,* $NaAlSiO_4$) about 25% to about 35%; sodium sulfate (*e.g.,* $Na_2SO_4$) 0% to about 10%; carboxymethylcellulose (CMC) 0% to about 2%; polymers (*e.g.,* maleic/acrylic acid copolymer, PVP, PEG) 1-3%; enzymes (calculated as pure enzyme protein) 0.0001-0.1%; and minor ingredients (*e.g.,* suds suppressors, perfume) 0-5%.

5) An aqueous liquid detergent composition comprising linear alkylbenzenesulfonate (calculated as acid) about 15% to about 21%; alcohol ethoxylate (*e.g.,* $C_{12-15}$ alcohol, 7 EO or $C_{12-15}$ alcohol, 5 EO) about 12% to about 18%; soap as fatty acid (*e.g.,* oleic acid) about 3% to about 13%; alkenylsuccinic acid ($C_{12-14}$) 0% to about 13%; aminoethanol about 8% to about 18%; citric acid about 2% to about 8%; phosphonate 0% to about 3%; polymers (*e.g.,* PVP, PEG) 0% to about 3%; borate (*e.g.,* $B_4O_7$) 0% to about 2%; ethanol 0% to about 3%; propylene glycol about 8% to about 14%; enzymes (calculated as pure enzyme protein) 0.0001-0.1%; and minor ingredients (*e.g.,* dispersants, suds suppressors, perfume, optical brightener) 0-5%.

6) An aqueous structured liquid detergent composition comprising linear alkylbenzenesulfonate (calculated as acid) about 15% to about 21%; alcohol ethoxylate (*e.g.,* $C_{12-15}$ alcohol, 7 EO, or $C_{12-15}$ alcohol, 5 EO) 3-9%; soap as fatty acid (*e.g.,* oleic acid) about 3% to about 10%; zeolite (as $NaAlSiO_4$) about 14% to about 22%; potassium citrate about 9% to about 18%; borate (*e.g.,* $B_4O_7$) 0% to about 2%; carboxymethylcellulose (CMC) 0% to about 2%; polymers (*e.g.,* PEG, PVP) 0% to about 3%; anchoring polymers such as, *e.g.,* lauryl methacrylate/acrylic acid copolymer; molar ratio 25:1, MW 3800) 0% to about 3%; glycerol 0% to about 5%; enzymes (calculated as pure enzyme protein) 0.0001-0.1%; and minor ingredients (e.g., dispersants, suds suppressors, perfume, optical brighteners) 0-5%.

7) A detergent composition formulated as a granulate having a bulk density of at least 600 g/L comprising fatty alcohol sulfate about 5% to about 10%; ethoxylated fatty acid monoethanolamide about 3% to about 9%; soap as fatty acid 0-3%; sodium carbonate *(e.g.,* $Na_2CO_3$) about 5% to about 10%; Soluble silicate (*e.g.,* $Na_2O$, $2SiO_2$) about 1% to about 4%; zeolite (*e.g.,* $NaAlSiO_4$) about 20% to about 40%; Sodium sulfate (*e.g.,* $Na_2SO_4$) about 2% to about 8%; sodium perborate (*e.g.,* $NaBO_3H_2O$) about 12% to about 18%; TAED about 2% to about 7%; polymers *(e.g.,* maleic/acrylic acid copolymer, PEG) about 1% to about 5%; enzymes (calculated as pure enzyme protein) 0.0001-0.1%; and minor ingredients (*e.g.,* optical brightener, suds suppressors, perfume) 0-5%.

8) A detergent composition formulated as a granulate comprising linear alkylbenzenesulfonate (calculated as acid) about 8% to about 14%; ethoxylated fatty acid monoethanolamide about 5% to about 11%; soap as fatty acid 0% to about 3%; sodium carbonate (*e.g.,* $Na_2CO_3$) about 4% to about 10%; soluble silicate ($Na_2O$, $2SiO_2$) about 1% to about 4%; zeolite (*e.g.,* $NaAlSiO_4$) about 30% to about 50%; sodium sulfate (*e.g.,* $Na_2SO_4$) about 3% to about 11%; sodium citrate (*e.g.,* $C_6H_5Na_3O_7$) about 5% to about 12%; polymers (*e.g.,* PVP, maleic/acrylic acid copolymer, PEG) about 1% to about 5%; enzymes (calculated as pure enzyme protein) 0.0001-0.1%; and minor ingredients (*e.g.,* suds suppressors, perfume) 0-5%.

9) A detergent composition formulated as a granulate comprising linear alkylbenzenesulfonate (calculated as acid) about 6% to about 12%; nonionic surfactant about 1% to about 4%; soap as fatty acid about 2% to about 6%; sodium carbonate (*e.g.,* $Na_2CO_3$) about 14% to about 22%; zeolite (*e.g.,* $NaAlSiO_4$) about 18% to about 32%; sodium sulfate (*e.g.,* $Na_2SO_4$) about 5% to about 20%; sodium citrate (*e.g.,* $C_6H_5Na_3O_7$) about 3% to about 8%; sodium perborate (*e.g.,* $NaBO_3H_2O$) about 4% to about 9%; bleach activator (*e.g.,* NOBS or TAED) about 1% to about 5%; carboxymethylcellulose (CMC) 0% to about 2%; polymers (*e.g.,* polycarboxylate or PEG) about 1% to about 5%; enzymes (calculated as pure enzyme protein) 0.0001-0.1%; and minor ingredients (*e.g.,* optical brightener, perfume) 0-5%.

10) An aqueous liquid detergent composition comprising linear alkylbenzenesulfonate (calculated as acid) about 15% to about 23%; alcohol ethoxysulfate (*e.g.,* $C_{12-15}$ alcohol, 2-3 EO) about 8% to about 15%; alcohol ethoxylate (*e.g.,* $C_{12-15}$ alcohol, 7 EO, or $C_{12-15}$ alcohol, 5 EO) about 3% to about 9%; soap as fatty acid (*e.g.,* lauric acid) 0% to about 3%; aminoethanol about 1% to about 5%; sodium citrate about 5% to about 10%; hydrotrope (*e.g.,* sodium toluensulfonate) about 2% to about 6%; borate (*e.g.,* $B_4O_7$) 0% to about 2%; carboxymethylcellulose 0% to about 1%; ethanol about 1% to about 3%; propylene glycol about 2% to about 5%; enzymes (calculated as pure enzyme protein) 0.0001-0.1%; and minor ingredients (*e.g.,* polymers, dispersants, perfume, optical brighteners) 0-5%.

11) An aqueous liquid detergent composition comprising linear alkylbenzenesulfonate (calculated as acid) about 20% to about 32%; alcohol ethoxylate (*e.g.*, $C_{12-15}$ alcohol, 7 EO, or $C_{12-15}$ alcohol, 5 EO) 6-12%; aminoethanol about 2% to about 6%; citric acid about 8% to about 14%; borate (*e.g.*, $B_4O_7$) about 1% to about 3%; polymer (*e.g.,* maleic/acrylic acid copolymer, anchoring polymer such as, *e.g.,* lauryl methacrylate/acrylic acid copolymer) 0% to about 3%; glycerol about 3% to about 8%; enzymes (calculated as pure enzyme protein) 0.0001-0.1%; and minor ingredients (*e.g.*, hydrotropes, dispersants, perfume, optical brighteners) 0-5%.

12) A detergent composition formulated as a granulate having a bulk density of at least 600 g/L comprising anionic surfactant (linear alkylbenzenesulfonate, alkyl sulfate, $\alpha$-olefinsulfonate, $\alpha$-sulfo fatty acid methyl esters, alkanesulfonates, soap) about 25% to about 40%; nonionic surfactant (*e.g.*, alcohol ethoxylate) about 1% to about 10%; sodium carbonate (*e.g.*, $Na_2CO_3$) about 8% to about 25%; soluble silicates (*e.g.*, $Na_2O, 2SiO_2$) about 5% to about 15%; sodium sulfate (*e.g.*, $Na_2SO_4$) 0% to about 5%; zeolite ($NaAlSiO_4$) about 15% to about 28%; sodium perborate (*e.g.*, $NaBO_3 \cdot 4H_2O$) 0% to about 20%; bleach activator (TAED or NOBS) about 0% to about 5%; enzymes (calculated as pure enzyme protein) 0.0001-0.1%; minor ingredients (*e.g.,* perfume, optical brighteners) 0-3%.

13) Detergent compositions as described in compositions 1)-12) *supra,* wherein all or part of the linear alkylbenzenesulfonate is replaced by ($C_{12}$-$C_{18}$) alkyl sulfate.

14) A detergent composition formulated as a granulate having a bulk density of at least 600 g/L comprising ($C_{12}$-$C_{18}$) alkyl sulfate about 9% to about 15%; alcohol ethoxylate about 3% to about 6%; polyhydroxy alkyl fatty acid amide about 1% to about 5%; zeolite (*e.g.,* $NaAlSiO_4$) about 10% to about 20%; layered disilicate (*e.g.*, SK56 from Hoechst) about 10% to about 20%; sodium carbonate (*e.g.*, $Na_2CO_3$) about 3% to about 12%; soluble silicate (*e.g.,* $Na_2O, 2SiO_2$) 0% to about 6%; sodium citrate about 4% to about 8%; sodium percarbonate about 13% to about 22%; TAED about 3% to about 8%; polymers (*e.g.*, polycarboxylates and PVP) 0% to about 5%; enzymes (calculated as pure enzyme protein) 0.0001-0.1%; and minor ingredients (*e.g.,* optical brightener, photobleach, perfume, suds suppressors) 0-5%.

15) A detergent composition formulated as a granulate having a bulk density of at least 600 g/L comprising ($C_{12}$-$C_{18}$) alkyl sulfate about 4% to about 8%; alcohol ethoxylate about 11% to about 15%; soap about 1% to about 4%; zeolite MAP or zeolite A about 35% to about 45%; sodium carbonate (as $Na_2CO_3$) about 2% to about 8%; soluble silicate (*e.g.*, $Na_2O, 2SiO_2$) 0% to about 4%; sodium percarbonate about 13% to about 22%; TAED 1-8%; carboxymethylcellulose (CMC) 0% to about 3%; polymers (*e.g.,* polycarboxylates and PVP) 0% to about 3%; enzymes (calculated as pure enzyme protein) 0.0001-0.1%; and minor ingredients (*e.g.*, optical brightener, phosphonate, perfume) 0-3%.

16) Detergent formulations as described in 1)-15) supra, which contain a stabilized or encapsulated peracid, either as an additional component or as a substitute for already specified bleach systems.

17) Detergent compositions as described supra in 1), 3), 7), 9), and 12), wherein perborate is replaced by percarbonate.

18) Detergent compositions as described supra in 1), 3), 7), 9), 12), 14), and 15), which additionally contain a manganese catalyst. The manganese catalyst for example is one of the compounds described in "Efficient manganese catalysts for low-temperature bleaching," Nature 369: 637-639 (1994).

19) Detergent composition formulated as a non-aqueous detergent liquid comprising a liquid nonionic surfactant such as, *e.g.,* linear alkoxylated primary alcohol, a builder system (*e.g.*, phosphate), an enzyme(s), and alkali. The detergent may also comprise anionic surfactant and/or a bleach system.

As above, the present amylase polypeptide may be incorporated at a concentration conventionally employed in detergents. It is at present contemplated that, in the detergent composition, the enzyme may be added in an amount corresponding to 0.00001-1.0 mg (calculated as pure enzyme protein) of amylase polypeptide per liter of wash liquor.

The detergent composition may also contain other conventional detergent ingredients, *e.g.*, deflocculant material, filler material, foam depressors, anti-corrosion agents, soil-suspending agents, sequestering agents, anti-soil redeposition agents, dehydrating agents, dyes, bactericides, fluorescers, thickeners, and perfumes.

The detergent composition may be formulated as a hand (manual) or machine (automatic) laundry detergent composition, including a laundry additive composition suitable for pre-treatment of stained fabrics and a rinse added fabric softener composition, or be formulated as a detergent composition for use in general household hard surface cleaning operations, or be formulated for manual or automatic dishwashing operations.

Any of the cleaning compositions described, herein, may include any number of additional enzymes. In general the enzyme(s) should be compatible with the selected detergent, *(e.g.,* with respect to pH-optimum, compatibility with other enzymatic and non-enzymatic ingredients, and the like), and the enzyme(s) should be present in effective amounts. The following enzymes are provided as examples.

*Proteases:* Suitable proteases include those of animal, vegetable or microbial origin. Chemically modified or protein engineered mutants are included, as well as naturally processed proteins. The protease may be a serine protease or a metalloprotease, an alkaline microbial protease, a trypsin-like protease, or a chymotrypsin-like protease. Examples of alkaline proteases are subtilisins, especially those derived from *Bacillus, e.g.,* subtilisin Novo, subtilisin Carlsberg, subtilisin 309, subtilisin 147, and subtilisin 168 *(see, e.g.,* WO 89/06279). Examples of trypsin-like proteases are trypsin *(e.g.,* of porcine or bovine origin), and *Fusarium* proteases *(see, e.g.,* WO 89/06270 and WO 94/25583). Examples of useful proteases also include but are not limited to the variants described in WO 92/19729, WO 98/20115, WO 98/20116, and WO 98/34946. Commercially available protease enzymes include but are not limited to: ALCALASE®, SAVINASE®, PRIMASE™, DURALASE™, ESPERASE®, KANNASE™, and BLAZE™ (Novo Nordisk A/S and Novozymes A/S); MAXATASE®, MAXACAL™, MAXAPEM™, PROPERASE®, PURAFECT®, PURAFECT OXP™, FN2™, and FN3™ (Danisco US Inc.). Other exemplary proteases include NprE from *Bacillus amyloliquifaciens* and ASP from *Cellulomonas* sp. strain 69B4.

*Lipases*: Suitable lipases include those of bacterial or fungal origin. Chemically modified, proteolytically modified, or protein engineered mutants are included. Examples of useful lipases include but are not limited to lipases from *Humicola* (synonym *Thermomyces*), *e.g.,* from *H. lanuginosa* (*T. lanuginosus*) (*see e.g.,* EP 258068 and EP 305216), from *H. insolens* (*see e.g.,* WO 96/13580); a *Pseudomonas* lipase *(e.g.,* from *P. alcaligenes* or *P. pseudoalcaligenes; see, e.g.,* EP 218 272), *P. cepacia* (*see e.g.,* EP 331 376), *P. stutzeri* (*see e.g.,* GB 1,372,034), *P. fluorescens, Pseudomonas* sp. strain SD 705 (*see e.g.,* WO 95/06720 and WO 96/27002), *P. wisconsinensis* (*see e.g.,* WO 96/12012); a *Bacillus* lipase *(e.g.,* from *B. subtilis; see e.g.,* Dartois et al. Biochemica et Biophysica Acta, 1131: 253-360 (1993)), *B. stearothermophilus* (*see e.g.,* JP 64/744992), or *B. pumilus* (*see e.g.,* WO 91/16422). Additional lipase variants contemplated for use in the formulations include those described for example in: WO 92/05249, WO 94/01541, WO 95/35381, WO 96/00292, WO 95/30744, WO 94/25578, WO 95/14783, WO 95/22615, WO 97/04079, WO 97/07202, EP 407225, and EP 260105. Some commercially available lipase enzymes include LIPOLASE® and LIPOLASE ULTRA™ (Novo Nordisk A/S and Novozymes A/S).

*Polyesterases*: Suitable polyesterases can be included in the composition, such as those described in, for example, WO 01/34899, WO 01/14629, and US6933140.

Amylases: The compositions can be combined with other amylases, such as non-production enhanced amylase. These can include commercially available amylases, such as but not limited to STAINZYME®, NATALASE®, DURAMYL®, TERMAMYL®, FUNGAMYL® and BAN™ (Novo Nordisk A/S and Novozymes A/S); RAPIDASE®, POWERASE®, and PURASTAR® (from Danisco US Inc.).

*Cellulases:* Cellulases can be added to the compositions. Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed for example in U.S. Patent Nos. 4,435,307; 5,648,263; 5,691,178; 5,776,757; and WO 89/09259. Exemplary cellulases contemplated for use are those having color care benefit for the textile. Examples of such cellulases are cellulases described in for example EP 0495257, EP 0531372, WO 96/11262, WO 96/29397, and WO 98/08940. Other examples are cellulase variants, such as those described in WO 94/07998; WO 98/12307; WO 95/24471; PCT/DK98/00299; EP 531315; U.S. Patent Nos. 5,457,046; 5,686,593; and 5,763,254. Commercially available cellulases include CELLUZYME® and CAREZYME® (Novo Nordisk A/S and Novozymes A/S); CLAZINASE® and PURADAX HA® (Danisco US Inc.); and KAC-500(B)™ (Kao Corporation).

*Peroxidases/Oxidases*: Suitable peroxidases/oxidases contemplated for use in the compositions include those of plant, bacterial or fungal origin. Chemically modified or protein engineered mutants are included. Examples of useful peroxidases include peroxidases from *Coprinus, e.g.,* from *C. cinereus,* and variants thereof as those described in WO 93/24618, WO 95/10602, and WO 98/15257. Commercially available peroxidases include for example GUARDZYME™ (Novo Nordisk A/S and Novozymes A/S).

The detergent composition can also comprise 2,6-β-D-fructan hydrolase, which is effective for removal/cleaning of biofilm present on household and/or industrial textile/laundry.

The detergent enzyme(s) may be included in a detergent composition by adding separate additives containing one or more enzymes, or by adding a combined additive comprising all of these enzymes. A detergent additive, *i.e.* a separate additive or a combined additive, can be formulated *e.g.*, as a granulate, a liquid, a slurry, and the like. Exemplary detergent additive formulations include but are not limited to granulates, in particular non-dusting granulates, liquids, in particular stabilized liquids or slurries.

Non-dusting granulates may be produced, *e.g.,* as disclosed in U.S. Patent Nos. 4,106,991 and 4,661,452 and may optionally be coated by methods known in the art. Examples of waxy coating materials are poly(ethylene oxide) products (*e.g.*, polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition may be in any convenient form, *e.g.*, a bar, a tablet, a powder, a granule, a paste, or a liquid. A liquid detergent may be aqueous, typically containing up to about 70% water, and 0% to about 30% organic solvent. Compact detergent gels containing about 30% or less water are also contemplated. The detergent composition can optionally comprise one or more surfactants, which may be non-ionic, including semi-polar and/or anionic and/or cationic and/or zwitterionic. The surfactants can be present in a wide range, from about 0.1% to about 60% by weight.

When included therein the detergent will typically contain from about 1% to about 40% of an anionic surfactant, such as linear alkylbenzenesulfonate, α-olefinsulfonate, alkyl sulfate (fatty alcohol sulfate), alcohol ethoxysulfate, secondary alkanesulfonate, α-sulfo fatty acid methyl ester, alkyl- or alkenylsuccinic acid, or soap.

When included therein, the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl-N-alkyl derivatives of glucosamine ("glucamides").

The detergent may contain 0% to about 65% of a detergent builder or complexing agent such as zeolite, diphosphate, triphosphate, phosphonate, carbonate, citrate, nitrilotriacetic acid, ethylenediaminetetraacetic acid (EDTA), diethylenetriaminepentaacetic acid, alkyl- or alkenylsuccinic acid, soluble silicates or layered silicates (e.g.,SKS-6 from Hoechst).

The detergent may comprise one or more polymers. Exemplary polymers include carboxymethylcellulose (CMC), poly(vinylpyrrolidone) (PVP), poly(ethylene glycol) (PEG), poly(vinyl alcohol) (PVA), poly(vinylpyridine-N-oxide), poly(vinylimidazole), polycarboxylates *e.g.*, polyacrylates, maleic/acrylic acid copolymers), and lauryl methacrylate/acrylic acid copolymers.

The enzyme(s) of the detergent composition may be stabilized using conventional stabilizing agents, *e.g.,* as polyol (*e.g.,* propylene glycol or glycerol), a sugar or sugar alcohol, lactic acid, boric acid, or a boric acid derivative (*e.g.*, an aromatic borate ester), or a phenyl boronic acid derivative (*e.g.*, 4-formylphenyl boronic acid). The composition may be formulated as described in WO 92/19709 and WO 92/19708.

It is contemplated that in the detergent compositions, in particular the enzyme variants, may be added in an amount corresponding to about 0.01 to about 100 mg of enzyme protein per liter of wash liquor (*e.g.*, about 0.05 to about 5.0 mg of enzyme protein per liter of wash liquor or 0.1 to about 1.0 mg of enzyme protein per liter of wash liquor).

Yet additional exemplary detergent formulations to which the present amylase can be added are described in, *e.g.,* WO2010065455, WO2011072099, WO2011130222, WO2011140364, WO2011156297, WO2011156298, WO2011130076, WO2011133381, WO2011156297, WO2011156298, EP1794295B1, US20110195481, US20110212876, US20110257063, WO2010039958, WO2011072117, WO2011098531, WO2011100410, WO2011130076, WO2011133381, WO2011140316, US20070215184, US20070251545, US20090075857, US20090137444, US20090143271, US20100011513, US20100093588, US20110201536, US20110232004, US20110237482, US20110312868, US20120003326, US20120004155, WO2011131585, EP707628B1, US5719115, EP736084B1, US5783545, EP767830B1, US5972668, EP746599B1, US5798328, EP662117B1, US5898025, US6380140, EP898613B1, US3975280, US6191092, US6329333, US6530386, EP1307547B1, US7153818, EP1421169B1, US6979669, EP1529101B1, US7375070, EP1385943B1, US7888104, EP1414977B1, US5855625, EP1921147B1, EP1921148B1, EP701605B1, EP1633469B1, EP1633470B1, EP1794293B1, EP171007B1, US4692260, US7569226, EP1165737B1, US6391838, US6060441, US2009017074, US7320887, EP1737952B1, US7691618, US20070256251, US20050261156, US20050261158, US20100234267, US20110136720, US20110201536, US7811076, US5929017, US5156773, EP2343310A1, WO2011083114, EP214761B1, US4876024, EP675944B1, US5763383, EP517761B1, US6624129, EP1054956B1, US6939702, US6964944, EP832174B1, US20060205628, US20070179076, US20080023031, US20110015110, US20110028372, US4973417, US5447649, US5840677, US5965503, US5972873, US5998344, US6071356, WO9009428, EP1661978A1, EP1698689A1, EP1726636A1, EP1867707A1, EP1876226A1, EP1876227A1, EP0205208A2, EP0206390A2, EP0271152, EP0271154, EP0341999, EP0346136, EP2135934, US20120208734, WO2011127102, WO2012142087, WO2012145062, EP1790713B1, US8066818B2, US8163686B2, US8283300B2, US8354366B2, US20120125374, US3929678, and US5898025.

## 7.6. Methods of Assessing Amylase Activity in Detergent Compositions

Numerous α-amylase cleaning assays are known in the art, including swatch and micro-swatch assays. The appended Examples describe only a few such assays.

In order to further illustrate the compositions and methods, and advantages thereof, the following specific examples are given with the understanding that they are illustrative rather than limiting.

## 8. Brewing Compositions

The present α-amylase may be a component of a brewing composition used in a process of brewing, *i.e.,* making a fermented malt beverage. Non-fermentable carbohydrates form the majority of the dissolved solids in the final beer. This residue remains because of the inability of malt amylases to hydrolyze the alpha-1,6-linkages of the starch. The non-fermentable carbohydrates contribute about 50 calories per 12 ounces of beer. an amylase, in combination with a glucoamylase and optionally a pullulanase and/or isoamylase, assist in converting the starch into dextrins and fermentable sugars, lowering the residual non-fermentable carbohydrates in the final beer.

The principal raw materials used in making these beverages are water, hops and malt. In addition, adjuncts such as common corn grits, refined corn grits, brewer's milled yeast, rice, sorghum, refined corn starch, barley, barley starch, dehusked barley, wheat, wheat starch, torrified cereal, cereal flakes, rye, oats, potato, tapioca, and syrups, such as corn syrup, sugar cane syrup, inverted sugar syrup, barley and/or wheat syrups, and the like may be used as a source of starch.

For a number of reasons, the malt, which is produced principally from selected varieties of barley, has the greatest effect on the overall character and quality of the beer. First, the malt is the primary flavoring agent in beer. Second, the malt provides the major portion of the fermentable sugar. Third, the malt provides the proteins, which will contribute to the body and foam character of the beer. Fourth, the malt provides the necessary enzymatic activity during mashing. Hops also contribute significantly to beer quality, including flavoring. In particular, hops (or hops constituents) add desirable bittering substances to the beer. In addition, the hops act as protein precipitants, establish preservative agents and aid in foam formation and stabilization.

Grains, such as barley, oats, wheat, as well as plant components, such as corn, hops, and rice, also are used for brewing, both in industry and for home brewing. The components used in brewing may be unmalted or may be malted, *i.e.,* partially germinated, resulting in an increase in the levels of enzymes, including α-amylase. For successful brewing, adequate levels of α-amylase enzyme activity are necessary to ensure the appropriate levels of sugars for fermentation, an amylase, by itself or in combination with another α-amylase(s), accordingly may be added to the components used for brewing.

As used herein, the term "stock" means grains and plant components that are crushed or broken. For example, barley used in beer production is a grain that has been coarsely ground or crushed to yield a consistency appropriate for producing a mash for fermentation. As used herein, the term "stock" includes any of the aforementioned types of plants and grains in crushed or coarsely ground forms. The methods described herein may be used to determine α-amylase activity levels in both flours and stock.

Processes for making beer are well known in the art. *See, e.g.,* Wolfgang Kunze (2004) "Technology Brewing and Malting," Research and Teaching Institute of Brewing, Berlin (VLB), 3rd edition. Briefly, the process involves: (a) preparing a mash, (b) filtering the mash to prepare a wort, and (c) fermenting the wort to obtain a fermented beverage, such as beer. Typically, milled or crushed malt is mixed with water and held for a period of time under controlled temperatures to permit the enzymes present in the malt to convert the starch present in the malt into fermentable sugars. The mash is then transferred to a mash filter where the liquid is separated from the grain residue. This sweet liquid is called "wort," and the left over grain residue is called "spent grain." The mash is typically subjected to an extraction, which involves adding water to the mash in order to recover the residual soluble extract from the spent grain. The wort is then boiled vigorously to sterilizes the wort and help develop the color, flavor and odor. Hops are added at some point during the boiling. The wort is cooled and transferred to a fermentor.

The wort is then contacted in a fermentor with yeast. The fermentor may be chilled to stop fermentation. The yeast flocculates and is removed. Finally, the beer is cooled and stored for a period of time, during which the beer clarifies and its flavor develops, and any material that might impair the appearance, flavor and shelf life of the beer settles out. The beer usually contains from about 2% to about 10% v/v alcohol, although beer with a higher alcohol content, *e.g.,* 18% v/v, may be obtained. Prior to packaging, the beer is carbonated and, optionally, filtered and pasteurized.

The brewing composition comprising an amylase, in combination with a glucoamylase and optionally a pullulanase and/or isoamylase, may be added to the mash of step (a) above, *i.e.,* during the preparation of the mash. Alternatively, or in addition, the brewing composition may be added to the mash of step (b) above, *i.e.,* during the filtration of the mash. Alternatively, or in addition, the brewing composition may be added to the wort of step (c) above, *i.e.,* during the fermenting of the wort.

A fermented beverage, such as a beer, can be produced by one of the methods above. The fermented beverage can be a beer, such as full malted beer, beer brewed under the "Reinheitsgebot," ale, IPA, lager, bitter, Happoshu (second beer), third beer, dry beer, near beer, light beer, low alcohol beer, low calorie beer, porter, bock beer, stout, malt liquor, non-alcoholic beer, non-alcoholic malt liquor and the like, but also alternative cereal and malt beverages such as fruit flavored malt beverages, *e.g.*, citrus flavored, such as lemon-, orange-, lime-, or berry-flavored malt beverages, liquor flavored malt beverages, *e.g.*, vodka-, rum-, or tequila-flavored malt liquor, or coffee flavored malt beverages, such as caffeine-flavored malt liquor, and the like.

## 9. Reduction of Iodine-Positive Starch

α-amylases may reduce the iodine-positive starch (IPS), when used in a method of liquefaction and/or saccharification. One source of IPS is from amylose that escapes hydrolysis and/or from retrograded starch polymer. Starch retrogradation occurs spontaneously in a starch paste, or gel on ageing, because of the tendency of starch molecules to bind to one another followed by an increase in crystallinity. Solutions of low concentration become increasingly cloudy due to the progressive association of starch molecules into larger articles. Spontaneous precipitation takes place and the precipitated starch appears to be reverting to its original condition of cold-water insolubility. Pastes of higher concentration on cooling set to a gel, which on ageing becomes steadily firmer due to the increasing association of the starch molecules. This arises because of the strong tendency for hydrogen bond formation between hydroxy groups on adjacent starch molecules. *See* J.A. Radley, ed., STARCH AND ITS DERIVATIVES 194-201 (Chapman and Hall, London (1968)).

The presence of IPS in saccharide liquor negatively affects final product quality and represents a major issue with downstream processing. IPS plugs or slows filtration system, and fouls the carbon columns used for purification. When IPS reaches sufficiently high levels, it may leak through the carbon columns and decrease production efficiency. Additionally, it may results in hazy final product upon storage, which is unacceptable for final product quality. The amount of IPS can be reduced by isolating the saccharification tank and blending the contents back. IPS nevertheless will accumulate in carbon columns and filter systems, among other things. The use of α-amylases is expected to improve overall process performance by reducing the amount of IPS.

In order to further illustrate the compositions and methods, and advantages thereof, the following specific examples are given with the understanding that they are illustrative rather than limiting.

## EXAMPLES

### Example 1

### Identification of secreted *Exiguobacterium* α-amylases belonging to CAZy glycosyl hydrolase family 13, subfamily 5

A search of the NCBI databases for amylases in the genus *Exiguobacterium* revealed the presence of an alphaamylase belonging to GH13 subfamily 5: *Exiguobacterium sibiricum* 255-15 alpha-amylase (NCBI Reference Sequence: YP_001813473.1; SEQ ID NO: 1). The amylase carries a signal peptide as predicted by as predicted by SignalP version 4.0 (Nordahl Petersen et al. (2011) Nature Methods, 8:785-786) indicating that it is a secreted enzyme

The amino acid sequence of the mature chain of *Exiguobacterium sibiricum* 255-15 alpha-amylase (EsiAmyl) is set forth below as SEQ ID NO: 1:

DNGTMMQYFEWYVPNDGNHWNRLGSDSTKLDQLGITSVWIPPAYKGTTQNDVGYGA
YDLYDLGEFNQKGTVRTKYGTKAQLKTAINQLHTAGIDVYGDVVMNHKGGADFTEA
VTAVEVNGSNRNQEISGDYQIQAWTGFDFAARNNTYSNFKWKWYHFDGTDWDQSRS
KSAIYKFRGTGKAWDTDVSTENGNYDYLMYADIDFDHPEVQQEMKNWGKWYVNELG
LDGFRLDAVKHIKHGYLADWLANVRQTTGKPLFTVAEYWQNDLGTLQNYLSRTNYQ
QSVFDAPLHYKFEQASKGGGYYDMRTIFDGTLVKTNPVQAVTLVENHDSQPGQSLEST
VQSWFKPLAYAMILTREQGYPSVFYGDYYGTKGTSNREIPALASKIDPLLKARKDFAFG
KQNDYLDNADVIGWTREGVTDRAKSGLATILSDGPGGSKWMYVGLQNKGEVWTDIT
GNNTASVTINQDGYGQFFVNGGSVSVYRQQ.

Sequencing of the genome of *Exiguobacterium acetylicum* DSM20416 (obtained from DSMZ: Deutsche Sammlung von Mikroorganismen und Zellkulturen, Braunschweig, GERMANY) resulted in the discovery of alpha-amylase EacAmyl, another member of Cazy family GH13, subfamily 5. The amino acid sequence of the predicted mature EacAmyl protein encoded by the gene *eacAmyl* is set forth below as SEQ ID NO: 2.

ATADNGTMMQYFEWYVPNDGNHWNRLGSDATKLDQLGITSVWIPPAYKGTSQNDVG
YGAYDLYDLGEFNQKGTVRTKYGTKAQLKTAIGQLHTAGIDVYGDVVMNHKGGADF
TEAVTAVEINPGNRNQEISGDYQIQAWTGFNFAARNNLYSNFKWKWYHFDGTDWDQS
RSKSAIYKFRGTGKAWDTDVSTENGNYDYLMYADLDFDHPEVQQEMKNWGKWYVN
ELGLDGFRLDAVKHIKHGYLADWLANVRQTTGKPLFTVAEYWQNDLGTLQNYLSRTN
YQQSVFDAPLHYKFEQASKGGGYYDMRTIFDGTLVKSNPVQAVTLVENHDSQPGQSLE
STVQSWFKPLAYAMILTREQGYPSVFYGDYYGTKGTSNREIPALASKIDPLLKARKDFA
FGKQNDYLDNQDIIGWTREGVSDRAKSGLATILSDGPGGSKWMYVGLQNKGEVWTDI
TGNNTASVTINQDGYGQFFVNGGSVSVYRQQ

Sequencing of the genome of *Exiguobacterium soli* DSM22015 ((obtained from DSMZ: Deutsche Sammlung von Mikroorganismen und Zellkulturen, Braunschweig, GERMANY) resulted in the discovery of alpha-amylase EsoAmyl, another member of Cazy family GH13, subfamily 5. The amino acid sequence of the predicted mature EsoAmyl protein encoded by the gene *esoAmyl* is set forth below as SEQ ID NO: 3.

ATADNGTMMQYFEWYLPNDGNHWNRLNTDTTKLDQLGITSVWIPPAYKGTTQNDVG
YGAYDLYDLGEFNQKGTVRTKYGTKAQLKTAISQLHTAGIDVYGDVVMNHKGGADFT
EAVTAVEVNGSNRNQEVSGDYQIQAWTGFDFAARNNTYSNFKWKWYHFDGTDWDQS
RSKSAIYKFRGTGKAWDTDVSTENGNYDYLMYADIDFDHPEVQQEMKNWGKWYVNE
LGLDGFRLDAVKHIKHGYLADWLANVRQTTGKPLFTVAEYWQNDLGTLQNYLSRTNY
QQSVFDAPLHYKFEQASKGGGYYDMRTIFDGTLVKSNPVQAVTLVENHDSQPGQSLES
TVQSWFKPLAYAMILTREQGYPSVFYGDYYGTKGTSNREIPALGSKIDPLLKARKDFAF
GKQNDYLDNADVIGWTREGVTDRAKSGLATILSDGPGGSKWMYVGTQNKGEVWTDI
TGNNSASVTINQDGYGQFFVNGGSVSVYRQQ

Sequencing of the genome of *Exiguobacterium undae* DSM14481 (obtained from DSMZ: Deutsche Sammlung von Mikroorganismen und Zellkulturen, Braunschweig, GERMANY) resulted in the discovery of alpha-amylase EunAmyl, another member of the Cazy family GH13, subfamily 5. The nucleotide sequence of *eunAmyl* amylase gene is set forth below as SEQ ID NO: 16. The sequence encoding the native signal peptide, as predicted by SignalP-NN (Emanuelsson et al. (2007) Nature Protocols, 2:953-971), is shown in bold:

**ATGAAACAAAAACGCATGATTGTCGCAACACTTGCGACAGCTACTTTTTTAGCG**
**CCACTTGTGCAACCGATTGCAGTCGGA**GCAACGGCGGACAATGGAACGATGATG
CAGTATTTTGAATGGTACTTGCCAAACGACGGCAATCATTGGAACCGCTTGAGCAG
TGATACGACGAAACTGGATCAGCTCGGGATCACCTCGGTCTGGATTCCGCCCGCTTA
CAAAGGAACGAGTCAAAATGATGTCGGGTACGGTGCGTATGATTTGTACGATCTCG

GAGAATTTAATCAAAAAGGAACTGTCCGGACAAAATACGGAACGAAAGCACAGCT
GAAATCAGCCATCAATCAACTGCATACAGCCGGGATTGATGTCTACGGTGATGTCG
TCATGAACCATAAAGGCGGCGCTGATTTCACGGAATCGGTAACGGCTGTTGAAGTC
AACGGCGGCAACCGCAATCAGGAAATTTCGGGAGATTATCAGATTCAAGCTTGGAC
CGGCTTTAATTTCGCCACACGTAACAATGCGTATTCGAATTTCAAGTGGAAATGGTA
TCACTTTGACGGGACAGACTGGGATCAGTCACGTTCCAAAAGTGCCATCTATAAGTT
CCGGGGGACAGGAAAAGCCTGGGATACTGATGTATCCACGGAAAACGGGAATTAT
GATTACTTAATGTATGCTGATGTCGATTTTGATCATCCGGAAGTTCAGCAGGAAATG
AAGAACTGGGGTAAATGGTACGTCAATGAGCTTGGTCTCGACGGATTCCGACTCGA
TGCCGTCAAACATATCAAACACGGTTATCTCGCGGACTGGCTTGCCAACGTCCGGC
AAACAACCGGCAAACCGTTATTTACGGTAGCCGAATACTGGCAAAATGACCTCGGC
ACGCTGAAAAACTATCTCAGTCGGACGAACTATAAGCAGTCGGTCTTCGATGCCCC
ACTGCATTACAAGTTCGAACAGGCGAGTAAAGGTGGCGGGTATTACGACATGCGGA
CAATCTTTAACGGAACCGTCGTCCAAGACAATCCGACGCTTGCCGTCACACTTGTCG
AAAACCATGACTCGCAACCCGGCCAATCGCTCGAATCAACGGTCCAGCCTTGGTTC
AAACCACTCGCTTACGCAATGATCTTAACGCGTGAACAAGGGTATCCGTCGGTCTTC
TACGGGGATTACTACGGTACAAAAGGTACTTCGAACCGCGAAATCCCGGCACTTGG
CTCTAAAATCGATCCCCTCTTAAAAGCCCGGAAAGACTTTGCCTATGGAAAACAAA
ACGACTATCTCGACAATGCCGATGTCATCGGTTGGACACGCGAAGGGGTAACGGAT
CGCGCAAAATCAGGTCTCGCGACCATCCTTTCCGATGGACCGGGCGGCAGCAAGTG
GATGTACGTCGGAACACAAACAAAGGTGAGGTTTGGACAGATATCACCGGCAAC
AACTCCGCATCTGTCACGATCAACCAGGACGGTTACGGTCAGTTCTTCGTCAATGGC
GGATCCGTCTCTGTTTACCGTCAGCAG

The amino acid sequence of the predicted mature EunAmyl protein encoded by the gene *eunAmyl* is set forth as SEQ ID NO: 4.

ATADNGTMMQYFEWYLPNDGNHWNRLSSDTTKLDQLGITSVWIPPAYKGTSQNDVGY
GAYDLYDLGEFNQKGTVRTKYGTKAQLKSAINQLHTAGIDVYGDVVMNHKGGADFTE
SVTAVEVNGGNRNQEISGDYQIQAWTGFNFATRNNAYSNFKWKWYHFDGTDWDQSR
SKSAIYKFRGTGKAWDTDVSTENGNYDYLMYADVDFDHPEVQQEMKNWGKWYVNE
LGLDGFRLDAVKHIKHGYLADWLANVRQTTGKPLFTVAEYWQNDLGTLKNYLSRTNY
KQSVFDAPLHYKFEQASKGGGYYDMRTIFNGTVVQDNPTLAVTLVENHDSQPGQSLES
TVQPWFKPLAYAMILTREQGYPSVFYGDYYGTKGTSNREIPALGSKIDPLLKARKDFAY
GKQNDYLDNADVIGWTREGVTDRAKSGLATILSDGPGGSKWMYVGTQNKGEVWTDI
TGNNSASVTINQDGYGQFFVNGGSVSVYRQQ

Sequencing of the genome of *Exiguobacterium oxidotolerans* DSM17272 (obtained from DSMZ: Deutsche Sammlung von Mikroorganismen und Zellkulturen, Braunschweig, GERMANY) resulted in the discovery of alpha-amylase EoxAmyl, another member of the Cazy family GH13, subfamily 5. The nucleotide sequence of the *eoxAmyl* gene is set forth below as SEQ ID NO: 17. The sequence encoding the native signal peptide, as predicted by SignalP-NN (Emanuelsson et al. (2007) Nature Protocols, 2:953-971), is shown in bold:

**ATGAAACATAAAAGCTTGATTGTCGCATCTCTTGCCAGCGTGACGTTTTTAGCG
CCACTTGCGCAACCGATTGCAGTAGGA**GCAACAGCAGACAACGGGACGATGATG
CAATACTTTGAATGGTATTTACCAAACGACGGGAACCACTGGAACCGTCTAGGAAA
CGACGCTTCTAAGCTTGATCAACTCGGAATTACATCTGTTTGGATTCCTCCTGCCTA
CAAAGGAACGACCCAAAATGATGTCGGCTACGGTGCCTACGATCTATATGACCTCG
GTGAGTTTAATCAAAAAGGAACAGTCCGGACGAAGTACGGCACGAAAACGCAATT
GAAGTCCGCCATCGGGCAATTGCACACGGCTGGAATCGATGTGTATGGTGATGTCG
TCATGAACCACAAGGGTGGTGCTGACTTTACGGAATCCGTCACAGCCGTCGAAGTC
AATCCGGGTAACCGTAATCAAGAAGTCTCTGGCGACTATCAAATCCAGGCCTGGAC
CGGGTTCAACTTCGCGGCACGGAGCAACGCCTATTCAAACTTCAAATGGAAATGGT
ATCACTTCGACGGAACGGATTGGGATCAATCCCGCTCAAAAAGTGCCATCTATAAA
TTCCGTGGAACAGGTAAGTCGTGGGACTCGAATGTGTCTTCTGAAAATGGAAACTA
TGATTACTTGATGTATGCAGACATTGATTTCGATCACCCGGAAGTGCAACAGGAAA
TGAAGAACTGGGGGAAATGGTACGTCAATGAACTCGGGCTCGACGGATTCCGTCTT
GATGCCGTCAAACACATCAAACATACGTATCTCGCAGATTGGTTGACGAACGTTCG
TCAGACGACGGGTAAGGAACTATTCACAGTCGCCGAATACTGGCAGAACGATCTCG
GGACCCTTAAAAACTATTTAAGTCAGACGAACTATAAACAATCCGTTTTTGACGCTC
CACTTCATTACAAATTCGAACAAGCGAGTAAAGGCGGCGGCTTTTATGACATGCGC
ACAATTTTTAACGGTACACTCGTCCAAGATAACCCGACGCTTGCCGTCACACTCGTT
GAAAACCATGATTCTCAACCTGGTCAATCGCTCGAATCGACCGTTCAATCCTGGTTC
AAGCCCCTTGCTTACGCGATGATTTTGACGCGAGAACAAGGGTATCCATCCGTCTTT
TACGGGGACTACTACGGCACGAAGGGTTCCTCGAACCGCGAAATCCCTGCCCTCGC
GTCAAAAATCGATCCGATTCTAAAAGCACGGAAAGACTATGCATTCGGTAAGCAAA
ACGATTACCTCGATAATCCGGATGTCATCGGTTGGACACGGGAAGGCGTCAGTGAC
CGCTCAAAATCAGGGCTTGCGACAATCCTATCTGACGGTCCTGGTGGTAGCAAGTG
GATGTATGTCGGTACGCAAAATAAAGGCGAAGTCTGGACAGACATCACCGGCAATA
ATTCGGCTTCCGTCACGATTAATGCCGACGGGTATGGTCAATTTTTCGTCAATGGTG
GTTCTGTCTCGATTTACCGCCAACAA**

The amino acid sequence of the predicted mature EoxAmyl protein encoded by the *eoxAmyl* gene is set forth as SEQ ID NO: 5.

ATADNGTMMQYFEWYLPNDGNHWNRLGNDASKLDQLGITSVWIPPAYKGTTQNDVG

YGAYDLYDLGEFNQKGTVRTKYGTKTQLKSAIGQLHTAGIDVYGDVVMNHKGGADFT

ESVTAVEVNPGNRNQEVSGDYQIQAWTGFNFAARSNAYSNFKWKWYHFDGTDWDQS

RSKSAIYKFRGTGKSWDSNVSSENGNYDYLMYADIDFDHPEVQQEMKNWGKWYVNE

LGLDGFRLDAVKHIKHTYLADWLTNVRQTTGKELFTVAEYWQNDLGTLKNYLSQTNY

KQSVFDAPLHYKFEQASKGGGFYDMRTIFNGTLVQDNPTLAVTLVENHDSQPGQSLES

TVQSWFKPLAYAMILTREQGYPSVFYGDYYGTKGSSNREIPALASKIDPILKARKDYAF

GKQNDYLDNPDVIGWTREGVSDRSKSGLATILSDGPGGSKWMYVGTQNKGEVWTDIT

GNNSASVTINADGYGQFFVNGGSVSIYRQQ

The amino acid sequence of the predicted mature EanAmyl protein from *Exiguobacterium antarcticum* B7 (YP_006790696.1) is set forth below as SEQ ID NO: 6.

ATADNGTMMQYFEWYLPNDGNHWNRLNTDTTKLDQLGITSVWIPPAYKGTTQNDVG

YGAYDLYDLGEFNQKGTVRTKYGTKAQLKTAISQLHTAGIDVYGDVVMNHKGGADFT

ESVTAVEVNGGNRNQEVSGDYQIQAWTGFDFAARNNTYSNFKWKWYHFDGTDWDQS

RSKSAIYKFRGTGKAWDTDVSTENGNYDYLMYADIDFDHPEVQQEMKNWGKWYVNE

LGLDGFRLDAVKHIKHGYLADWLANVRQTTGKPLFTVAEYWQNDLGTLQNYLSRTNY

QQSVFDAPLHYKFEQASKGGGYYDMRTIFDGTLVKSNPVQAVTLVENHDSQPGQSLES

TVQSWFKPLAYAMILTREQGYPSVFYGDYYGTKGTSNREIPALGSKIDPLLKARKDFAF

GKQNDYLDNADVIGWTREGVTDRAKSGLATILSDGPGGSKWMYVGTQNKGEVWTDI

TGNNTASVTINQDGYGQFFVNGGSVSVYRQQ

## Example 2

### Expression of the *Exiguobacterium* amylases in *Bacillus subtilis*

The amylase EsiAmyl (SEQ ID NO: 1) was expressed in *B. subtilis* by using the pHPLT expression vector (Solingen et al. (2001) Extremophiles 5:333-341; US Patent Application 20100021587). A synthetic gene encoding the amylase was synthesized as *Pst* I - *Hpa* I fragments at Geneart / Life Technologies (Regensburg, Germany) and fused in-frame to the amyL (*B. licheniformis* alpha-amylase) signal peptide sequence and in front of the amyL transcription terminator, both present in the pHPLT vector.

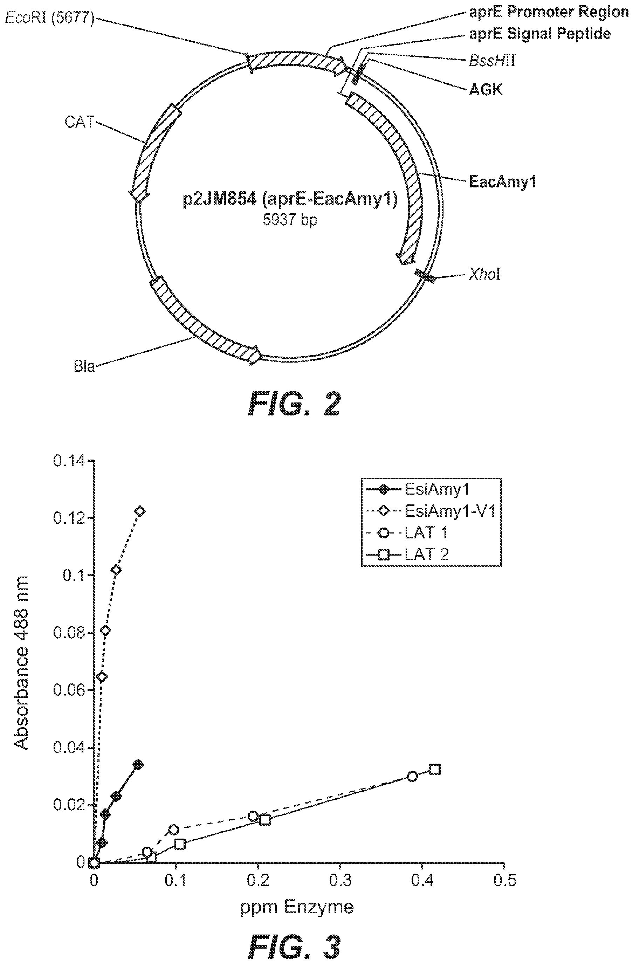

Synthetic genes encoding mature EacAmyl, EsoAmyl, EunAmyl, and EoxAmyl were synthesized by Generay (Shanghai, China) The synthesized genes were inserted into the p2JM-modified vector (Vogtentanz (2007) Protein Expr. Purif., 55:40-52), resulting in expression plasmids p2JM854 (aprE- EacAmyl), p2JM856 (aprE-EsoAmyl), p2JM857 (aprE-EunAmyl), and p2JM859 (aprE-EoxAmyl). Each plasmid contains an *aprE* promoter, an *aprE* signal sequence used to direct protein secretion in *B. subtilis,* an oligonucleotide encoding Ala-Gly-Lys to facilitate the secretion of the target protein, and the synthetic nucleotide sequence encoding the mature protein. An exemplary plasmid map of p2JM854 (aprE- EacAmyl) is shown in Figure 2. for expression in *Bacillus subtilis.*

The pHPLT expression plasmids were transformed into a suitable two-protease-deleted *Bacillus subtilis* strain and the resulting transformants were selected on plates containing Heart infusion agar (Difco, Cat.No. 244400) and 10 mg/L neomycin sulphate (Sigma, Neomycin sulphate Cat. No. N-1876). Selective growth of *B. subtilis* transformants harbouring the pHPLT amylase expression plasmids was performed in shake flasks containing MBD medium (a MOPS based defined medium), 5 mM $CaCl_2$ and 10 mg/L neomycin. MBD medium was made essentially as known in the art (see, Neidhardt et al. (1974) J. Bacteriol., 119:736-747), except that $NH_4Cl_2$, $FeSO_4$, and $CaCl_2$ were omitted from the base medium, 3 mM $K_2HPO_4$ was used, and the base medium was supplemented with 60 mM urea, 75 g/L glucose, and 1% soytone. The micronutrients were made up as a 100X stock solution containing in one liter, 400 mg $FeSO_4 \cdot 7H_2O$, 100 mg $MnSO_4 \cdot H_2O$, 100 mg $ZnSO_4 \cdot 7H_2O$, 50 mg $CuCl_2 \cdot 2H_2O$, 100 mg $CoCl_2 \cdot 6H_2O$, 100 mg $NaMoO_4 \cdot 2H_2O$, 100 mg $Na_2B_4O_7 \cdot 10H_2O$, 10 ml of 1M $CaCl_2$, and 10 ml of 0.5 M sodium citrate. Growth resulted in the production of secreted amylase with starch hydrolyzing activity.

The p2JM plasmids were amplified using Illustra TempliPhi 100 Amplification Kit (GE Healthcare Life Sciences, NJ). A suitable *B. subtilis* strain was transformed with the amplification product using a method known in the art (WO 02/14490). The *B. subtilis* transformants were selected on Luria Agar plates supplemented with 5 ppm chloramphenicol. The colonies from the transformation plates were inoculated into 5 ml LB medium and incubated at 37°C overnight. Selective growth of *B. subtilis* transformants harboring the plasmids was performed at 37°C for 48 hours in MBD medium (enriched semi-defined medium based on MOPs buffer, with urea as major nitrogen source, glucose as the main carbon source, and supplemented with 2% soytone for robust cell growth) containing 5 mM $CaCl_2$ and 5 ppm chloramphenicol. Cells were harvested by centrifugation and supernatants were analyzed by SDS-PAGE.

The amino acid sequence of the mature form of EacAmyl expressed from plasmid p2JM854 (aprE-EacAmyl) is set forth below as SEQ ID NO: 7. The three residue addition (AGK) is shown in bold.

**AGK**ATADNGTMMQYFEWYVPNDGNHWNRLGSDATKLDQLGITSVWIPPAYKGTSQN
DVGYGAYDLYDLGEFNQKGTVRTKYGTKAQLKTAIGQLHTAGIDVYGDVVMNHKGG
ADFTEAVTAVEINPGNRNQEISGDYQIQAWTGFNFAARNNLYSNFKWKWYHFDGTDW
DQSRSKSAIYKFRGTGKAWDTDVSTENGNYDYLMYADLDFDHPEVQQEMKNWGKW
YVNELGLDGFRLDAVKHIKHGYLADWLANVRQTTGKPLFTVAEYWQNDLGTLQNYLS
RTNYQQSVFDAPLHYKFEQASKGGGYYDMRTIFDGTLVKSNPVQAVTLVENHDSQPG
QSLESTVQSWFKPLAYAMILTREQGYPSVFYGDYYGTKGTSNREIPALASKIDPLLKAR
KDFAFGKQNDYLDNQDIIGWTREGVSDRAKSGLATILSDGPGGSKWMYVGLQNKGEV
WTDITGNNTASVTINQDGYGQFFVNGGSVSVYRQQ

The amino acid sequence of the mature form of EsoAmylnexpressed from plasmid p2JM856 (aprE- EsoAmyl) is set forth below as SEQ ID NO: 8. The three residue addition (AGK) is shown in bold.

**AGK**ATADNGTMMQYFEWYLPNDGNHWNRLNTDTTKLDQLGITSVWIPPAYKGTTQN
DVGYGAYDLYDLGEFNQKGTVRTKYGTKAQLKTAISQLHTAGIDVYGDVVMNHKGG
ADFTEAVTAVEVNGSNRNQEVSGDYQIQAWTGFDFAARNNTYSNFKWKWYHFDGTD
WDQSRSKSAIYKFRGTGKAWDTDVSTENGNYDYLMYADIDFDHPEVQQEMKNWGK
WYVNELGLDGFRLDAVKHIKHGYLADWLANVRQTTGKPLFTVAEYWQNDLGTLQNY
LSRTNYQQSVFDAPLHYKFEQASKGGGYYDMRTIFDGTLVKSNPVQAVTLVENHDSQP
GQSLESTVQSWFKPLAYAMILTREQGYPSVFYGDYYGTKGTSNREIPALGSKIDPLLKA
RKDFAFGKQNDYLDNADVIGWTREGVTDRAKSGLATILSDGPGGSKWMYVGTQNKG
EVWTDITGNNSASVTINQDGYGQFFVNGGSVSVYRQQ

The amino acid sequence of the mature form of EunAmyl amylase expressed from plasmid p2JM857 (aprE-EunAmyl) is set forth below as SEQ ID NO: 9. The three residue addition (AGK) is shown in bold.

**AGK**ATADNGTMMQYFEWYLPNDGNHWNRLSSDTTKLDQLGITSVWIPPAYKGTSQN
DVGYGAYDLYDLGEFNQKGTVRTKYGTKAQLKSAINQLHTAGIDVYGDVVMNHKGG
ADFTESVTAVEVNGGNRNQEISGDYQIQAWTGFNFATRNNAYSNFKWKWYHFDGTD
WDQSRSKSAIYKFRGTGKAWDTDVSTENGNYDYLMYADVDFDHPEVQQEMKNWGK
WYVNELGLDGFRLDAVKHIKHGYLADWLANVRQTTGKPLFTVAEYWQNDLGTLKNY
LSRTNYKQSVFDAPLHYKFEQASKGGGYYDMRTIFNGTVVQDNPTLAVTLVENHDSQP
GQSLESTVQPWFKPLAYAMILTREQGYPSVFYGDYYGTKGTSNREIPALGSKIDPLLKA
RKDFAYGKQNDYLDNADVIGWTREGVTDRAKSGLATILSDGPGGSKWMYVGTQNKG
EVWTDITGNNSASVTINQDGYGQFFVNGGSVSVYRQQ

The amino acid sequence of the mature form of EoxAmyl expressed from plasmid p2JM859 (aprE-EoxAmyl) is set forth below as SEQ ID NO: 10. The three residue addition (AGK) is shown in bold.

**AGK**ATADNGTMMQYFEWYLPNDGNHWNRLGNDASKLDQLGITSVWIPPAYKGTTQN
DVGYGAYDLYDLGEFNQKGTVRTKYGTKTQLKSAIGQLHTAGIDVYGDVVMNHKGG
ADFTESVTAVEVNPGNRNQEVSGDYQIQAWTGFNFAARSNAYSNFKWKWYHFDGTD
WDQSRSKSAIYKFRGTGKSWDSNVSSENGNYDYLMYADIDFDHPEVQQEMKNWGKW
YVNELGLDGFRLDAVKHIKHTYLADWLTNVRQTTGKELFTVAEYWQNDLGTLKNYLS
QTNYKQSVFDAPLHYKFEQASKGGGFYDMRTIFNGTLVQDNPTLAVTLVENHDSQPGQ
SLESTVQSWFKPLAYAMILTREQGYPSVFYGDYYGTKGSSNREIPALASKIDPILKARKD
YAFGKQNDYLDNPDVIGWTREGVSDRSKSGLATILSDGPGGSKWMYVGTQNKGEVWT
DITGNNSASVTINADGYGQFFVNGGSVSIYRQQ

## Example 3

### Expression of variants of the *Exiguobacterium* amylases in *Bacillus subtilis*

Suzuki et al. ((1989) J. Biol. Chem., 264:18933-38) showed that deletion of R176 and G177 (*i.e.*, the "RG deletion") in *B. amyloliquefaciens* amylase enhances the stability of the enzyme. The substitution of serine at position 242 in *G. stearothermophilus* amylase into glutamine was shown to enhance the stability of the enzyme (US8206966). Mutation G475K in *Bacillus* sp. TS-23 amylase enhanced cleaning performance of the enzyme (US20120045817). Equivalent mutations were made in the *Exiguobacterium* amylases described in Example 2.

The amino acid sequence of a variant of EsiAmyl amylase having an RG deletion and the substitutions S239G and G474K (*i.e.*, EsiAmyl-V1) is set forth below as SEQ ID NO: 11:

DNGTMMQYFEWYVPNDGNHWNRLGSDSTKLDQLGITSVWIPPAYKGTTQNDVGYGA
YDLYDLGEFNQKGTVRTKYGTKAQLKTAINQLHTAGIDVYGDVVMNHKGGADFTEA
VTAVEVNGSRNQEISGDYQIQAWTGFDFAARNNTYSNFKWKWYHFDGTDWDQSRS
KSAIYKFTGKAWDTDVSTENGNYDYLMYADIDFDHPEVQQEMKNWGKWYVNELGLD
GFRLDAVKHIKHQYLADWLANVRQTTGKPLFTVAEYWQNDLGTLQNYLSRTNYQQSV
FDAPLHYKFEQASKGGGYYDMRTIFDGTLVKTNPVQAVTLVENHDSQPGQSLESTVQS
WFKPLAYAMILTREQGYPSVFYGDYYGTKGTSNREIPALASKIDPLLKARKDFAFGKQN
DYLDNADVIGWTREGVTDRAKSGLATILSDGPGGSKWMYVGLQNKGEVWTDITGNNT
ASVTINQDGYGQFFVNKGSVSVYRQQ

The amino acid sequence of a variant of EacAmyl-V1 amylase having an RG deletetion (*i.e* EacAmy1-V1) is set forth below as SEQ ID NO: 12:

**AGK**ATADNGTMMQYFEWYVPNDGNHWNRLGSDATKLDQLGITSVWIPPAYKGTSQN
DVGYGAYDLYDLGEFNQKGTVRTKYGTKAQLKTAIGQLHTAGIDVYGDVVMNHKGG
ADFTEAVTAVEINPGNRNQEISGDYQIQAWTGFNFAARNNLYSNFKWKWYHFDGTDW
DQSRSKSAIYKFTGKAWDTDVSTENGNYDYLMYADLDFDHPEVQQEMKNWGKWYV
NELGLDGFRLDAVKHIKHGYLADWLANVRQTTGKPLFTVAEYWQNDLGTLQNYLSRT
NYQQSVFDAPLHYKFEQASKGGGYYDMRTIFDGTLVKSNPVQAVTLVENHDSQPGQSL
ESTVQSWFKPLAYAMILTREQGYPSVFYGDYYGTKGTSNREIPALASKIDPLLKARKDF
AFGKQNDYLDNQDIIGWTREGVSDRAKSGLATILSDGPGGSKWMYVGLQNKGEVWTD
ITGNNTASVTINQDGYGQFFVNGGSVSVYRQQ

The amino acid sequence of a variant of EsoAmyl-V1 amylase having an RG deletetion (*i.e* EsoAmy1-V1) is set forth below as SEQ ID NO: 13:

**AGK**ATADNGTMMQYFEWYLPNDGNHWNRLNTDTTKLDQLGITSVWIPPAYKGTTQN
DVGYGAYDLYDLGEFNQKGTVRTKYGTKAQLKTAISQLHTAGIDVYGDVVMNHKGG
ADFTEAVTAVEVNGSRNQEVSGDYQIQAWTGFDFAARNNTYSNFKWKWYHFDGTD
WDQSRSKSAIYKFTGKAWDTDVSTENGNYDYLMYADIDFDHPEVQQEMKNWGKWY
VNELGLDGFRLDAVKHIKHGYLADWLANVRQTTGKPLFTVAEYWQNDLGTLQNYLSR
TNYQQSVFDAPLHYKFEQASKGGGYYDMRTIFDGTLVKSNPVQAVTLVENHDSQPGQS
LESTVQSWFKPLAYAMILTREQGYPSVFYGDYYGTKGTSNREIPALGSKIDPLLKARKD
FAFGKQNDYLDNADVIGWTREGVTDRAKSGLATILSDGPGGSKWMYVGTQNKGEVW
TDITGNNSASVTINQDGYGQFFVNGGSVSVYRQQ

The amino acid sequence of a variant of EunAmyl-V1 amylase having an RG deletetion (*i.e* EunAmy1-V1) is set forth below as SEQ ID NO: 14:

**AGK**ATADNGTMMQYFEWYLPNDGNHWNRLSSDTTKLDQLGITSVWIPPAYKGTSQN

DVGYGAYDLYDLGEFNQKGTVRTKYGTKAQLKSAINQLHTAGIDVYGDVVMNHKGG

ADFTESVTAVEVNGGNRNQEISGDYQIQAWTGFNFATRNNAYSNFKWKWYHFDGTD

WDQSRSKSAIYKFTGKAWDTDVSTENGNYDYLMYADVDFDHPEVQQEMKNWGKWY

VNELGLDGFRLDAVKHIKHGYLADWLANVRQTTGKPLFTVAEYWQNDLGTLKNYLSR

TNYKQSVFDAPLHYKFEQASKGGGYYDMRTIFNGTVVQDNPTLAVTLVENHDSQPGQ

SLESTVQPWFKPLAYAMILTREQGYPSVFYGDYYGTKGTSNREIPALGSKIDPLLKARK

DFAYGKQNDYLDNADVIGWTREGVTDRAKSGLATILSDGPGGSKWMYVGTQNKGEV

WTDITGNNSASVTINQDGYGQFFVNGGSVSVYRQQ

The amino acid sequence of a variant of EoxAmy1-V1 amylase having an RG deletion (i.e EoxAmy1-V1) is set forth below as SEQ ID NO: 15:

ATADNGTMMQYFEWYLPNDGNHWNRLGNDASKLDQLGITSVWIPPAYKGT

TQNDVGYGAYDLYDLGEFNQKGTVRTKYGTKTQLKSAIGQLHTAGIDVYGDVVMNH

KGGADFTESVTAVEVNPGNRNQEVSGDYQIQAWTGFNFAARSNAYSNFKWKWYHFD

GTDWDQSRSKSAIYKFTGKSWDSNVSSENGNYDYLMYADIDFDHPEVQQEMKNWGK

WYVNELGLDGFRLDAVKHIKHTYLADWLTNVRQTTGKELFTVAEYWQNDLGTLKNY

LSQTNYKQSVFDAPLHYKFEQASKGGGFYDMRTIFNGTLVQDNPTLAVTLVENHDSQP

GQSLESTVQSWFKPLAYAMILTREQGYPSVFYGDYYGTKGSSNREIPALASKIDPILKAR

KDYAFGKQNDYLDNPDVIGWTREGVSDRSKSGLATILSDGPGGSKWMYVGTQNKGEV

WTDITGNNSASVTINADGYGQFFVNGGSVSIYRQQ

The variant amylases were expressed as in Example 2.

**Example 4**

**Purification of *Exiguobacterium* amylases**

EacAmy1 (SEQ ID NO: 7) and EacAmy1-V1 (SEQ ID NO: 12) amylases were purified using anion-exchange and size-exclusion chromatography columns. Briefly, crude samples were desalted by VIVAFLOW200 and exchanged into buffer A (20mM, Tris-HCl, pH8.0). The desalted samples were then loaded onto a Q-Sepharose HP column pre-equilibrated with buffer A. The proteins were eluted with a gradient of 0-50% buffer B (buffer A with 1 M NaCl) in 8 CVs. The target proteins were in the flow-through based on activity assay. The flowthroughs were concentrated by 10 KDa Amicon Ultra-15 and then applied to a Superdex column in buffer C (20 mM sodium phosphate pH 7.0 with 0.15 M NaCl). The target proteins were collected and concentrated using 10 KDa Amicon Ultra-15 devices. The samples were above 98% pure and stored in 40% glycerol at -80°C until usage.

EsoAmy1 (SEQ ID NO: 8), EunAmy1 (SEQ ID NO: 9), and EoxAmy1 (SEQ ID NO: 10) amylases were purified by ammonium sulphate precipitation and anion-exchange and size-exclusion chromatography columns. Ammonium sulphate was added to fermentation broth containing the amylases to a final saturation of 70%. The solution was stirred at 4°C overnight, and then centrifuged at 14,000X g for 1 hour. The target protein-containing pellet was resuspended in 20 mM HEPES, pH 8 with 2 mM $CaCl_2$ (buffer A), and then loaded onto a Q-Sepharose column pre-equilibrated with buffer A. After column washing, the protein was eluted by gradient elution of 0-50% 20 mM HEPES pH 8 with 1 M NaCl (buffer B).The flow-through fraction was concentrated and applied to a Superdex in 20 mM sodium phosphate buffer pH 7 with 0.15 M NaCl. Fractions containing target protein were pooled and concentrated using 10 KDa Amicon Ultra-15 devices. The sample was above 90% pure and stored in 40% glycerol at-80°C until usage.

EsoAmy1-V1 (SEQ ID NO: 13) and EunAmy1-V1 (SEQ ID NO: 14) amylases were purified via three chromatography steps: anion-exchange, size-exclusion and anion-exchange chromatography. Crude sample was desalted by VIVAFLOW200 and exchanged into buffer A (20mM, Tris-HCl, pH 8.0). The desalted sample was then loaded onto a Q-Sepharose HP column pre-equilibrated with buffer A. After column washing, the column was eluted with a gradient of 0-50% buffer B (buffer A with 1 M NaCl) in 8 CVs. The flow-through was concentrated by 10 KDa Amicon Ultra-15 and then applied to a Superdex column in buffer C (20 mM sodium phosphate pH 7.0 with 0.15 M NaCl for EsoAmy1-V1 and 20 mM HEPES pH 8.0 with 0.1 M NaCl and 20% Glycerol for EunAmy1-V1 (buffer D)). The active fractions were pooled, desalted and loaded onto a Q-Sepharose HP column pre-equilibrated with 20mM, sodium carbonate pH 9.0 for EsoAmy1-V1 or SP HP column pre-equilibrated with 20 mM HEPES, pH 8 with 20% Glycerol for EunAmy1-V1. EunAmy1-V1 was in the flow-through based on activity assay and EsoAmy1-V1 was eluted with a gradient of 0-50% buffer E (buffer D with 1 M NaCl). The purified proteins were collected and concentrated using 10 KDa Amicon Ultra-15 devices. The samples were above 98% pure and stored in 40% glycerol at -80°C until usage.

EoxAmy1-v1 (SEQ ID NO: 17) was purified via four chromatography steps: anion-exchange, hydrophobic interaction, anion-exchange and size-exclusion chromatography. 0.2 L of crude broth from the fermentor was exchanged into buffer A (50mM Tris-HCl pH 7.5 with 5% glycerol). The desalted sample was loaded onto a 20 mL Q-Sepharose FF column pre-equilibrated with buffer A. The target protein was in the flowthrough based on activity assay. Ammonium sulfate was added to the flowthrough to a final concentration of 1M. The flowthrough was then loaded onto a 20 mL Phenyl-Sepharose HP column pre-equilibrated with buffer B (buffer A with 1 M ammonium sulfate). After washing with 40% buffer B to remove bacterial proteins, the target protein was eluted with a linear gradient of 40% - 10% buffer B in 3 CVs, followed by 1 CVs of 10% buffer B and 2 CVs of buffer A to clean the column. The target protein was eluted from the column by 40% - 10% buffer B. The fractions containing the target protein were pooled and desalted by ultrafiltration. The desalted sample was then loaded onto an 8 ml mono Q 10/100 GL column pre-equilibrated with buffer A. The target protein was again in the flowthrough based on activity assay. The flowthrough was concentrated and applied to a 120 ml Superdex 75 16/60 column in buffer C (50 mM Tris-HCl pH 8.5 with 0.1 M sodium chloride). The target protein was collected and concentrated using 10 KDa Amicon Ultra-15 devices. The sample was above 95% pure and stored in 40% glycerol at -80°C until usage.

## Example 5

### Alpha-amylase activity assay of *Exiguobacterium* amylases

Alpha-amylase activities of EacAmyl (SEQ ID NO: 7), EsoAmyl (SEQ ID NO: 8), EunAmyl (SEQ ID NO: 9), EoxAmyl (SEQ ID NO: 10), EacAmyl-V1 (SEQ ID NO: 12), EsoAmy1-V1 (SEQ ID NO: 13), EunAmy1-V1 (SEQ ID NO: 14), and EoxAmy1-V1 (SEQ ID NO: 17) were assayed using a colorimetric assay to monitor the release of reducing sugars from a potato amylopectin substrate. The activity is reported as equivalents of glucose released per minute. Substrate solutions were prepared by mixing 9 mL of 1% (w/w, in water) potato amylopectin (Sigma, Cat. No. 10118), 1 mL of 0.5 M buffer (pH 5.0 sodium acetate or pH 8.0 HEPES), and 40 $\mu$L of 0.5 M $CaCl_2$ in a 15-mL conical tube. Serial dilutions of enzyme samples in dilution buffer were prepared in non-binding microtiter plates (MTP, Corning 3641). Then 90 $\mu$L of substrate solution (preincubated at 50 °C for 5 min at 600 rpm) and 10 $\mu$L of the diluted enzyme samples were mixed into the MTPs. Reactions were carried out for 10 minutes at 50°C at 600 rpm in a thermomixer (Eppendorf), and aliquots of 0.5 N NaOH were added to each well to stop the reaction. Total reducing sugars present in each well were measured using a PAHBAH method: (Lever, M. *et al.* (1973) 82:649-655.): 80 $\mu$L of 0.5 N NaOH was aliquoted into a microtiter plate followed by the addition of 20 $\mu$L of PAHBAH reagent (5 % w/v 4-hydroxybenzoic acid hydrazide in 0.5 N HCl) and 10 $\mu$L of the reaction mixture. Plates were incubated at 95°C for 5 minutes, samples were transferred to polystyrene microtiter plates (Costar 9017) and absorbance was measured at 410 nm. Resulting absorbance values were plotted against enzyme concentration and linear regression was used to determine the slope of the line. The amylase activities are as shown in Table 2 when calculated using the following equation:

$$\text{Specific Activity (U/mg)} = \text{Slope (enzyme)} / \text{slope (std)} * 100$$

where 1 U = 1 $\mu$mol glucose equivalent/min

**Table 2.** Specific activities of of α-amylases

| Enzyme Name | Spec. Activity (U/mg) | |
|---|---|---|
| | pH 5 | pH 8 |
| EacAmy1 | 1931.8 | 2903.9 |
| EacAmy1-V1 | 2888.0 | 2593.1 |
| EsoAmy1 | 1065.7 | 2023.2 |
| EsoAmy1-V1 | 1004.5 | 907.3 |
| EunAmy1 | 562.5 | 969.5 |
| EunAmy1-V1 | 2358.0 | 2589.1 |
| EoxAmy1 | 4430.0 | 3629.0 |
| EoxAmy1-V1 | 2463.4 | 2724.3 |

### Example 6

**Effect of pH on amylase activities of *Exiguobacterium* amylases**

The effect of pH on the α-amylase activities of EacAmyl (SEQ ID NO: 7), EsoAmyl (SEQ ID NO: 8), EunAmyl (SEQ ID NO: 9), EoxAmyl (SEQ ID NO: 10), EacAmyl-V1 (SEQ ID NO: 12), EsoAmyl-V1 (SEQ ID NO: 13), EunAmy1-V1 (SEQ ID NO: 14), and EoxAmy1-V1 (SEQ ID NO: 17) were monitored using the PAHBAH assay protocol as described above, with a pH range from 3.0 to 10.0. Working buffers consisted of the combination of glycine/sodium acetate/HEPES (250 mM), with pH varying from 3.0 to 10.0. Substrate solutions were prepared by mixing 896 μL of 1% (w/w, in water) potato amylopectin (Sigma, Cat. No. 10118), 100 μL of 250 mM buffer working solution (pH from 3.0 to 10.0), and 4 μL of 0.5 M CaCl$_2$. Enzyme working solutions were prepared in water at a certain dose (showing signal within linear range as per dose response curve). All incubations were carried out as described previously for measuring the alpha amylase activity in Example 5. The absorbance from a buffer-only control was subtracted, and the resulting values were converted to percentages of relative activity, by defining the activity at the optimal pH as 100%. The pH profiles (Table 3A) and the pH optima and approximate pH range for ≥70% of activities (Table 3B) for these amylases, under the conditions of this assay, are shown below.

**Table 3A.** pH profiles of α-amylases

| pH | Relative activity (%) | | | |
|---|---|---|---|---|
| | EacAmy1 | EacAmy1-V1 | EsoAmy1 | EsoAmy1-V1 |
| 3 | 0 | 0 | 1 | 2 |
| 4 | -4 | -2 | -3 | 4 |
| 5 | 58 | 93 | 31 | 91 |
| 6 | 100 | 100 | 93 | 100 |
| 7 | 98 | 97 | 100 | 97 |
| 8 | 77 | 83 | 79 | 84 |
| 9 | 42 | 46 | 42 | 58 |
| 10 | 7 | 11 | 9 | 17 |
| pH | Relative activity (%) | | | |
| | EunAmy1 | EunAmy1-V1 | EoxAmy1 | EoxAmy1-V1 |
| 3 | 1 | 0 | -1 | 2 |
| 4 | -2 | -2 | -2 | 10 |
| 5 | 22 | 83 | 87 | 66 |
| 6 | 100 | 100 | 99 | 100 |

(continued)

| pH | Relative activity (%) | | | |
|----|---------|------------|---------|------------|
|    | EunAmy1 | EunAmy1-V1 | EoxAmy1 | EoxAmy1-V1 |
| 7  | 97      | 100        | 100     | 92         |
| 8  | 66      | 89         | 86      | 80         |
| 9  | 31      | 56         | 58      | 46         |
| 10 | 7       | 15         | 21      | 8          |

**Table 3B.** pH optima and pH range for ≥70% of activity of of α-amylases

| Amylase | pH optimum | pH range for ≥70% of activity |
|---------|------------|-------------------------------|
| EacAmy1 | 6.0-7.0 | 5.2-8.2 |
| EacAmy1-V1 | 6.0-7.0 | 4.7-8.4 |
| EsoAmy1 | 6.0-7.0 | 5.6-8.3 |
| EsoAmy1-V1 | 6.0 | 4.7-8.6 |
| EunAmy1 | 6.0-7.0 | 5.6-7.9 |
| EunAmy1-V1 | 6.0-7.0 | 4.8-8.6 |
| EoxAmy1 | 6.0-7.0 | 4.8-8.6 |
| EoxAmy1-V1 | 6.0 | 5.1-8.3 |

## Example 7

### Effect of temperature on amylase activities of *Exiguobacterium* amylases

The effect of temperature on the α-amylase activities of EacAmyl (SEQ ID NO: 7), EsoAmyl (SEQ ID NO: 8), EunAmyl (SEQ ID NO: 9), EoxAmyl (SEQ ID NO: 10), EacAmyl-V1 (SEQ ID NO: 12), EsoAmyl-V1 (SEQ ID NO: 13), EunAmyl-V1 (SEQ ID NO: 14), and EoxAmyl-V1 (SEQ ID NO: 17) were monitored using the PAHBAH assay protocol as described above, at temperatures ranging from 30°C to 95°C. Substrate solutions were prepared by mixing 3.6 mL of 1% (w/w, in water) potato amylopectin (Sigma, Cat. No. 10118), 0.4 mL of 0.5 M pH 5.0 sodium acetate buffer, and 16 μL of 0.5 M $CaCl_2$ into a 15-mL conical tube. Enzyme working solutions were prepared in water at a certain dose (showing signal within linear range as per dose response curve). Reactions were incubated at temperatures ranging from 30 to 95°C, for 10 min at 600 rpm in a thermomixer (Eppendorf). Alpha amylase activity was measured as described in Example 5. The absorbance from a buffer-only control was subtracted, and the resulting values were converted to percentages of relative activity, by defining the activity at the optimal temperature at 100%. The temperature profiles (Table 4A) and temperature optima and approximate temperature range for ≥70% of activity (Table 4B) for these amylases, under the conditions of this assay, are shown below.

**Table 4A.** Temperature profiles of a-amylases

| Temp. (°C) | Relative activity (%) | | | |
|------------|---------|------------|---------|------------|
|            | EacAmy1 | EacAmy1-V1 | EsoAmy1 | EsoAmy1-V1 |
| 30 | 74  | 93  | 98  | 84  |
| 40 | 100 | 100 | 100 | 100 |
| 50 | 90  | 79  | 80  | 91  |
| 60 | 73  | 35  | 61  | 74  |
| 70 | 59  | 12  | 58  | 52  |
| 80 | 56  | 4   | 49  | 33  |

(continued)

| Temp. (°C) | Relative activity (%) | | | |
|---|---|---|---|---|
| | EacAmy1 | EacAmy1-V1 | EsoAmy1 | EsoAmy1-V1 |
| 90 | 10 | 2 | 13 | 16 |
| 95 | 9 | 2 | 18 | 12 |

| Temp. (°C) | Relative activity (%) | | | |
|---|---|---|---|---|
| | EunAmy1 | EunAmy1-V1 | EoxAmy1 | EoxAmy1-V1 |
| 30 | 85 | 91 | 68 | 91 |
| 40 | 100 | 100 | 100 | 100 |
| 50 | 64 | 95 | 91 | 91 |
| 60 | 59 | 83 | 44 | 63 |
| 70 | 56 | 80 | 18 | 40 |
| 80 | 54 | 56 | 18 | 11 |
| 90 | 19 | 30 | 3 | 6 |
| 95 | 7 | 22 | 3 | 0 |

**Table 4B.** Temperature optima and Temperature range for ≥70% of activity for α-amylases

| Amylase | Temperature optimum | Temperature range for ≥70% of activity |
|---|---|---|
| EacAmy1 | 40 | <30-62 |
| EacAmy1-V1 | 40 | <30-52 |
| EsoAmy1 | 40 | <30-55 |
| EsoAmy1-V1 | 40 | <30-62 |
| EunAmy1 | 40 | <30-48 |
| EunAmy1-V1 | 40 | <30-75 |
| EoxAmy1 | 40 | 31-55 |
| EoxAmy1-V1 | 40 | <30-57 |

## Example 8

### Thermostability of *Exiguobacterium* amylases

The thermostabilities of EacAmyl (SEQ ID NO: 7), EsoAmyl (SEQ ID NO: 8), EunAmyl (SEQ ID NO: 9), EoxAmyl (SEQ ID NO: 10), EacAmyl-V1 (SEQ ID NO: 12), EsoAmyl-V1 (SEQ ID NO: 13), EunAmy1-V1 (SEQ ID NO: 14), and EoxAmyl-V1 (SEQ ID NO: 17) were measured by monitoring the enzyme activity before and after incubation at temperatures ranging from 40°C to 95°C for 2 h. Enzyme samples were diluted in 50mM sodium acetate buffer (pH 5.0) containing 2 mM $CaCl_2$ to appropriate concentration (showing signal within linear range as per dose response curve) and 40μl aliquots were added to PCR strip tubes. The tubes were transferred to a PCR machine at the desired temperature ranging from 40°C to 95°C. After incubation for 2 h, residual enzyme activity was assayed using the amylopectin/ PAHBAH method as described, above. The residual activities were converted to percentages of relative activity, by defining the activity of the sample kept on ice as 100% (Table 5).

**Table 5.** Thermostability of $\alpha$-amylases

| T (°C) | Residual activity (%) | | | | | | | |
|---|---|---|---|---|---|---|---|---|
| | Eac Amyl | Eac Amy1-V1 | Eso Amy1 | Eso Amy1-V1 | Eun Amy1 | Eun Amy1-V1 | Eox Amy1 | Eox Amy1-V1 |
| 40 | 95 | 28 | 93 | 12 | 91 | 82 | 0 | 96 |
| 45 | 101 | 4 | 95 | 0 | 79 | 75 | 0 | 91 |
| 50 | 62 | 1 | 70 | -1 | 49 | 72 | 0 | 91 |
| 55 | 12 | 1 | 31 | -3 | 16 | 74 | 1 | 88 |
| 60 | 1 | 1 | 2 | -3 | 1 | 60 | 0 | 82 |
| 65 | 3 | 1 | 0 | 0 | 1 | 20 | 0 | 48 |
| 70 | 3 | 1 | 2 | -2 | 1 | 2 | 0 | 7 |
| 75 | 3 | 0 | 2 | -3 | 2 | 2 | 0 | 1 |
| 80 | 4 | 0 | 0 | -1 | 1 | 4 | -1 | 1 |
| 85 | 3 | 1 | 3 | -2 | 1 | 3 | 0 | 0 |
| 90 | 2 | 2 | 2 | -2 | 1 | 4 | 0 | 0 |
| 95 | 4 | 1 | 0 | -1 | 1 | 3 | 0 | 0 |

**Example 9**

**Cleaning performance of *Exiguobacterium* amylases and variants thereof**

The cleaning performances of EsiAmyl (SEQ ID NO: 1), EsiAmyl-V1 (SEQ ID NO: 11), EacAmyl (SEQ ID NO: 7), EsoAmyl (SEQ ID NO: 8), EunAmyl (SEQ ID NO: 9), EoxAmyl (SEQ ID NO: 10), EacAmy1-V1 (SEQ ID NO: 12), EsoAmyl-V1 (SEQ ID NO: 13), and EunAmyl-V1 (SEQ ID NO: 14) were analyzed in a microswatch assay. Two samples of *Bacillus licheniformis* amylase (*i.e.*, LAT, PURASTAR®) were included as a benchmark (for EsiAmyl and EsiAmyl-V1). BASE (*i.e.*, SEQ ID NO: 2 in US8153412) amylase was included as a benchmark for assays with EacAmyl, EsoAmyl, EunAmyl, EoxAmyl, EacAmy1-V1, EsoAmy1-V1, and EunAmy1-V1. CS-28 rice starch on cotton swatches (Center for Testmaterials BV, Vlaardingen, Netherlands) containing an indicator dye bound to the starch, were pre-punched by the manufacturer to form discs measuring 5.5 mm in diameter. Two discs were placed in each well of flat-bottom non-binding 96-well assay plates. Prior to the assay, the swatches were pre-washed in Milli-Q water at 25°C with shaking at 350 rpm for 1 hour (water was changed every 20 minutes) (except for EsiAmyl and EsiAmy1-V1).

The cleaning assay was carried out in a buffer consisting of 25 mM HEPES (pH 8.2), with 2 mM CaCl$_2$ and 0.005% Tween-80 added. Filtered culture supernatants were diluted 1:100 in an enzyme dilution buffer (10 mM NaCl, 0.1 mM CaCl$_2$, 0.005% TWEEN 80). An appropriate volume of HEPES buffer (162 to 180 $\mu$L) was added to each well, and then varying amounts of the diluted enzyme solution, from 0 $\mu$L to 18 $\mu$L were added for a total volume of 180 $\mu$L in every well. The plates were incubated at 30°C or 25°C (for EsiAmyl and EsiAmyl-VI) with agitation at 1150 rpm for 15 minutes. Color release was quantified spectrophotometrically at 488 nm by the transfer of 100 $\mu$L of the final wash solution to a fresh medium-binding microtiter plate. Enzyme performance was judged by the amount of colour released into the wash liquor. For EsiAmyl and EsiAmy1-V1, the performances of the enzymes were compared to each other by subtracting the blank wells and fitting curves based on the actual concentration of each amylase, as determined by reversed phase HPLC.

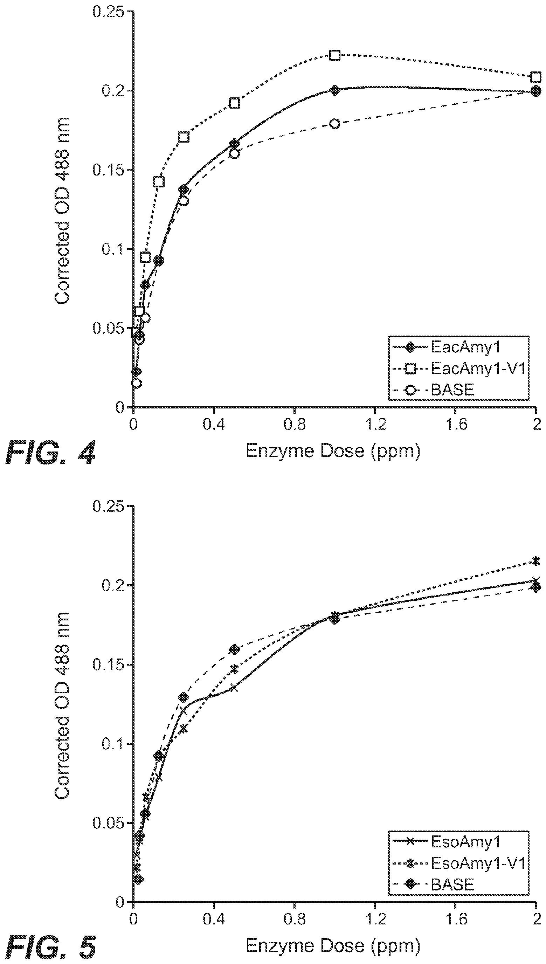

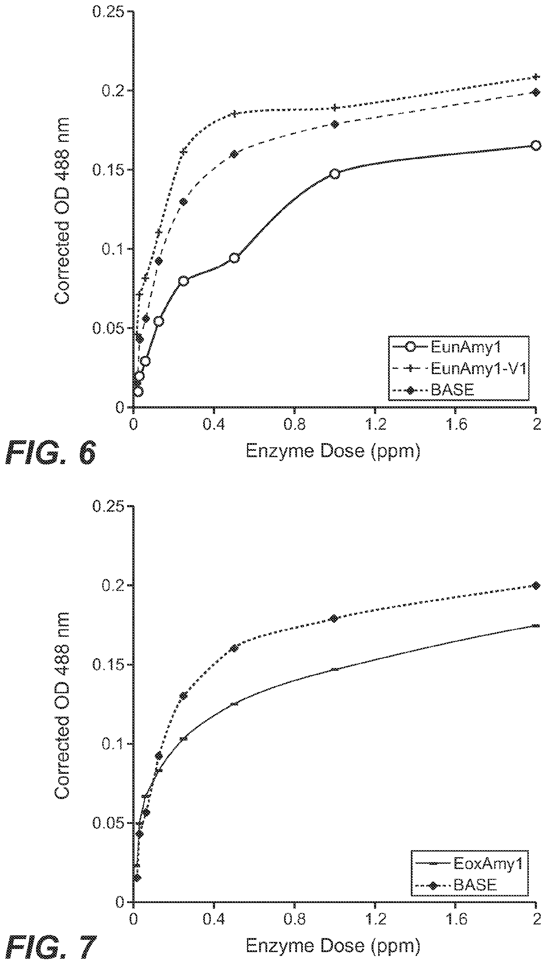

The cleaning performances of EsiAmyl and EsiAmyl-V1 are shown graphically in Figure 3, EacAmyl and EacAmy1-V1 in Figure 4, EsoAmyl and EsoAmy1-V1 in Figure 5, EunAmyl and EunAmy1-V1 in Figure 6, and EoxAmyl in Figure 7. The data indicates that the *Exiguobacterium* amylases are highly efficient at removing starchy stains from textile swatches. EsiAmyl and EsiAmy1-V1 are more effective than *B. licheniformis* alpha-amylase (LAT, PURASTAR®). The V1 variant of each individual *Exiguobacterium* amylase generally shows improved cleaning performance over its wild-type parent. As expected, the V1 variants of each individual *Exiguobacterium* amylase is more thermostable than its wild-type parent molecule (data not shown).

**Claims**

1. A recombinant *Exiguobacterium* α-amylase having α-amylase activity and comprising an amino acid sequence having at least 96% amino acid sequence identity to the amino acid sequence of SEQ ID NO: 4 or having at least 93% amino acid sequence identity to the amino acid sequence of SEQ ID NO: 5, wherein the alpha-amylase comprises a deletion of one of more residues corresponding to R179, G180, T181, or G182, and/or one or more substitutions selected from G242Q, T242Q, D188P, N188P, and G477K, referring to SEQ ID NO: 4 or SEQ ID NO: 5 for numbering.

2. The α-amylase of claim 1 further comprising conservative substitutions of one or several amino acid residues, and/or a deletion, substitution, insertion, or addition of one or a few amino acid residues other than 179, 180, 181, 182, 242, 188, and 477, referring to SEQ ID NO: 4 or SEQ ID NO: 5 for numbering.

3. A composition comprising the α-amylase of claim 1 of claim 2.

4. The composition of claim 3:

   (a) further comprising a surfactant; and/or
   (b) wherein the composition is a detergent composition; and/or
   (c) wherein the composition is a laundry detergent, a laundry detergent additive, or a manual or automatic dishwashing detergent; and/or
   (d) further comprising one or more additional enzymes selected from the group consisting of protease, hemicellulase, cellulase, peroxidase, lipolytic enzyme, metallolipolytic enzyme, xylanase, lipase, phospholipase, esterase, perhydrolase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase, laccase, metalloproteinase, amadoriase and an amylase other than a recombinant *Exiguobacterium* α-amylase.

5. The composition of claim 3, wherein:

   (a) the composition is for saccharifying a composition comprising starch, for SSF post liquefaction, or for direct SSF without prior liquefaction; or
   (b) the composition is for producing a fermented beverage or a baked food product; or
   (c) the composition is for textile desizing, optionally wherein the composition further comprises a surfactant.

6. A recombinant polynucleotide encoding a polypeptide of claim 1 or claim 2; optionally having at least 80% nucleic acid sequence identity to the polynucleotide of SEQ ID NO: 16.

7. An expression vector comprising the polynucleotide of claim 6.

8. A host cell comprising the expression vector of claim 7.

9. Use of the α-amylase of claim 1 or claim 2 in the production of a composition comprising glucose, in the production of a liquefied starch, in the production of a foodstuff or beverage, in cleaning starchy stains, or in textile desizing.

10. A method for removing a starchy stain or soil from a surface, comprising:

    contacting the surface with a composition comprising an effective amount of the recombinant *Exiguobacterium* α-amylase of claim 1 or claim 2; and
    allowing the α-amylase to hydrolyze starch components present in the starchy stain to produce smaller starch-derived molecules that dissolve in aqueous solution;
    thereby removing the starchy stain from the surface.

11. The method of claim 10, wherein:

    (a) the aqueous composition further comprises a surfactant; and/or
    (b) the surface is a textile surface or a surface on dish ware; and/or
    (c) the composition further comprises at least one additional enzymes selected from the group consisting of protease, hemicellulase, cellulase, peroxidase, lipolytic enzyme, metallolipolytic enzyme, xylanase, lipase, phospholipase, esterase, perhydrolase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase, laccase, metalloproteinase, amadoriase, and an amylase other than an *Exiguobacterium* α-amylase.

12. A method for desizing a textile comprising:

contacting a sized textile with an effective amount of the *Exiguobacterium* α-amylase of claim 1 or claim 2; and allowing the α-amylase to hydrolyze starch components in the size to produce smaller starch-derived molecules that dissolve in aqueous solution;
thereby removing the size from the textile.

13. A method for saccharifying a composition comprising starch to produce a composition comprising glucose, the method comprising:

contacting the composition comprising starch with effective amount of the *Exiguobacterium* α-amylase of claim 1 or claim 2; and
saccharifying the composition comprising starch to produce the composition comprising glucose;
wherein the α-amylase catalyzes the saccharification of the starch solution to glucose;
optionally wherein the composition comprising starch comprises liquefied starch, gelatinized starch, or granular starch.

14. A method for preparing a foodstuff or beverage comprising:

contacting a foodstuff or beverage comprising starch with the *Exiguobacterium* α-amylase of claim 1 or claim 2; and
allowing the α-amylase to hydrolyze the starch to produce smaller starch-derived molecules;
optionally further comprising contacting the foodstuff or beverage with glucoamylase, hexokinase, xylanase, glucose isomerase, xylose isomerase, phosphatase, phytase, pullulanase, β-amylase, α-amylase that is not the variant α-amylase, protease, cellulase, hemicellulase, lipase, cutinase, isoamylase, redox enzyme, esterase, transferase, pectinase, alpha-glucosidase, beta-glucosidase, or a combination thereof.

15. The method of any one of claims 10-14, wherein the α-amylase is expressed and secreted by a host cell, optionally:

(a) wherein the composition comprising starch is contacted with the host cell; and/or
(b) wherein the host cell further expresses and secretes a glucoamylase or other enzyme; and/or
(c) wherein the host cell is capable of fermenting the composition.

**Patentansprüche**

1. Rekombinante *Exiguobacterium* α-Amylase, die α-Amylase-Aktivität aufweist und eine Aminosäuresequenz umfasst, die mindestens 96% Aminosäuresequenz-Identität zu der Aminosäuresequenz von SEQ ID NO: 4 aufweist oder mit mindestens 93% Aminosäuresequenz-Identität zu der Aminosäuresequenz von SEQ ID NO: 5 aufweist, wobei die α-Amylase eine Deletion von einem oder mehreren Resten entsprechend R179, G180, T181 oder G182 und/oder eine oder mehrere Substitutionen umfasst, die ausgewählt sind aus G242Q, T242Q, D188P, N188P und G477K, wobei zur Nummerierung auf SEQ ID NO: 4 oder SEQ ID NO: 5 Bezug genommen wird.

2. α-Amylase nach Anspruch 1, ferner umfassend konservative Substitutionen von einem oder mehreren Aminosäureresten und/oder eine Deletion, Substitution, Insertion oder Addition von einem oder einigen anderen Aminosäureresten als 179, 180, 181. 182, 242, 188 und 477, wobei zur Nummerierung auf SEQ ID NO: 4 oder SEQ ID NO: 5 Bezug genommen wird.

3. Zusammensetzung, umfassend α-Amylase nach Anspruch 1 oder Anspruch 2

4. Zusammensetzung nach Anspruch 3:

(a) ferner umfassend ein Tensid; und/oder

(b) wobei die Zusammensetzung eine Detergens-Zusammensetzung ist; und/oder

(c) wobei die Zusammensetzung ein Waschmittel, ein Waschmitteladditiv oder ein manuelles oder automatisches Geschirrspülmittel ist; und/oder

(d) ferner umfassend ein oder mehrere zusätzliche Enzyme, die ausgewählt sind aus der Gruppe bestehend aus Protease, Hemicellulase, Cellulase, Peroxidase, lipolytischem Enzym, metallolipolytischem Enzym, Xylanase, Lipase, Phospholipase, Esterase, Perhydrolase. Cutinase, Pektinase, Pektatlyase, Mannanase, Keratinase, Reduktase, Oxidase, Phenoloxidase, Lipoxygenase, Ligninase, Pullulanase, Tannase, Pentosanase, Malanase, $\beta$-Glucanase, Arabinosidase, Hyaluronidase, Chondroitinase, Laccase, Metalloproteinase, Amadoriase und anderer Amylase als eine rekombinante *Exiguobacterium* $\alpha$-Amylase.

5. Zusammensetzung nach Anspruch 3, wobei:

(a) wobei die Zusammensetzung zur Verzuckerung einer Stärke umfassenden Zusammensetzung zur SSF-Nachverflüssigung oder zur direkten SSF ohne vorherige Verflüssigung dient; oder

(b) die Zusammensetzung zum Herstellen eines fermentierten Getränks oder eines gebackenen Lebensmittelprodukts dient; oder

(c) die Zusammensetzung zur Textilentschlichtung dient, wobei die Zusammensetzung gegebenenfalls ferner ein Tensid aufweist.

6. Rekombinantes Polynucleotid, das ein Polypeptid nach Anspruch 1 oder Anspruch 2 codiert; gegebenenfalls aufweisend mindestens 80% Nucleinsäuresequenz-Identität zu dem Polynucleotid von SEQ ID NO: 16.

7. Expressionsvektor, umfassend das Polynucleotid nach Anspruch 6.

8. Wirtszelle umfassend den Expressionsvektor nach Anspruch 7.

9. Verwendung der $\alpha$-Amylase nach Anspruch 1 oder Anspruch 2 in der Herstellung einer Glucose umfassenden Zusammensetzung, in der Herstellung einer verflüssigten Stärke, in der Herstellung eines Lebensmittels oder Getränks, in der Reinigung von Stärkeflecken oder beim Textilentschlichten.

10. Verfahren zum Entfernen eines Stärkeflecks oder von Schmutz von einer Oberfläche, umfassend:

Kontaktieren einer Oberfläche mit einer Zusammensetzung, die eine wirksame Menge der rekombinanten *Exiguobacterium* $\alpha$-Amylase nach Anspruch 1 oder Anspruch 2 umfasst; und

die $\alpha$-Amylase die in dem Stärkefleck vorhandenen Stärkekomponenten hydrolysieren lassen, um kleinere stärkederivierte Moleküle zu erzeugen, die sich in wässriger Lösung auflösen;

dadurch Entfernen des Stärkeflecks von der Oberfläche.

11. Verfahren nach Anspruch 10, wobei:

(a) die wässrige Zusammensetzung ferner ein Tensid umfasst; und/oder

(b) die Oberfläche eine Textiloberfläche oder ein Oberfläche auf Geschirr ist; und/oder

(c) die Zusammensetzung ferner mindestens eines von zusätzlichen Enzymen umfasst, die ausgewählt sind aus der Gruppe bestehend aus Protease, Hemicellulase, Cellulase, Peroxidase, lipolytischem Enzym, metallolipolytischem Enzym, Xylanase, Lipase, Phospholipase, Esterase, Perhydrolase. Cutinase, Pektinase, Pektatlyase, Mannanase, Keratinase, Reduktase, Oxidase, Phenoloxidase, Lipoxygenase, Ligninase, Pullulanase, Tannase, Pentosanase, Malanase, $\beta$-Glucanase, Arabinosidase, Hyaluronidase, Chondroitinase, Laccase, Metalloproteinase, Amadoriase und einer anderen Amylase als eine *Exiguobacterium* $\alpha$-Amylase.

12. Verfahren zum Entschlichten eines Textilerzeugnisses, umfassend:

Kontaktieren eines beschlichteten Textilerzeugnisses mit einer wirksamen Menge der *Exiguobacterium* $\alpha$-Amylase nach Anspruch 1 oder Anspruch 2; und

die $\alpha$-Amylase die in der Schlichte vorhandenen Stärkekomponenten hydrolysieren lassen, um kleinere stärkederivierte Moleküle zu erzeugen, die sich in wässriger Lösung auflösen;

dadurch Entfernen der Schlichte von dem Textilerzeugnis.

**13.** Verfahren zum Verzuckern einer Stärke umfassenden Zusammensetzung, um eine Glucose umfassende Zusammensetzung zu erzeugen, wobei das Verfahren umfasst:

Kontaktieren einer Oberfläche mit einer Zusammensetzung, die eine effektive Menge der rekombinanten *Exiguobacterium* α-Amylase nach Anspruch 1 oder Anspruch 2 umfasst; und
Verzuckern der Stärke umfassenden Zusammensetzung, um die Glucose umfassende Zusammensetzung zu erzeugen;
wobei die α-Amylase die Verzuckerung der Stärkelösung zu Glucose katalysiert;
gegebenenfalls wobei die Stärke umfassende Zusammensetzung verflüssigte Stärke, gelatinierte Stärke oder granuläre Stärke umfasst.

**14.** Verfahren zum Herstellen eines Lebensmittels oder Getränks, umfassend:

das Kontaktieren des Stärke umfassenden Lebensmittels oder Getränks mit *Exiguobacterium* α-Amylase nach Anspruch 1 oder Anspruch 2; und
die α-Amylase die Stärke hydrolysieren lassen, um kleinere stärkederivierte Moleküle zu erzeugen;
gegebenenfalls ferner umfassend Kontaktieren des Lebensmittels oder Getränks mit Glucoamylase, Hexokinase, Xylanase, Glucoseisomerase, Xyloseisomerase, Phosphatase, Phytase, Pullulanase, β-Amylase, α-Amylase, die keine variante α-Amylase, Protease. Cellulase, Hemicellulase, Lipase, Cutinase, Isoamylase, Redoxenzym, Esterase, Transferase, Pektinase, Alpha-Glucosidase, Beta-Glucosidase oder einer Kombination davon ist.

**15.** Verfahren nach einem der Ansprüche 10 bis 14, wobei die α-Amylase durch eine Wirtszelle exprimiertes und sekretiertes Enzym ist, gegebenenfalls:

(a) wobei die Stärke umfassende Zusammensetzung mit der Wirtszelle in Kontakt gebracht wird; und/oder
(b) wobei die Wirtszelle ferner eine Glucoamylase oder ein anderes Enzym exprimiert oder sekretiert; und/oder
(c) wobei die Wirtszelle zum Fermentieren der Zusammensetzung in der Lage ist.

## Revendications

**1.** α-amylase d'*Exiguobacterium* recombinante présentant une activité α-amylase et comprenant une séquence d'acides aminés qui présente une identité de séquence d'acides aminés d'au moins 96 % par rapport à la séquence d'acides aminés de SEQ ID NO: 4 ou qui présente une identité de séquence d'acides aminés d'au moins 93 % par rapport à la séquence d'acides aminés de SEQ ID NO: 5, dans laquelle l'alpha-amylase comprend une délétion d'un résidu ou de plusieurs résidus correspondant à R179, G180, T181 ou G182, et/ou une ou plusieurs substitution(s) qui est/sont sélectionnée(s) parmi G242Q, T242Q, D188P, N188P et G477K, en faisant référence à SEQ ID NO: 4 ou à SEQ ID NO: 5 pour la numérotation.

**2.** α-amylase selon la revendication 1, comprenant en outre des substitutions conservatrices d'un résidu ou de plusieurs résidus d'acides aminés, et/ou une délétion, une substitution, une insertion ou une addition d'un résidu ou de quelques résidus d'acides aminés autres que 179, 180, 181, 182, 242, 188 et 477, en faisant référence à SEQ ID NO: 4 ou à SEQ ID NO: 5 pour la numérotation.

**3.** Composition comprenant l'a-amylase selon la revendication 1 ou la revendication 2.

**4.** Composition selon la revendication 3 :

(a) comprenant en outre un agent tensioactif ; et/ou
(b) dans laquelle la composition est une composition de détergent ; et/ou
(c) dans laquelle la composition est un détergent lessiviel, un additif de détergent lessiviel ou un détergent pour la vaisselle à la main ou à la machine ; et/ou
(d) comprenant en outre une ou plusieurs enzyme(s) additionnelle(s) qui est/sont sélectionnée(s) parmi le groupe qui est constitué par la protéase, l'hémicellulase, la cellulase, la peroxydase, une enzyme lipolytique, une enzyme métallolipolytique, la xylanase, la lipase, la phospholipase, l'estérase, la perhydrolase, la cutinase, la pectinase, la lyase de pectate, la mannanase, la kératinase, la réductase, l'oxydase, la phénoloxydase, la lipoxygénase, la ligninase, la pullulanase, la tannase, la pentosanase, la malanase, la β-glucanase, l'arabinosidase, l'hyaluronidase, la chondroitinase, la laccase, la métalloprotéinase, l'amadoriase et une amylase autre qu'une α-amylase d'*Exiguobacterium* recombinante.

5. Composition selon la revendication 3, dans laquelle :

   (a) la composition est destinée à la saccharification d'une composition qui comprend de l'amidon, à la post-liquéfaction de SSF ou à la SSF directe sans liquéfaction préliminaire ; ou
   (b) la composition est destinée à la production d'une boisson fermentée ou d'un produit alimentaire cuit au four ; ou
   (c) la composition est destinée au désencollage des textiles, en option dans laquelle la composition comprend en outre un agent tensioactif.

6. Polynucléotide recombinant codant un polypeptide selon la revendication 1 ou la revendication 2 ; en option, présentant une identité de séquence d'acides nucléiques d'au moins 80 % par rapport au polynucléotide de SEQ ID NO: 16.

7. Vecteur d'expression comprenant le polynucléotide selon la revendication 6.

8. Cellule hôte comprenant le vecteur d'expression selon la revendication 7.

9. Utilisation de l'a-amylase selon la revendication 1 ou la revendication 2 au niveau de la production d'une composition qui comprend du glucose, au niveau de la production d'un amidon liquéfié, au niveau de la production d'un produit alimentaire ou d'une boisson, au niveau du nettoyage de taches amylacées ou au niveau du désencollage des textiles.

10. Procédé pour enlever une tache amylacée ou une salissure d'une surface, comprenant :

    la mise en contact de la surface avec une composition qui comprend une quantité efficace de l'a-amylase d'*Exiguobacterium* recombinante selon la revendication 1 ou la revendication 2 ; et
    le fait de permettre que l'a-amylase hydrolyse les composants d'amidon qui sont présents dans la tache amylacée de manière à produire des molécules plus petites dérivées de l'amidon qui se dissolvent dans une solution aqueuse ;
    d'où ainsi l'enlèvement de la tache amylacée de la surface.

11. Procédé selon la revendication 10, dans lequel :

    (a) la composition aqueuse comprend en outre un agent tensioactif ; et/ou
    (b) la surface est une surface de textile ou une surface sur de la vaisselle ; et/ou
    (c) la composition comprend en outre au moins une enzyme additionnelle qui est sélectionnée parmi le groupe qui est constitué par la protéase, l'hémicellulase, la cellulase, la peroxydase, une enzyme lipolytique, une enzyme métallolipolytique, la xylanase, la lipase, la phospholipase, l'estérase, la perhydrolase, la cutinase, la pectinase, la lyase de pectate, la mannanase, la kératinase, la réductase, l'oxydase, la phénoloxydase, la lipoxygénase, la ligninase, la pullulanase, la tannase, la pentosanase, la malanase, la β-glucanase, l'arabinosidase, l'hyaluronidase, la chondroitinase, la laccase, la métalloprotéinase, l'amadoriase et une amylase autre qu'une α-amylase d'*Exiguobacterium.*

12. Procédé pour désencoller un textile comprenant
    la mise en contact d'un textile chargé de produits collants avec une quantité efficace de l'a-amylase d'*Exiguobacterium* selon la revendication 1 ou la revendication 2 : et
    le fait de permettre que l'a-amylase hydrolyse les composants d'amidon qui sont présents dans la charge de produits collants de manière à produire des molécules plus petites dérivées de l'amidon qui se dissolvent dans une solution aqueuse ;
    d'où ainsi l'enlèvement de la charge de produits collants du textile.

13. Procédé pour saccharifier une composition qui comprend de l'amidon de manière à produire une composition qui comprend du glucose, le procédé comprenant :

    la mise en contact de la composition qui comprend de l'amidon avec une quantité efficace de l'a-amylase d'*Exiguobacterium* selon la revendication 1 ou la revendication 2 : et

la saccharification de la composition qui comprend de l'amidon de manière à produire la composition qui comprend du glucose ;

dans lequel l'$\alpha$-amylase catalyse la saccharification de la solution d'amidon en glucose ;

en option, dans lequel la composition qui comprend de l'amidon comprend de l'amidon liquéfié, de l'amidon gélatinisé ou de l'amidon granulaire.

14. Procédé pour préparer un produit alimentaire ou une boisson comprenant :

la mise en contact d'un produit alimentaire ou d'une boisson qui comprend de l'amidon avec l'a-amylase d'*Exiguobacterium* selon la revendication 1 ou la revendication 2 ; et

le fait de permettre que l'a-amylase hydrolyse l'amidon de manière à produire des molécules plus petites dérivées de l'amidon ;

en option, comprenant en outre la mise en contact du produit alimentaire ou de la boisson avec la glucoamylase, l'hexokinase, la xylanase, l'isomérase de glucose, l'isomérase de xylose, la phosphatase, la phytase, la pullulanase, la $\beta$-amylase, une $\alpha$-amylase qui n'est pas la variante de l'a-amylase, la protéase, la cellulase, l'hémicellulase, la lipase, la cutinase, l'isoamylase, une enzyme redox, l'estérase, la transférase, la pectinase, l'alpha-glucosidase, la bêta-glucosidase ou une combinaison de celles-ci.

15. Procédé selon l'une quelconque des revendications 10 à 14, dans lequel l'$\alpha$-amylase est exprimée et secrétée par une cellule hôte, en option :

(a) dans lequel la composition qui comprend de l'amidon est mise en contact avec la cellule hôte ; et/ou

(b) dans lequel la cellule hôte exprime et secrète en outre une glucoamylase ou une autre enzyme ; et/ou

(c) dans lequel la cellule hôte dispose de la capacité de faire fermenter la composition.

CLUSTAL W (1.83) multiple sequence alignment

```
                                                                   60
                                                                   |
EanAmyl    ATADNGTMMQYFEWYLPNDGNHWNRLNTDTTKLDQLGITSVWIPPAYKGTTQNDVGYGAY
EsoAmyl    ATADNGTMMQYFEWYLPNDGNHWNRLNTDTTKLDQLGITSVWIPPAYKGTTQNDVGYGAY
EacAmyl    ATADNGTMMQYFEWYVPNDGNHWNRLGSDATKLDQLGITSVWIPPAYKGTSQNDVGYGAY
EsiAmyl    ---DNGTMMQYFEWYVPNDGNHWNRLGSDSTKLDQLGITSVWIPPAYKGTTQNDVGYGAY
EunAmyl    ATADNGTMMQYFEWYLPNDGNHWNRLSSDTTKLDQLGITSVWIPPAYKGTSQNDVGYGAY
EoxAmyl    ATADNGTMMQYFEWYLPNDGNHWNRLGNDASKLDQLGITSVWIPPAYKGTTQNDVGYGAY
BLA        -ANLNGTLMQYFEWYMPNDGQHWKRLQNDSAYLAEHGITAVWIPPAYKGTSQADVGYGAY
BAA        ---VNGTLMQYFEWYTPNDGQHWKRLQNDAEHLSDIGITAVWIPPAYKGLSQSDNGYGPY
BSG        AAPFNGTMMQYFEWYLPDDGTLWTKVANEANNLSSLGITALWLPPAYKGTSRDVGYGVY
               ***:******* *:** *.:: .:: *  . ***::*:***** :: * *** *

                                                                   120
                                                                   |
EanAmyl    DLYDLGEFNQKGTVRTKYGTKAQLKTAISQLHTAGIDVYGDVVMNHKGGADFTESVTAVE
EsoAmyl    DLYDLGEFNQKGTVRTKYGTKAQLKTAISQLHTAGIDVYGDVVMNHKGGADFTEAVTAVE
EacAmyl    DLYDLGEFNQKGTVRTKYGTKAQLKTAIGQLHTAGIDVYGDVVMNHKGGADFTEAVTAVE
EsiAmyl    DLYDLGEFNQKGTVRTKYGTKAQLKTAINQLHTAGIDVYGDVVMNHKGGADFTEAVTAVE
EunAmyl    DLYDLGEFNQKGTVRTKYGTKAQLKSAINQLHTAGIDVYGDVVMNHKGGADFTESVTAVE
EoxAmyl    DLYDLGEFNQKGTVRTKYGTKTQLKSAIGQLHTAGIDVYGDVVMNHKGGADFTESVTAVE
BLA        DLYDLGEFHQKGTVRTKYGTKGELQSAIKSLHSRDINVYGDVVINHKGGADATEDVTAVE
BAA        DLYDLGEFQQKGTVRTKYGTKSELQDAIGSLHSRNVQVYGDVVLNHKAGADATEDVTAVE
BSG        DLYDLGEFNQKGTVRTKYGTKAQYLQAIQAAHAAGMQVYADVVFDHKGGADGTEWVDAVE
           ********:***************:   **   *:  .::**,***::**.*** ** * ***

                                                                   180
                                                                   |
EanAmyl    VNGGNRNQEVSGDYQIQAWTGFDFAARNNTYSNFKWKWYHFDGTDWDQSRSKSAIYKFRG
EsoAmyl    VNGSNRNQEVSGDYQIQAWTGFDFAARNNTYSNFKWKWYHFDGTDWDQSRSKSAIYKFRG
EacAmyl    INPGNRNQEISGDYQIQAWTGFNFAARNNLYSNFKWKWYHFDGTDWDQSRSKSAIYKFRG
EsiAmyl    VNGSNRNQEISGDYQIQAWTGFDFAARNNTYSNFKWKWYHFDGTDWDQSRSKSAIYKFRG
EunAmyl    VNGGNRNQEISGDYQIQAWTGFNFATRNNAYSNFKWKWYHFDGTDWDQSRSKSAIYKFRG
EoxAmyl    VNPGNRNQEVSGDYQIQAWTGFNFAARSNAYSNFKWKWYHFDGTDWDQSRSKSAIYKFRG
BLA        VDPADRNRVISGEHRIKAWTHFHFPGRGSTYSDFKWHWYHFDGTDWDESRKLNRIYKF--
BAA        VNPANRNQETSEEYQIKAWTDFRFPGRGNTYSDFKWHWYHFDGADWDESRKISRIFKFRG
BSG        VNPSDRNQEISGTYQIQAWTKFDFPGRGNTYSSFKWRWYHFDGVDWDESRKLSRIYKFRG
           :: .:**: *  ::*:*** * *. *.. **.***:***** .***:**. . *:**

                                                                   240
                                                                   |
EanAmyl    TGKAWDTDVSTENGNYDYLMYADIDFDHPEVQQEMKNWGKWYVNELGLDGFRLDAVKHIK
EsoAmyl    TGKAWDTDVSTENGNYDYLMYADIDFDHPEVQQEMKNWGKWYVNELGLDGFRLDAVKHIK
EacAmyl    TGKAWDTDVSTENGNYDYLMYADLDFDHPEVQQEMKNWGKWYVNELGLDGFRLDAVKHIK
EsiAmyl    TGKAWDTDVSTENGNYDYLMYADIDFDHPEVQQEMKNWGKWYVNELGLDGFRLDAVKHIK
EunAmyl    TGKAWDTDVSTENGNYDYLMYADVDFDHPEVQQEMKNWGKWYVNELGLDGFRLDAVKHIK
EoxAmyl    TGKSWDSNVSSENGNYDYLMYADIDFDHPEVQQEMKNWGKWYVNELGLDGFRLDAVKHIK
BLA        QGKAWDWEVSNENGNYDYLMYADIDYDHPDVAAEIKRWGTWYANELQLDGFRLDAVKHIK
BAA        EGKAWDWEVSSENGNYDYLMYADVDYDHPDVVAETKKWGIWYANELSLDGFRIDAAKHIK
BSG        IGKAWDWEVDTENGNYDYLMYADLDMDHPEVVTELKNWGKWYVNTTNIDGFRLDAVKHIK
           **:** :*..*********:* ***:* * *.** **.* :****:**.****
```

*FIG. 1A*

300
:

```
EanAmy1    HGYLADWLANVRQTTGKPLFTVAEYWQNDLGTLQNYLSRTNYQQSVFDAPLHYKFEQASK
EsoAmy1    HGYLADWLANVRQTTGKPLFTVAEYWQNDLGTLQNYLSRTNYQQSVFDAPLHYKFEQASK
EacAmy1    HGYLADWLANVRQTTGKPLFTVAEYWQNDLGTLQNYLSRTNYQQSVFDAPLHYKFEQASK
EsiAmy1    HGYLADWLANVRQTTGKPLFTVAEYWQNDLGTLQNYLSRTNYQQSVFDAPLHYKFEQASK
EunAmy1    HGYLADWLANVRQTTGKPLFTVAEYWQNDLGTLKNYLSRTNYKQSVFDAPLHYKFEQASK
EoxAmy1    HTYLADWLTNVRQTTGKELFTVAEYWQNDLGTLKNYLSQTNYKQSVFDAPLHYKFEQASK
BLA        FSFLRDWVNHVREKTGKEMFTVAEYWQNDLGALENYLNKTNFNHSVFDVPLHYQFHAAST
BAA        FSFLRDWVQAVRQATGKEMFTVAEYWQNNAGKLENYLNKTSFNQSVFDVPLHFNLQAASS
BSG        FSFFPDWLSYVRSQTGKPLFTVGEYWSYDINKLHNYITKTDGTMSLFDAPLHNKFYTASK
           . :: **: **. *** :**.***. : . .*.**:.:*.    *:**.*** :: **.
```

360
:

```
EanAmy1    GGGYYDMRTIFDGTLVKSNPVQAVTLVENHDSQPGQSLESTVQSWFKPLAYAMILTREQG
EsoAmy1    GGGYYDMRTIFDGTLVKSNPVQAVTLVENHDSQPGQSLESTVQSWFKPLAYAMILTREQG
EacAmy1    GGGYYDMRTIFDGTLVKSNPVQAVTLVENHDSQPGQSLESTVQSWFKPLAYAMILTREQG
EsiAmy1    GGGYYDMRTIFDGTLVKTNPVQAVTLVENHDSQPGQSLESTVQSWFKPLAYAMILTREQG
EunAmy1    GGGYYDMRTIFNGTVVVQDNPTLAVTLVENHDSQPGQSLESTVQPWFKPLAYAMILTREQG
EoxAmy1    GGGFYDMRTIFNGTLVQDNPTLAVTLVENHDSQPGQSLESTVQSWFKPLAYAMILTREQG
BLA        QGGYYDMRKLLNSTVVSKHPLKAVTFVDNHDTQPGQSLESTVQTWFKPLAYAFILTRESG
BAA        QGGGYDMRRLLDGTVVSRHPEKAVTFVENHDTQPGQSLESTVQTWFKPLAYAFILTRESG
BSG        SGGAFDMRTLMTNTLMKDQPTLAVTFVDNHDTEPGQALQSWVDPWFKPLAYAFILTRQEG
           ** :*** :: .*::. :* ***:*:***::***:*:* *:.********:****:.*
```

420
:

```
EanAmy1    YPSVFYGDYYGTKGTSNREIPALGSKIDPLLKARKDFAFGKQNDYLDNADVIGWTREGVT
EsoAmy1    YPSVFYGDYYGTKGTSNREIPALGSKIDPLLKARKDFAFGKQNDYLDNADVIGWTREGVT
EacAmy1    YPSVFYGDYYGTKGTSNREIPALASKIDPLLKARKDFAFGKQNDYLDNQDIIGWTREGVS
EsiAmy1    YPSVFYGDYYGTKGTSNREIPALASKIDPLLKARKDFAFGKQNDYLDNADVIGWTREGVT
EunAmy1    YPSVFYGDYYGTKGTSNREIPALGSKIDPLLKARKDFAYGKQNDYLDNADVIGWTREGVT
EoxAmy1    YPSVFYGDYYGTKGSSNREIPALASKIDPILKARKDYAFGKQNDYLDNPDVIGWTREGVS
BLA        YPQVFYGDMYGTKGDSQREIPALKHKIEPILKARKQYAYGAQHDYFDHHDIVGWTREGDS
BAA        YPQVFYGDMYGTKGTSPKEIPSLKDNIEPILKARKEYAYGPQHDYIDHPDVIGWTREGDS
BSG        YPCVFYGDYYGI---PQYNIPSLKSKIDPLLIARRDYAYGTQHDYLDHSDIIGWTREGGT
           ** ***** **    . :**:* :*:*:* **::*:* *:**:*: *:*****:* :
```

480
:

```
EanAmy1    DRAKSGLATILSDGPGGSKWMYVGTQNKGEVWTDITGNNTASVTINQDGYGQFFVNGGSV
EsoAmy1    DRAKSGLATILSDGPGGSKWMYVGTQNKGEVWTDITGNNSASVTINQDGYGQFFVNGGSV
EacAmy1    DRAKSGLATILSDGPGGSKWMYVGLQNKGEVWTDITGNNTASVTINQDGYGQFFVNGGSV
EsiAmy1    DRAKSGLATILSDGPGGSKWMYVGLQNKGEVWTDITGNNTASVTINQDGYGQFFVNGGSV
EunAmy1    DRAKSGLATILSDGPGGSKWMYVGTQNKGEVWTDITGNNSASVTINQDGYGQFFVNGGSV
EoxAmy1    DRSKSGLATILSDGPGGSKWMYVGTQNKGEVWTDITGNNSASVTINADGYGQFFVNGGSV
BLA        SVANSGLAALITDGPGGAKRMYVGRQNAGETWHDITGNRSEPVVINSEGWGEFHVNGGSV
BAA        SAAKSGLAALITDGPGGSKRMYAGLKNAGETWYDITGNRSDTVKIGSDGWGEFHVNDGSV
BSG        EKPGSGLAALITDGPGGSKWMYVGKQHAGKVFYDLTGNRSDTVTINSDGWGEFKVNGGSV
           . . ****:::**:****:* **.* :: *:.: *:***.: .* *. :*:*:* **.***
```

*FIG. 1B*

54

```
                              486
                               |
EanAmy1              SVYRQQ (SEQ ID NO: 6)
EsoAmy1              SVYRQQ (SEQ ID NO: 3)
EacAmy1              SVYRQQ (SEQ ID NO: 2)
EsiAmy1              SVYRQQ (SEQ ID NO: 1)
EunAmy1              SVYRQQ (SEQ ID NO: 4)
EoxAmy1              SIYRQQ (SEQ ID NO: 5)
BLA                  SIYVQR (SEQ ID NO: 18)
BAA                  SIYVQK (SEQ ID NO: 19)
BSG                  SVWVPR (SEQ ID NO: 20)
                     *::   :
```

## FIG. 1C

**FIG. 2**

**FIG. 3**

FIG. 4

FIG. 5

**FIG. 6**

**FIG. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 20100104675 A
- US 8058033 B
- WO 9117243 A
- EP 238023 A
- US 6022725 A
- US 5281526 A
- US 5422267 A
- WO 2011153516 A2
- US 20060094080 A
- US 20070004018 A
- US 20070015266 A
- WO 9200381 A
- WO 0004136 A
- WO 8402921 A
- WO 9928448 A
- US RE32153 E
- US 4587215 A
- EP 135138 A
- WO 8601831 A
- WO 9500636 A
- US 6077316 A
- US 4106991 A
- US 4661452 A
- GB 1483591 A
- EP 238216 A
- WO 9206154 A
- WO 2005056783 A
- WO 9219709 A
- WO 9219708 A
- WO 8906279 A
- WO 8906270 A
- WO 9425583 A
- WO 9219729 A
- WO 9820115 A
- WO 9820116 A
- WO 9834946 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- WO 9535381 A
- WO 9600292 A
- WO 9530744 A
- WO 9425578 A
- WO 9514783 A
- WO 9522615 A
- WO 9704079 A
- WO 9707202 A
- EP 407225 A
- EP 260105 A
- WO 0134899 A
- WO 0114629 A
- US 6933140 B
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- WO 9812307 A
- WO 9524471 A
- DK 9800299 W
- EP 531315 A
- US 5457046 A
- US 5686593 A
- US 5763254 A
- WO 9324618 A
- WO 9510602 A
- WO 9815257 A
- WO 2010065455 A
- WO 2011072099 A
- WO 2011130222 A
- WO 2011140364 A
- WO 2011156297 A
- WO 2011156298 A
- WO 2011130076 A
- WO 2011133381 A
- EP 1794295 B1
- US 20110195481 A
- US 20110212876 A
- US 20110257063 A
- WO 2010039958 A
- WO 2011072117 A
- WO 2011098531 A

- WO 2011100410 A
- WO 2011140316 A
- US 20070215184 A
- US 20070251545 A
- US 20090075857 A
- US 20090137444 A
- US 20090143271 A
- US 20100011513 A
- US 20100093588 A
- US 20110201536 A
- US 20110232004 A
- US 20110237482 A
- US 20110312868 A
- US 20120003326 A
- US 20120004155 A
- WO 2011131585 A
- EP 707628 B1
- US 5719115 A
- EP 736084 B1
- US 5783545 A
- EP 767830 B1
- US 5972668 A
- EP 746599 B1
- US 5798328 A
- EP 662117 B1
- US 5898025 A
- US 6380140 B
- EP 898613 B1
- US 3975280 A
- US 6191092 B
- US 6329333 B
- US 6530386 B
- EP 1307547 B1
- US 7153818 B
- EP 1421169 B1
- US 6979669 B
- EP 1529101 B1
- US 7375070 B
- EP 1385943 B1
- US 7888104 B
- EP 1414977 B1
- US 5855625 A
- EP 1921147 B1
- EP 1921148 B1
- EP 701605 B1
- EP 1633469 B1
- EP 1633470 B1
- EP 1794293 B1
- EP 171007 B1
- US 4692260 A
- US 7569226 B
- EP 1165737 B1
- US 6391838 B
- US 6060441 A
- US 2009017074 A
- US 7320887 B
- EP 1737952 B1
- US 7691618 B
- US 20070256251 A
- US 20050261156 A
- US 20050261158 A
- US 20100234267 A
- US 20110136720 A
- US 7811076 B
- US 5929017 A
- US 5156773 A
- EP 2343310 A1
- WO 2011083114 A
- EP 214761 B1
- US 4876024 A
- EP 675944 B1
- US 5763383 A
- EP 517761 B1
- US 6624129 B
- EP 1054956 B1
- US 6939702 B
- US 6964944 B
- EP 832174 B1
- US 20060205628 A
- US 20070179076 A
- US 20080023031 A
- US 20110015110 A
- US 20110028372 A
- US 4973417 A
- US 5447649 A
- US 5840677 A
- US 5965503 A
- US 5972873 A
- US 5998344 A
- US 6071356 A
- WO 9009428 A
- EP 1661978 A1
- EP 1698689 A1
- EP 1726636 A1
- EP 1867707 A1
- EP 1876226 A1
- EP 1876227 A1
- EP 0205208 A2
- EP 0206390 A2
- EP 0271152 A
- EP 0271154 A
- EP 0341999 A
- EP 0346136 A
- EP 2135934 A
- US 20120208734 A
- WO 2011127102 A
- WO 2012142087 A
- WO 2012145062 A
- EP 1790713 B1
- US 8066818 B2
- US 8163686 B2
- US 8283300 B2
- US 8354366 B2
- US 20120125374 A
- US 3929678 A
- US 20100021587 A

- WO 0214490 A
- US 8206966 B

- US 20120045817 A
- US 8153412 B

**Non-patent literature cited in the description**

- **THOMPSON et al.** *Nucleic Acids Res.,* 1994, vol. 22, 4673-4680
- **VISHNIVETSKAYA, T.A. et al.** *Extremophiles,* 2009, vol. 13, 541-55
- **HARRISON et al.** *Applied Environ. Microbiol.,* 2011, vol. 77, 3916-22
- **LIU et al.** Improved heterologous gene expression in Trichoderma reesei by cellobiohydrolase I gene (cbh1) promoter optimization. *Acta Biochim. Biophys. Sin (Shanghai),* 2008, vol. 40 (2), 158-65
- **SAMBROOK et al.** MOLECULAR CLONING: A LABORATORY MANUAL. Cold Spring Harbor, 1989
- MOLECULAR CLONING: A LABORATORY MANUAL. 2001
- **CAO et al.** *Science,* 2000, vol. 9, 991-1001
- **CAMPBELL et al.** *Curr. Genet.,* 1989, vol. 16, 53-56
- **SUMITANI et al.** New type of starch-binding domain: the direct repeat motif in the C-terminal region of Bacillus sp. 195 $\alpha$-amylase contributes to starch binding and raw starch degrading. *Biochem. J.,* 2000, vol. 350, 477-484
- **SUMITANI et al.** *Biochem. J.,* 2000, vol. 350, 477-484
- **LIU et al.** *Sheng Wu Gong Cheng Xue Bao,* 2011, vol. 27, 1049-56
- **PAPAGIANNI.** *Biotechnol. Adv.,* 2007, vol. 25, 244-63
- **JOHN et al.** *Biotechnol. Adv.,* 2009, vol. 27, 145-52
- **BOEL et al.** *EMBO J.,* 1984, vol. 3, 1097-1102

- **CHEN et al.** *Prot. Eng.,* 1996, vol. 9, 499-505
- **CHEN et al.** *Prot. Eng.,* 1995, vol. 8, 575-582
- **CHEN et al.** *Biochem. J.,* 1994, vol. 301, 275-281
- **FIEROBE et al.** *Biochemistry,* 1996, vol. 35, 8698-8704
- **LI et al.** *Protein Eng.,* 1997, vol. 10, 1199-1204
- **FOGARTY et al.** *PROGRESS IN INDUSTRIAL MICROBIOLOGY,* 1979, vol. 15, 112-115
- **CHRISTOPHERSEN et al.** *Starch,* 1997, vol. 50, 39-45
- Efficient manganese catalysts for low-temperature bleaching. *Nature,* 1994, vol. 369, 637-639
- **DARTOIS et al.** *Biochemica et Biophysica Acta,* 1993, vol. 1131, 253-360
- **WOLFGANG KUNZE.** Technology Brewing and Malting. Research and Teaching Institute of Brewing, 2004
- STARCH AND ITS DERIVATIVES. Chapman and Hall, 1968, 194-201
- **NORDAHL PETERSEN et al.** *Nature Methods,* 2011, vol. 8, 785-786
- **EMANUELSSON et al.** *Nature Protocols,* 2007, vol. 2, 953-971
- **SOLINGEN et al.** *Extremophiles,* 2001, vol. 5, 333-341
- **VOGTENTANZ.** *Protein Expr. Purif.,* 2007, vol. 55, 40-52
- **NEIDHARDT et al.** *J. Bacteriol.,* 1974, vol. 119, 736-747
- **SUZUKI et al.** *J. Biol. Chem.,* 1989, vol. 264, 18933-38